# EUROPÄISCHE PATENTSCHRIFT

(11) **EP 3 523 834 B1**
(45) Veröffentlichungstag und Bekanntmachung des Hinweises auf die Patenterteilung: **25.01.2023**
(21) Anmeldenummer: 17787893.1
(22) Anmeldetag: 06.10.2017
(51) Int. Cl.: H01L 51/54

(54) **ELEKTRONISCHE VORRICHTUNG**
ELECTRONIC DEVICE
DISPOSITIF ÉLECTRONIQUE

(30) Priorität: 10.10.2016 EP 16193116
(43) Veröffentlichungstag der Anmeldung: 14.08.2019
(60) Teilanmeldung: 22190898.1
(73) Patentinhaber: Merck Patent GmbH, 64293 Darmstadt (DE)
(72) Erfinder: VOGES, Frank, 67098 Bad Duerkheim (DE); MUJICA-FERNAUD, Teresa, 64283 Darmstadt (DE); MONTENEGRO, Elvira, 69469 Weinheim (DE); EBERLE, Thomas, 76829 Landau (DE)
(86) Internationale Anmeldenummer: PCT/EP2017/075437
(87) Internationale Veröffentlichungsnummer: WO 2018/069167

(56) Entgegenhaltungen:
- EP-A1- 3 348 552
- WO-A1-2016/062368
- CN-A- 105 924 395
- KR-A- 20150 106 501
- ZENGZE CHU ET AL: "Synthesis of spiro[fluorene-9,9-xanthene] derivatives and their application as hole-transporting materials for organic light-emitting devices", SYNTHETIC METALS, ELSEVIER SEQUOIA, LAUSANNE, CH, Bd. 162, Nr. 7, 12. Februar 2012 (2012-02-12), Seiten 614-620, XP028406976, ISSN: 0379-6779, DOI: 10.1016/J.SYNTHMET.2012.02.009 [gefunden am 2012-02-20]

## Beschreibung

Die vorliegende Anmeldung betrifft eine elektronische Vorrichtung, welche eine Xanthen- oder Thioxanthen-Verbindung einer weiter unten definierten Formel enthält. Die elektronische Vorrichtung ist bevorzugt eine organische Elektrolumineszenzvorrichtung (OLED). Weiterhin betrifft die Anmeldung bestimmte Xanthen- oder Thioxanthen-Verbindungen als solche, ihre Verwendung in den oben genannten Vorrichtungen, sowie Verfahren zu ihrer Herstellung.

Unter elektronischen Vorrichtungen im Sinne dieser Anmeldung werden organische elektronische Vorrichtungen verstanden (organic electronic devices), d.h. Vorrichtungen, die organische Halbleitermaterialien als Funktionsmaterialien enthalten. Insbesondere werden darunter OLEDs verstanden. Unter der Bezeichnung OLEDs werden elektronische Vorrichtungen verstanden, welche eine oder mehrere Schichten enthaltend organische Verbindungen aufweisen und unter Anlegen von elektrischer Spannung Licht emittieren. Der Aufbau und das allgemeine Funktionsprinzip von OLEDs sind dem Fachmann bekannt.

Bei elektronischen Vorrichtungen, insbesondere OLEDs, besteht großes Interesse an der Verbesserung der Leistungsdaten, insbesondere Lebensdauer, Effizienz und Betriebsspannung. In diesen Punkten konnte noch keine vollständig zufriedenstellende Lösung gefunden werden.

Einen großen Einfluss auf die Leistungsdaten von elektronischen Vorrichtungen haben Schichten mit lochtransportierender Funktion. Zu diesen Schichten zählen unter anderem lochinjizierende Schichten, Lochtransportschichten und Elektronenblockierschichten. Zur Verwendung in diesen Schichten werden weiterhin neue Materialien mit lochtransportierenden Eigenschaften gesucht.

Zusätzlich besteht Bedarf an neuen Device-Aufbauten, sowie an neuen Kombinationen von Funktionsmaterialien in verschiedenen Schichten der OLEDs. Dabei sind insbesondere die Schichten mit lochtransportierender Funktion, ihre Zusammensetzung und ihre Abfolge von Bedeutung, um die Leistungsdaten von OLEDs zu verbessern.

Im Stand der Technik, beispielsweise in den Offenlegungsschriften WO 2014/072017 und CN 103666454, ZENGZE CHU ET AL: SYNTHETIC METALS, Bd. 162, Nr. 7, Seiten 614-620, KR 2015 0106501 A , CN 105 924 395 A, WO 2016/062368 A1 und EP 3 348 552 A1 sind Xanthen- und Thioxanthen-Verbindungen, die eine Arylaminogruppe tragen, als OLED-Funktionsmaterialien beschrieben.

Gegenüber den dort beschriebenen OLED-Aufbauten, welche die genannten Verbindungen enthalten, besteht jedoch noch Verbesserungsbedarf bezüglich der Leistungsdaten der OLEDs, insbesondere Betriebsspannung, Lebensdauer und Effizienz. Weiterhin besteht noch Verbesserungsbedarf bezüglich der dort offenbarten konkreten Verbindungen.

Im Rahmen der vorliegenden Erfindung wurde gefunden, dass OLEDs, welche bestimmte Xanthen- oder Thioxanthen-Verbindungen in einer an die Anode angrenzenden Schicht enthalten, oder diese Verbindungen in einer Schicht enthalten, welche mindestens zwei weitere Schichten zwischen dieser Schicht und der anodennächsten emittierenden Schicht aufweist, hervorragende Leistungsdaten aufweisen. Weiterhin wurde gefunden, dass bestimmte neue Xanthen- oder Thioxanthen-Verbindungen hervorragende Leistungsdaten aufweisen.

Gegenstand der vorliegenden Erfindung ist daher eine elektronische Vorrichtung, umfassend, in dieser Reihenfolge, eine Anode, eine lochtransportierende Schicht, eine emittierende Schicht, und eine Kathode, wobei die lochtransportierende Schicht eine Verbindung einer Formel (I) enthält 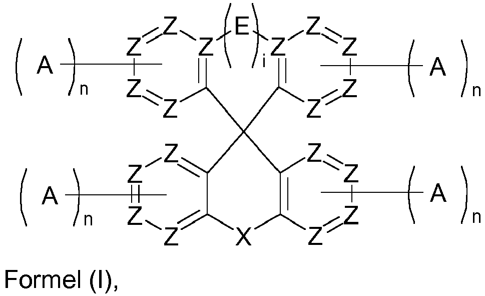 wobei gilt:
- A: ist eine Arylaminogruppe, wahlweise substituiert mit einem oder mehreren Resten R¹, oder eine Carbazol enthaltende Gruppe, wahlweise substituiert mit einem oder mehreren Resten R¹;
- E: ist eine Einfachbindung;
- X: ist O oder S;
- Z: ist bei jedem Auftreten gleich oder verschieden CR² oder N oder C, wobei eine Gruppe Z genau dann gleich C ist, wenn eine Gruppe A oder E an die betreffende Gruppe Z gebunden ist;
- R¹: ist bei jedem Auftreten gleich oder verschieden gewählt aus H, D, F, C(=O)R³, CN, Si(R³)₃, N(R³)₂, P(=O)(R³)₂, OR³, S(=O)R³, S(=O)₂R³, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R¹ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R³ substituiert sein können; und wobei eine oder mehrere CH₂-Gruppen in den genannten Alkyl-, Alkoxy-, Alkenyl- und Alkinylgruppen durch -R³C=CR³-, -C≡C-, Si(R³)₂, C=O, C=NR³, -C(=O)O-, -C(=O)NR³-, NR³, P(=O)(R³), -O-, -S-, SO oder SO₂ ersetzt sein können;
- R²: ist bei jedem Auftreten gleich oder verschieden gewählt aus H, D, F, C(=O)R³, CN, Si(R³)₃, N(R³)₂, P(=O)(R³)₂, OR³, S(=O)R³, S(=O)₂R³, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R² miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R³ substituiert sein können; und wobei eine oder mehrere CH₂-Gruppen in den genannten Alkyl-, Alkoxy-, Alkenyl- und Alkinylgruppen durch -R³C=CR³-, -C=C-, Si(R³)₂, C=O, C=NR³, -C(=O)O-, -C(=O)NR³-, NR³, P(=O)(R³), -O-, -S-, SO oder SO₂ ersetzt sein können;
- R³: ist bei jedem Auftreten gleich oder verschieden gewählt aus H, D, F, C(=O)R⁴, CN, Si(R⁴)₃, N(R⁴)₂, P(=O)(R⁴)₂, OR⁴, S(=O)R⁴, S(=O)₂R⁴, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R³ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁴ substituiert sein können; und wobei eine oder mehrere CH₂-Gruppen in den genannten Alkyl-, Alkoxy-, Alkenyl- und Alkinylgruppen durch -R⁴C=CR⁴-, -C≡C-, Si(R¹)₂, C=O, C=NR⁴, -C(=O)O-, -C(=O)NR⁴-, NR⁴, P(=O)(R⁴), -O-, -S-, SO oder SO₂ ersetzt sein können;
- R⁴: ist bei jedem Auftreten gleich oder verschieden gewählt aus H, D, F, CN, Alkyl- oder Alkoxygruppen mit 1 bis 20 C-Atomen, Alkenyl- oder Alkinylgruppen mit 2 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R⁴ miteinander verknüpft sein können und einen Ring bilden können; und wobei die genannten Alkyl-, Alkoxy-, Alkenyl- und Alkinylgruppen, aromatischen Ringsysteme und heteroaromatischen Ringsysteme mit F oder CN substituiert sein können;
- i: ist gleich 1;
- n: ist bei jedem Auftreten gleich oder verschieden 0 oder 1, wobei die Summe aller Indices n gleich 1, 2, 3 oder 4 ist.
wobei
zwischen der lochtransportierenden Schicht und der emittierenden Schicht mindestens zwei weitere Schichten angeordnet sind, und
keine weiteren emittierenden Schichten zwischen der emittierenden Schicht und der Anode angeordnet sind.

Weiterer Gegenstand der Anmeldung sind Xanthen- und Thioxanthenverbindungen einer bestimmten Formel (S) als solche, welche weiter unten definiert und beschrieben sind.

Unter einer Arylaminogruppe als Gruppe A wird eine Gruppe verstanden, die mindestens eine Einheit umfasst, in der mindestens eine Arylgruppe oder Heteroarylgruppe an ein dreibindiges Stickstoffatom gebunden ist. Wie die Gruppe weiter aufgebaut ist, und ob und welche weiteren Einheiten sie umfasst, ist für die Definition unerheblich.

Unter einer Carbazol enthaltenden Gruppe als Gruppe A werden auch Gruppen verstanden, die Derivate des Carbazols enthalten, beispielsweise Carbazolgruppen mit ankondensierten Benzolringen, oder Aza-Carbazolverbindungen. Wie die Gruppe weiter aufgebaut ist, und ob und welche weiteren Einheiten sie umfasst, ist für die Definition unerheblich.

Eine Arylgruppe im Sinne dieser Erfindung enthält 6 bis 40 aromatische Ringatome, von denen keines ein Heteroatom darstellt. Unter einer Arylgruppe im Sinne dieser Erfindung wird entweder ein einfacher aromatischer Cyclus, also Benzol, oder ein kondensierter aromatischer Polycyclus, beispielsweise Naphthalin, Phenanthren oder Anthracen, verstanden. Ein kondensierter aromatischer Polycyclus besteht im Sinne der vorliegenden Anmeldung aus zwei oder mehr miteinander kondensierten einfachen aromatischen Cyclen. Unter Kondensation zwischen Cyclen ist dabei zu verstehen, dass die Cyclen mindestens eine Kante miteinander teilen.

Eine Heteroarylgruppe im Sinne dieser Erfindung enthält 5 bis 40 aromatische Ringatome, von denen mindestens eines ein Heteroatom darstellt. Die Heteroatome der Heteroarylgruppe sind bevorzugt ausgewählt aus N, O und S. Unter einer Heteroarylgruppe im Sinne dieser Erfindung wird entweder ein einfacher heteroaromatischer Cyclus, beispielsweise Pyridin, Pyrimidin oder Thiophen, oder ein kondensierter heteroaromatischer Polycyclus, beispielsweise Chinolin oder Carbazol, verstanden. Ein kondensierter heteroaromatischer Polycyclus besteht im Sinne der vorliegenden Anmeldung aus zwei oder mehr miteinander kondensierten einfachen heteroaromatischen Cyclen. Unter Kondensation zwischen Cyclen ist dabei zu verstehen, dass die Cyclen mindestens eine Kante miteinander teilen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Triphenylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Ein aromatisches Ringsystem im Sinne dieser Erfindung enthält 6 bis 40 C-Atome im Ringsystem und umfasst keine Heteroatome als aromatische Ringatome. Ein aromatisches Ringsystem im Sinne dieser Erfindung enthält daher keine Heteroarylgruppen. Unter einem aromatischen Ringsystem im Sinne dieser Erfindung soll ein System verstanden werden, das nicht notwendigerweise nur Arylgruppen enthält, sondern in dem auch mehrere Arylgruppen durch eine Einfachbindung oder durch eine nicht-aromatische Einheit, wie beispielsweise ein oder mehrere wahlweise substituierte C-, Si-, N-, O- oder S-Atome, verbunden sein können. Dabei umfasst die nicht-aromatische Einheit bevorzugt weniger als 10 % der von H verschiedenen Atome, bezogen auf die Gesamtzahl der von H verschiedenen Atome des Systems. So sollen beispielsweise auch Systeme wie 9,9'-Spirobifluoren, 9,9'-Diarylfluoren, Triarylamin, Diarylether und Stilben als aromatische Ringsysteme im Sinne dieser Erfindung verstanden werden, und ebenso Systeme, in denen zwei oder mehr Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Arylgruppen über Einfachbindungen miteinander verknüpft sind, als aromatische Ringsysteme im Sinne dieser Erfindung verstanden, wie beispielsweise Systeme wie Biphenyl und Terphenyl.

Ein heteroaromatisches Ringsystem im Sinne dieser Erfindung enthält 5 bis 40 aromatische Ringatome, von denen mindestens eines ein Heteroatom darstellt. Die Heteroatome des heteroaromatischen Ringsystems sind bevorzugt ausgewählt aus N, O und/oder S. Ein heteroaromatisches Ringsystem entspricht der oben genannten Definition eines aromatischen Ringsystems, weist jedoch mindestens ein Heteroatom als eines der aromatischen Ringatome auf. Es unterscheidet sich dadurch von einem aromatischen Ringsystem im Sinne der Definition der vorliegenden Anmeldung, welches gemäß dieser Definition kein Heteroatom als aromatisches Ringatom enthalten kann.

Unter einem aromatischen Ringsystem mit 6 bis 40 aromatischen Ringatomen oder einem heteroaromatischen Ringsystem mit 5 bis 40 aromatischen Ringatomen, werden insbesondere Gruppen verstanden, die abgeleitet sind von den oben unter Arylgruppen und Heteroarylgruppen genannten Gruppen sowie von Biphenyl, Terphenyl, Quaterphenyl, Fluoren, Spirobifluoren, Dihydrophenanthren, Dihydropyren, Tetrahydropyren, Indenofluoren, Truxen, Isotruxen, Spirotruxen, Spiroisotruxen, Indenocarbazol, oder von Kombinationen dieser Gruppen.

Im Rahmen der vorliegenden Erfindung werden unter einer geradkettigen Alkylgruppe mit 1 bis 20 C-Atomen bzw. einer verzweigten oder cyclischen Alkylgruppe mit 3 bis 20 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden.

Unter einer Alkoxy- oder Thioalkylgruppe mit 1 bis 20 C-Atomen, in der auch einzelne H-Atome oder CH₂-Gruppen durch die oben bei der Definition der Reste genannten Gruppen substituiert sein können, werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet.

In der Verbindung der Formel (I) ist X bevorzugt gleich O.

Weiterhin ist die Summe der Indices n in Formel (I) bevorzugt gleich 1 oder 2, besonders bevorzugt gleich 1.

Weiterhin sind bevorzugt höchstens 2 Gruppen Z pro Ring gleich N. Weiterhin bevorzugt sind höchstens 4 Gruppen Z pro Verbindung der Formel (I), ganz besonders bevorzugt höchstens 2 Gruppen Z pro Verbindung der Formel (I) gleich Z.

Besonders bevorzugt ist Z gleich CR², wobei in dem Fall, dass eine Gruppe A oder E an die betreffende Gruppe Z gebunden ist, diese Gruppe Z gleich C ist.

Bevorzugt ist R¹ bei jedem Auftreten gleich oder verschieden gewählt aus H, D, F, CN, Si(R³)₃, geradkettigen Alkyl- oder Alkoxygruppen mit 1 bis 20 C-Atomen, verzweigten oder cyclischen Alkyl- oder Alkoxygruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkyl- und Alkoxygruppen, die genannten aromatischen Ringsysteme und die genannten heteroaromatischen Ringsysteme jeweils mit einem oder mehreren Resten R³ substituiert sein können; und wobei in den genannten Alkyl- oder Alkoxygruppen eine oder mehrere CH₂-Gruppen durch -C=C-, -R³C=CR³-, Si(R³)₂, C=O, C=NR³, -NR³-, -O-, -S-, -C(=O)O- oder -C(=O)NR³- ersetzt sein können.

Besonders bevorzugt ist R¹ bei jedem Auftreten gleich oder verschieden gewählt aus H, F, CN, geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkylgruppen, die genannten aromatischen Ringsysteme und die genannten heteroaromatischen Ringsysteme jeweils mit einem oder mehreren Resten R³ substituiert sein können.

Bevorzugt ist R² bei jedem Auftreten gleich oder verschieden gewählt aus H, D, F, CN, Si(R³)₃, geradkettigen Alkyl- oder Alkoxygruppen mit 1 bis 20 C-Atomen, verzweigten oder cyclischen Alkyl- oder Alkoxygruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkyl- und Alkoxygruppen, die genannten aromatischen Ringsysteme und die genannten heteroaromatischen Ringsysteme jeweils mit einem oder mehreren Resten R³ substituiert sein können; und wobei in den genannten Alkyl- oder Alkoxygruppen eine oder mehrere CH₂-Gruppen durch -C=C-, -R³C=CR³-, Si(R³)₂, C=O, C=NR³, -NR³-, -O-, -S-, -C(=O)O- oder -C(=O)NR³- ersetzt sein können.

Besonders bevorzugt ist R² bei jedem Auftreten gleich oder verschieden gewählt aus H, F, CN, geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkylgruppen, die genannten aromatischen Ringsysteme und die genannten heteroaromatischen Ringsysteme jeweils mit einem oder mehreren Resten R³ substituiert sein können.

Ganz besonders bevorzugt ist R² gleich H.

Bevorzugt ist R³ bei jedem Auftreten gleich oder verschieden gewählt aus H, D, F, CN, Si(R⁴)₃, geradkettigen Alkyl- oder Alkoxygruppen mit 1 bis 20 C-Atomen, verzweigten oder cyclischen Alkyl- oder Alkoxygruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkyl- und Alkoxygruppen, die genannten aromatischen Ringsysteme und die genannten heteroaromatischen Ringsysteme jeweils mit einem oder mehreren Resten R⁴ substituiert sein können; und wobei in den genannten Alkyl- oder Alkoxygruppen eine oder mehrere CH₂-Gruppen durch -C≡C-, -R⁴C=CR⁴-, Si(R⁴)₂, C=O, C=NR⁴, -NR⁴-, -O-, -S-, -C(=O)O- oder -C(=O)NR⁴- ersetzt sein können.

Besonders bevorzugt ist R³ bei jedem Auftreten gleich oder verschieden gewählt aus H, F, CN, geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkylgruppen, die genannten aromatischen Ringsysteme und die genannten heteroaromatischen Ringsysteme jeweils mit einem oder mehreren Resten R⁴ substituiert sein können.

Bevorzugt ist die Gruppe A eine Arylaminogruppe, welche mit einem oder mehreren Resten R¹ substituiert sein kann.

Die Arylaminogruppe als Gruppe A entspricht bevorzugt einer Formel (A) 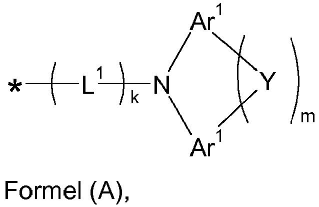 wobei gilt:
- L¹: ist bei jedem Auftreten gleich oder verschieden C=O, Si(R¹)₂, PR¹, P(=O)(R¹), O, S, SO, SO₂, eine Alkylengruppe mit 1 bis 20 C-Atomen oder eine Alkenylen- oder Alkinylengruppe mit 2 bis 20 C-Atomen, wobei in den genannten Gruppen eine oder mehrere CH₂-Gruppen durch C=O, C=NR¹, C=O-O, C=O-NR¹, Si(R¹)₂, NR¹, P(=O)(R¹), O, S, SO oder SO₂ ersetzt sein können und wobei ein oder mehrere H-Atome in den oben genannten Gruppen durch D, F oder CN ersetzt sein können, oder ein aromatisches oder heteroaromatisches Ringsystem mit 6 bis 24 aromatischen Ringatomen, welches durch einen oder mehrere Reste R¹ substituiert sein kann;
- Ar¹: ist bei jedem Auftreten gleich oder verschieden ein aromatisches oder heteroaromatisches Ringsystem mit 6 bis 30 aromatischen Ringatomen, das mit einem oder mehreren Resten R¹ substituiert sein kann;
- Y: ist gewählt aus einer Einfachbindung, BR¹, C(R¹)₂, C(R¹)₂-C(R¹)₂, Si(R¹)₂, Si(R¹)₂-Si(R¹)₂, C=O, C=NR¹, C=C(R¹)₂, C(=O)N(R¹), O, S, S=O, SO₂ und NR¹;
- k: ist gleich 0, 1, 2 oder 3;
- m: ist gleich 0 oder 1;
wobei die Gruppe A über die mit * markierte Bindung an den Rest der Verbindung der Formel (I) gebunden ist.

Bevorzugt ist in Formel (A) L¹ bei jedem Auftreten gleich oder verschieden Si(R¹)₂, O, S, eine Alkylengruppe mit 1 bis 10 C-Atomen oder eine Alkenylen- oder Alkinylengruppe mit 2 bis 10 C-Atomen, wobei bei den genannten Gruppen eine oder mehrere CH₂-Gruppen durch Si(R¹)₂, O oder S ersetzt sein können und wobei ein oder mehrere H-Atome in den genannten Gruppen durch D, F oder CN ersetzt sein können, oder ein aromatisches oder heteroaromatisches Ringsystem mit 6 bis 24 aromatischen Ringatomen, welches durch einen oder mehrere Reste R¹ substituiert sein kann.

Besonders bevorzugt ist L¹ bei jedem Auftreten gleich oder verschieden ein aromatisches oder heteroaromatisches Ringsystem mit 6 bis 18 aromatischen Ringatomen, welches mit einem oder mehreren Resten R¹ substituiert sein kann. Ganz besonders bevorzugt ist L¹ bei jedem Auftreten gleich oder verschieden Phenyl, Biphenyl, Naphthyl, Terphenyl, Fluorenyl, Spirobifluoren, Indenofluorenyl, Carbazol, Dibenzofuran, oder Dibenzothiophen, die jeweils mit einem oder mehreren Resten R¹ substituiert sein können.

Besonders bevorzugte Gruppen L¹ sind die folgenden Gruppen: 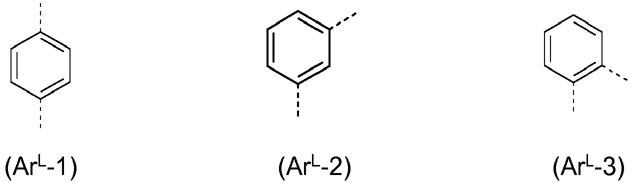 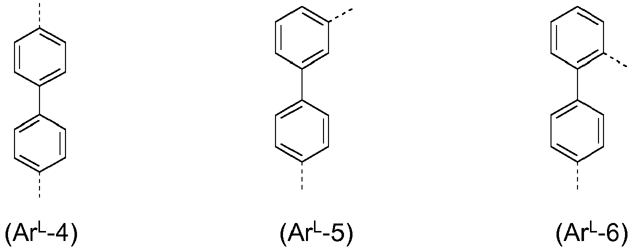 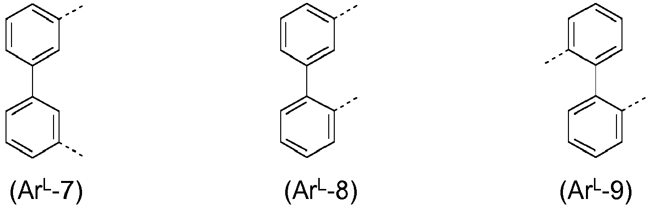 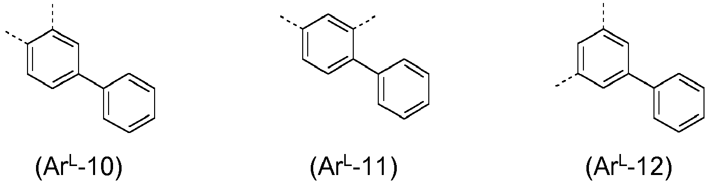 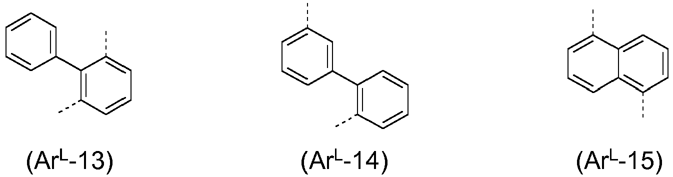 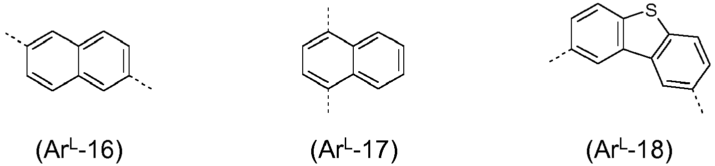 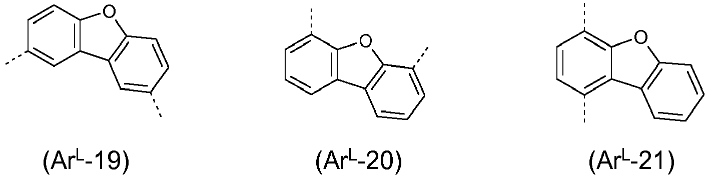 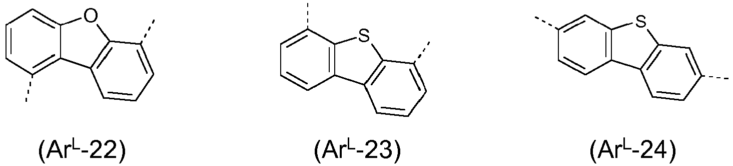 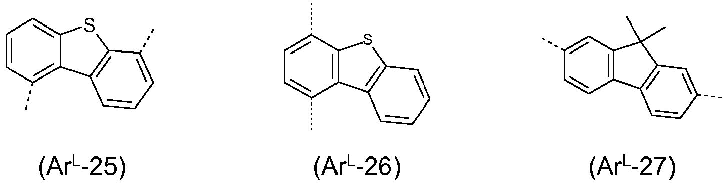 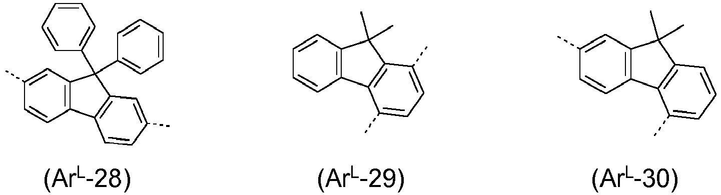 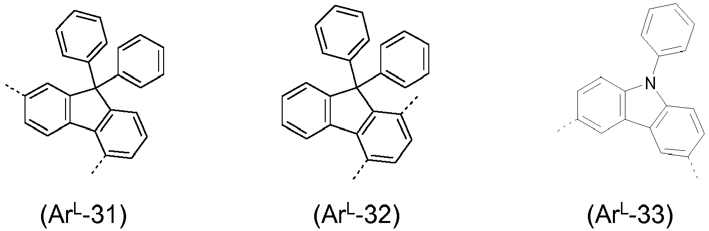 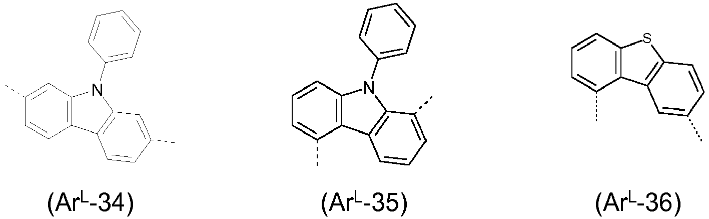 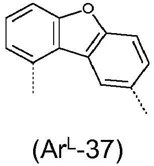 wobei die gestrichelten Bindungen die Bindungen von L¹ an den Rest der Verbindung kennzeichnen, und wobei die Gruppen an den unsubstituiert gezeichneten Positionen jeweils mit Resten R¹ substituiert sein können, und wobei die Gruppen an den unsubstituiert gezeichneten Positionen bevorzugt nicht mit Resten R¹ substituiert sind.

Weiterhin bevorzugt ist in Formel (A) k gleich 0 oder 1, besonders bevorzugt gleich 0.

Weiterhin bevorzugt ist in Formel (A) m gleich 0, d.h. die beiden Gruppen Ar¹ sind nicht miteinander verbunden.

Weiterhin bevorzugt ist in Formel (A) Ar¹ bei jedem Auftreten gleich oder verschieden ein aromatisches oder heteroaromatisches Ringsystem mit 6 bis 24 aromatischen Ringatomen, das mit einem oder mehreren Resten R¹ substituiert sein kann. Ganz besonders bevorzugt sind darunter Phenyl, Biphenyl, Naphthyl, Terphenyl, Fluorenyl, Spirobifluoren, Indenofluorenyl, Carbazolyl, Dibenzofuranyl, und Dibenzothiophenyl, die mit einem oder mehreren Resten R¹ substituiert sein können.

Bevorzugte Gruppen Ar¹ sind in der folgenden Tabelle abgebildet:

| | | |
|---|---|---|
| 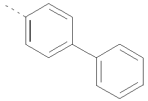 | 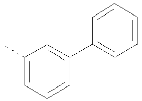 | 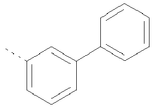 |
| Ar¹-1 | Ar¹-3 | Ar¹-3 |
| 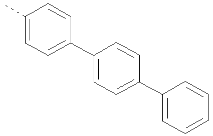 | 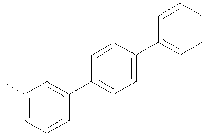 | 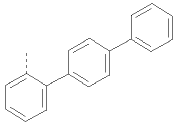 |
| Ar¹-4 | Ar¹-5 | Ar¹-6 |
| 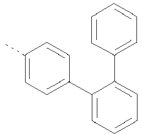 | 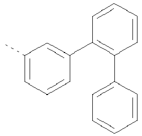 | 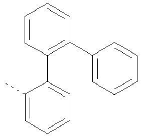 |
| Ar¹-7 | Ar¹-8 | Ar¹-9 |
| 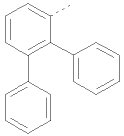 | 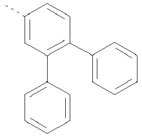 | 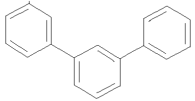 |
| Ar¹-10 | Ar¹-11 | Ar¹-12 |
| 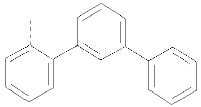 | 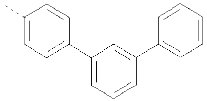 | 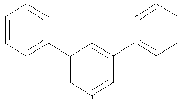 |
| Ar¹-13 | Ar¹-14 | Ar¹-15 |
| 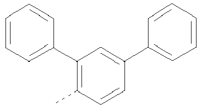 | 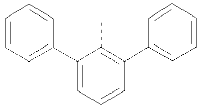 | 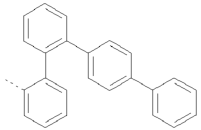 |
| Ar¹-16 | Ar¹-17 | Ar¹-18 |
| 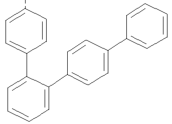 | 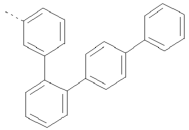 | 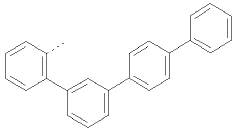 |
| Ar¹-19 | Ar¹-20 | Ar¹-21 |
| 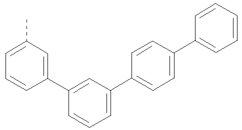 | 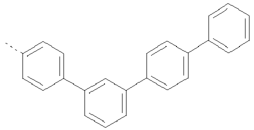 | 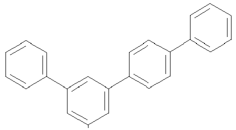 |
| Ar¹-22 | Ar¹-23 | Ar¹-24 |
| 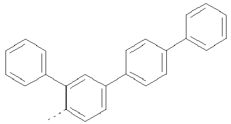 | 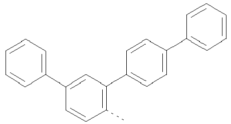 | 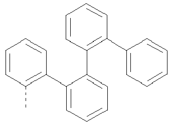 |
| Ar¹-25 | Ar¹-26 | Ar¹-27 |
| 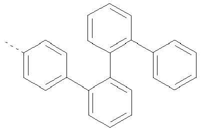 | 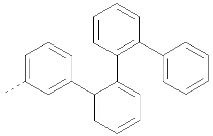 | 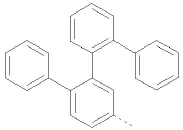 |
| Ar¹-28 | Ar¹-29 | Ar¹-30 |
| 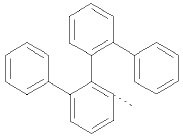 | 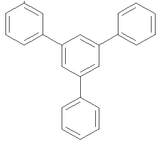 | 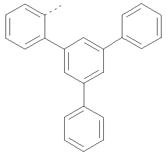 |
| Ar¹-31 | Ar¹-32 | Ar¹-33 |
| 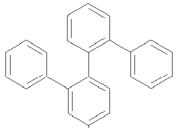 | 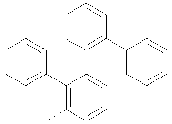 | 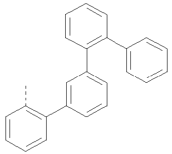 |
| Ar¹-34 | Ar¹-35 | Ar¹-36 |
| 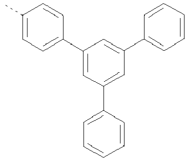 | 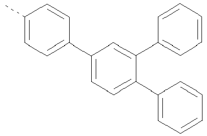 | 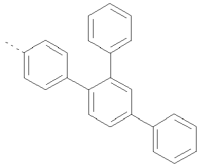 |
| Ar¹-37 | Ar¹-38 | Ar¹-39 |
| 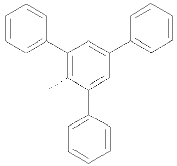 | 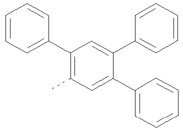 | 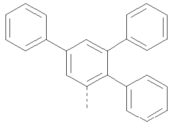 |
| Ar¹-40 | Ar¹-41 | Ar¹-42 |
| 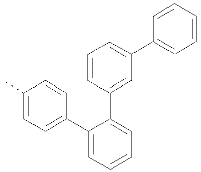 | 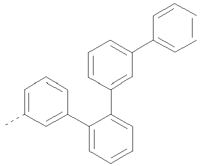 | 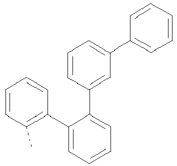 |
| Ar¹-43 | Ar¹-44 | Ar¹-45 |
| 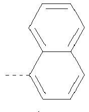 | 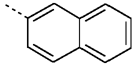 | 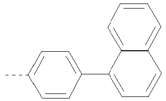 |
| Ar¹-46 | Ar¹-47 | Ar¹-48 |
| 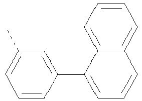 | 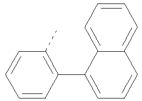 | 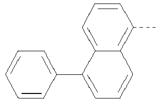 |
| Ar¹-49 | Ar¹-50 | Ar¹-51 |
| 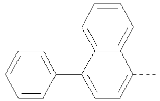 | 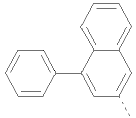 | 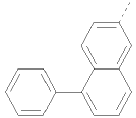 |
| Ar¹-52 | Ar¹-53 | Ar¹-54 |
| 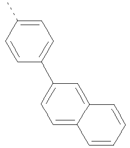 | 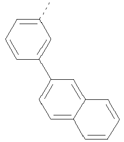 | 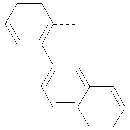 |
| Ar¹-55 | Ar¹-56 | Ar¹-57 |
| 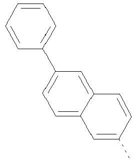 | 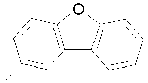 | 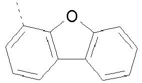 |
| Ar¹-58 | Ar¹-59 | Ar¹-60 |
| 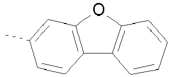 | 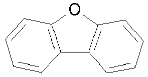 | 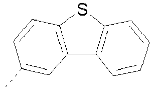 |
| Ar¹-61 | Ar¹-62 | Ar¹-63 |
| 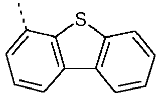 | 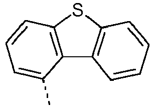 | 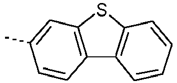 |
| Ar¹-64 | Ar¹-65 | Ar¹-66 |
| 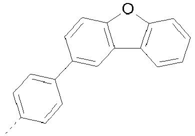 | 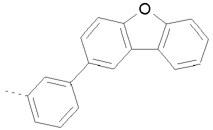 | 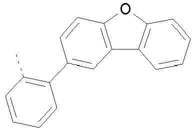 |
| Ar¹-67 | Ar¹-68 | Ar¹-69 |
| 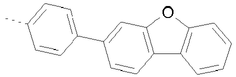 | 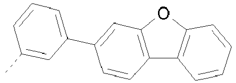 | 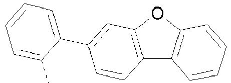 |
| Ar¹-70 | Ar¹-71 | Ar¹-72 |
| 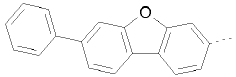 | 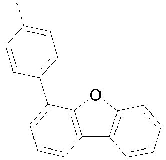 | 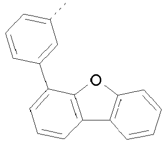 |
| Ar¹-73 | Ar¹-74 | Ar¹-75 |
| 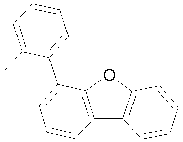 | 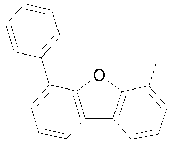 | 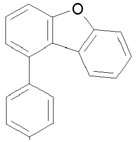 |
| Ar¹-76 | Ar¹-77 | Ar¹-78 |
| 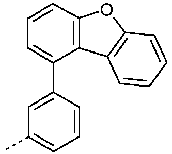 | 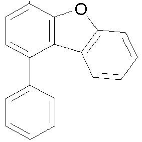 | 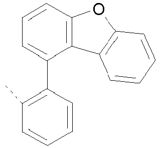 |
| Ar¹-79 | Ar¹-80 | Ar¹-81 |
| 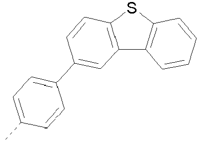 | 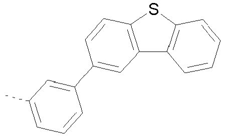 | 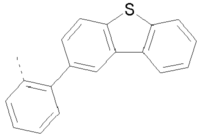 |
| Ar¹-82 | Ar¹-83 | Ar¹-84 |
| 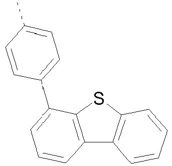 | 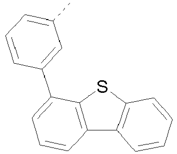 | 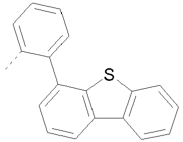 |
| Ar¹-85 | Ar¹-86 | Ar¹-87 |
| 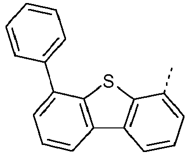 | 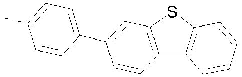 | 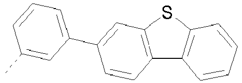 |
| Ar¹-88 | Ar¹-89 | Ar¹-90 |
| 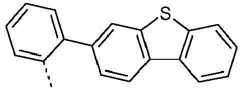 | 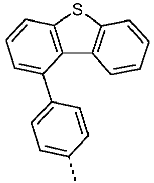 | 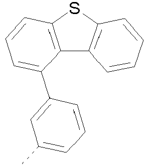 |
| Ar¹-91 | Ar¹-92 | Ar¹-93 |
| 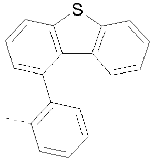 | 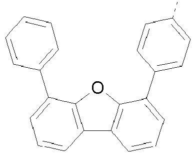 | 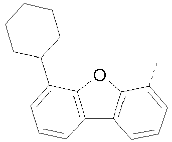 |
| Ar¹-94 | Ar¹-95 | Ar¹-96 |
| 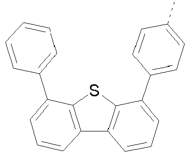 | 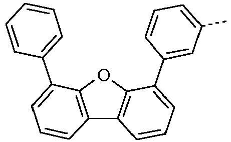 | 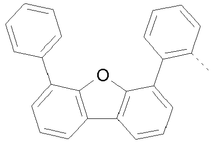 |
| Ar¹-94 | Ar¹-95 | Ar¹-96 |
| 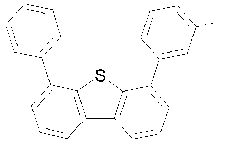 | 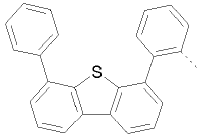 | 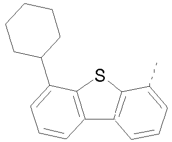 |
| Ar¹-97 | Ar¹-98 | Ar¹-99 |
| 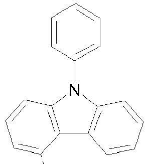 | 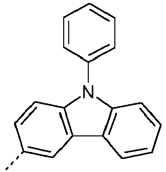 | 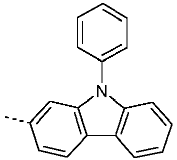 |
| Ar¹-100 | Ar¹-101 | Ar¹-102 |
| 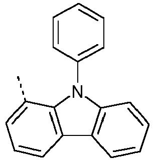 | 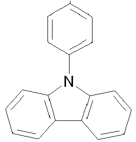 | 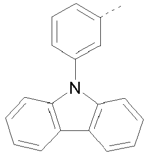 |
| Ar¹-103 | Ar¹-104 | Ar¹-105 |
| 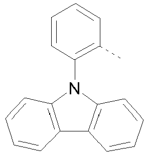 | 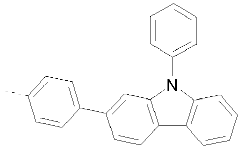 | 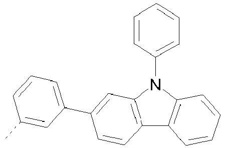 |
| Ar¹-106 | Ar¹-107 | Ar¹-108 |
| 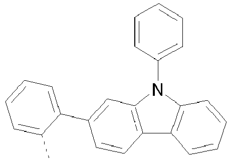 | 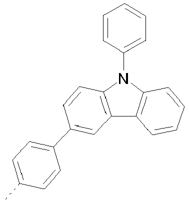 | 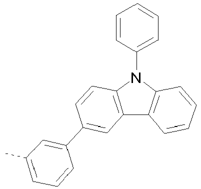 |
| Ar¹-109 | Ar¹-110 | Ar¹-111 |
| 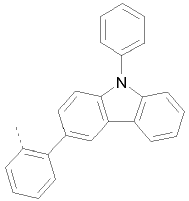 | 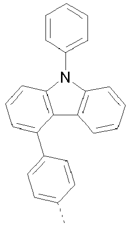 | 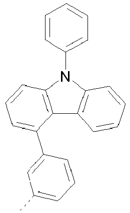 |
| Ar¹-112 | Ar¹-113 | Ar¹-114 |
| 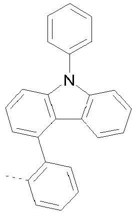 | 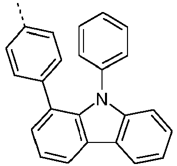 | 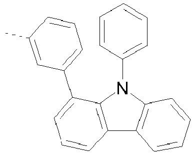 |
| Ar¹-115 | Ar¹-116 | Ar¹-117 |
| 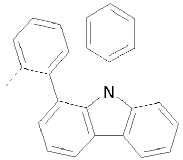 | 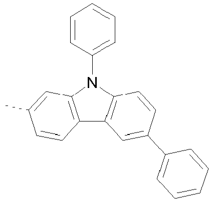 | 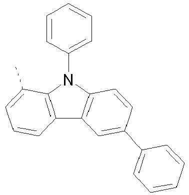 |
| Ar¹-118 | Ar¹-119 | Ar¹-120 |
| 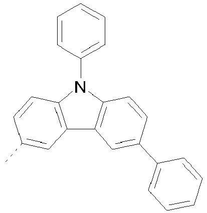 | 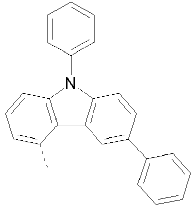 | 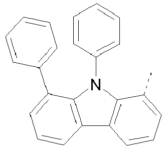 |
| Ar¹-121 | Ar¹-122 | Ar¹-123 |
| 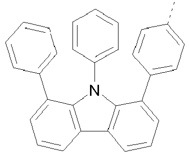 | 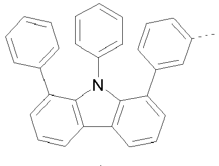 | 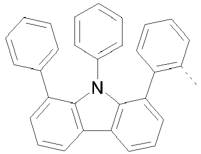 |
| Ar¹-124 | Ar¹-125 | Ar¹-126 |
| 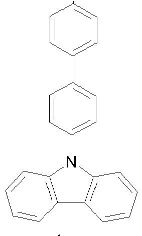 | 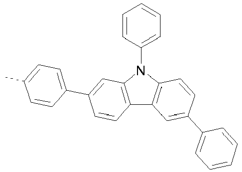 | 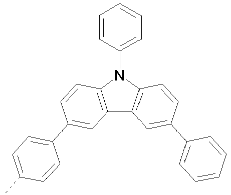 |
| Ar¹-127 | Ar¹-128 | Ar¹-129 |
| 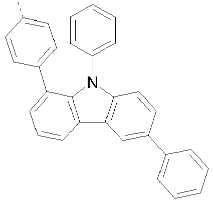 | 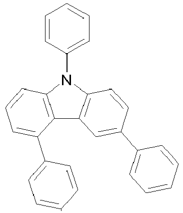 | 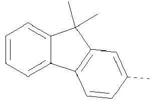 |
| Ar¹-130 | Ar¹-131 | Ar¹-132 |
| 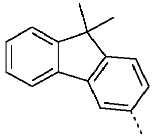 | 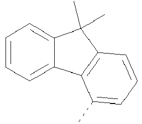 | 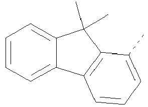 |
| Ar¹-133 | Ar¹-134 | Ar¹-135 |
| 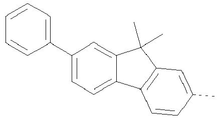 | 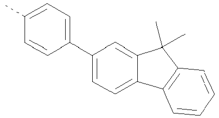 | 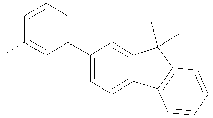 |
| Ar¹-136 | Ar¹-137 | Ar¹-138 |
| 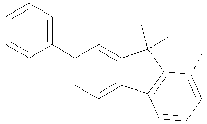 | 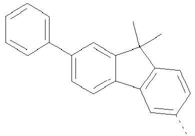 | 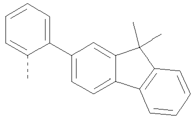 |
| Ar¹-139 | Ar¹-140 | Ar¹-141 |
| 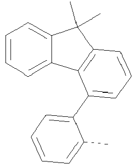 | 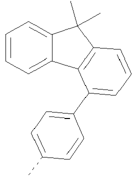 | 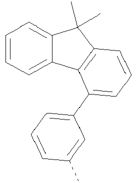 |
| Ar¹-142 | Ar¹-143 | Ar¹-144 |
| 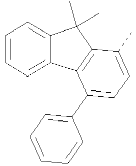 | 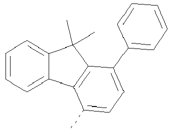 | 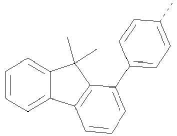 |
| Ar¹-145 | Ar¹-146 | Ar¹-147 |
| 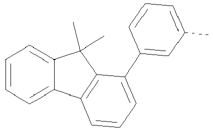 | 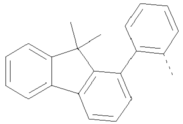 | 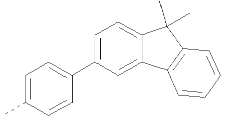 |
| Ar¹-148 | Ar¹-149 | Ar¹-150 |
| 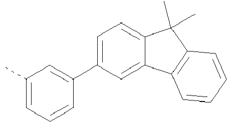 | 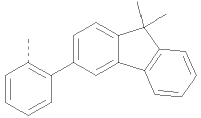 | 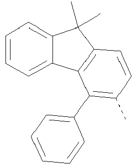 |
| Ar¹-151 | Ar¹-152 | Ar¹-153 |
| 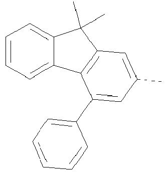 | 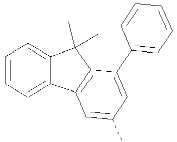 | 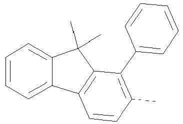 |
| Ar¹-154 | Ar¹-155 | Ar¹-156 |
| 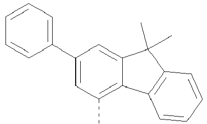 | 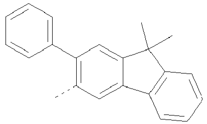 | 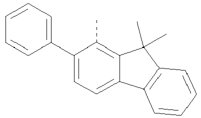 |
| Ar¹-157 | Ar¹-158 | Ar¹-159 |
| 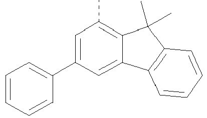 | 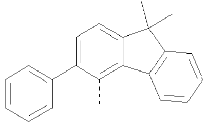 | 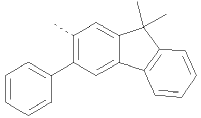 |
| Ar¹-160 | Ar¹-161 | Ar¹-162 |
| 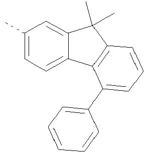 | 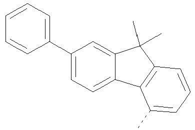 | 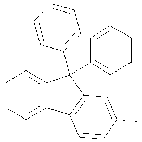 |
| Ar¹-163 | Ar¹-164 | Ar¹-165 |
| 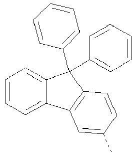 | 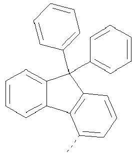 | 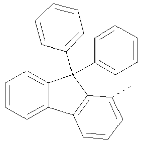 |
| Ar¹-166 | Ar¹-167 | Ar¹-168 |
| 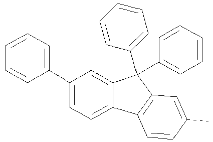 | 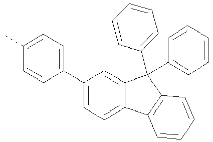 | 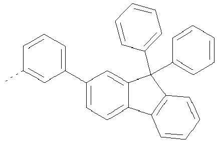 |
| Ar¹-169 | Ar¹-170 | Ar¹-171 |
| 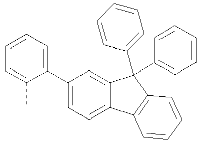 | 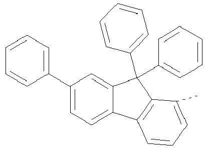 | 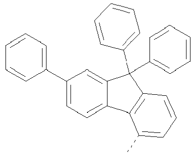 |
| Ar¹-172 | Ar¹-173 | Ar¹-174 |
| 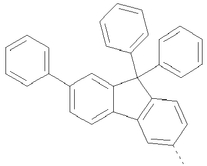 | 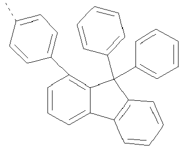 | 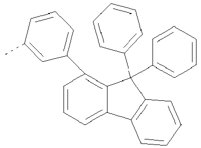 |
| Ar¹-175 | Ar¹-176 | Ar¹-177 |
| 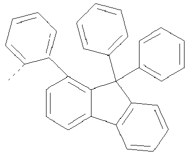 | 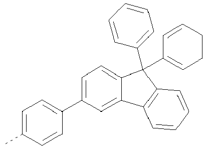 | 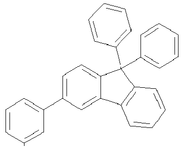 |
| Ar¹-178 | Ar¹-179 | Ar¹-180 |
| 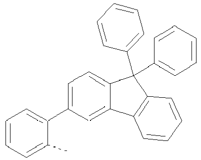 | 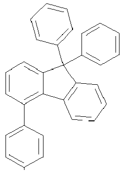 | 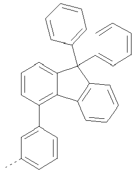 |
| Ar¹-181 | Ar¹-182 | Ar¹-183 |
| 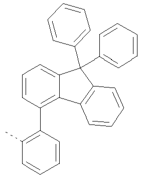 | 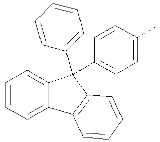 | 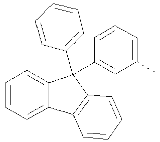 |
| Ar¹-184 | Ar¹-185 | Ar¹-186 |
| 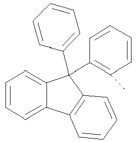 | 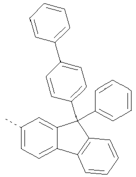 | 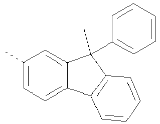 |
| Ar¹-187 | Ar¹-188 | Ar¹-189 |
| 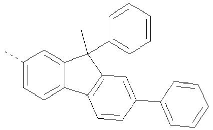 | 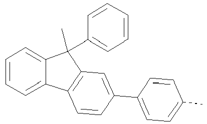 | 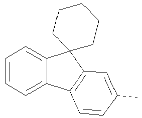 |
| Ar¹-190 | Ar¹-191 | Ar¹-192 |
| 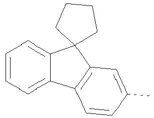 | 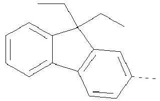 | 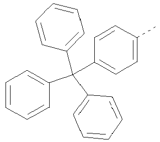 |
| Ar¹-193 | Ar¹-194 | Ar¹-195 |
| 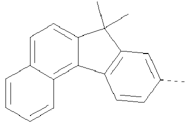 | 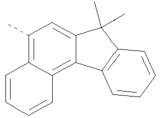 | 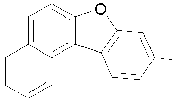 |
| Ar¹-196 | Ar¹-197 | Ar¹-198 |
| 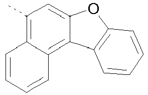 | 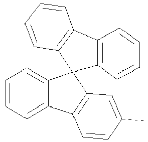 | 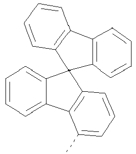 |
| Ar¹-199 | Ar¹-200 | Ar¹-201 |
| 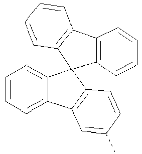 | 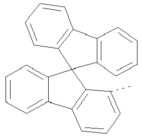 | 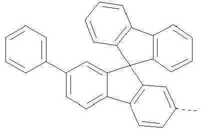 |
| Ar¹-202 | Ar¹-203 | Ar¹-204 |
| 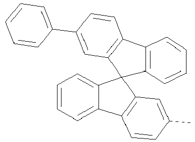 | 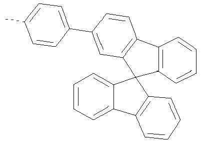 | 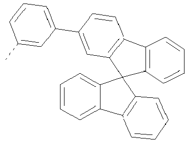 |
| Ar¹-205 | Ar¹-206 | Ar¹-207 |
| 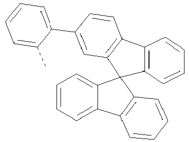 | 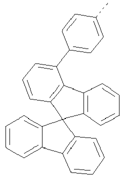 | 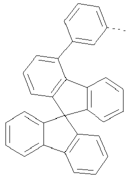 |
| Ar¹-208 | Ar¹-209 | Ar¹-210 |
| 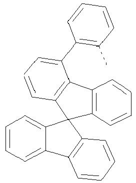 | 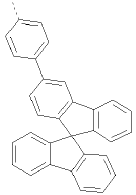 | 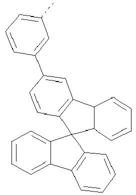 |
| Ar¹-211 | Ar¹-212 | Ar¹-213 |
| 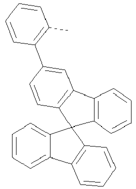 | 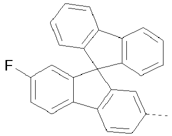 | 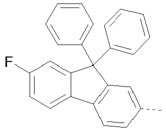 |
| Ar¹-214 | Ar¹-215 | Ar¹-216 |
| 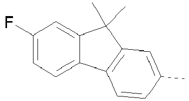 | 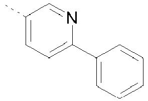 | 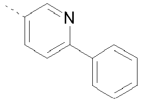 |
| Ar¹-217 | Ar¹-218 | Ar¹-219 |
| 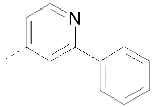 | 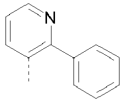 | 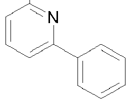 |
| Ar¹-220 | Ar¹-221 | Ar¹-222 |
| 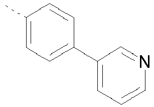 | 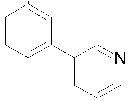 | 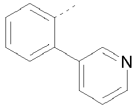 |
| Ar¹-223 | Ar¹-224 | Ar¹-225 |
| 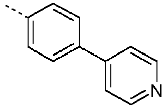 | 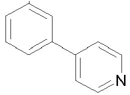 | 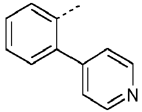 |
| Ar¹-226 | Ar¹-227 | Ar¹-228 |
| 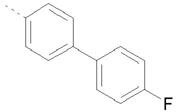 | 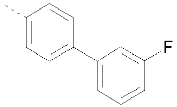 | 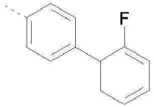 |
| Ar¹-229 | Ar¹-230 | Ar¹-231 |
| 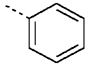 | 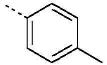 | 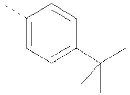 |
| Ar¹-232 | Ar¹-233 | Ar¹-234 |
| 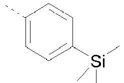 | 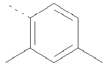 | 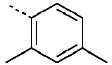 |
| Ar¹-235 | Ar¹-236 | Ar¹-237 |
| 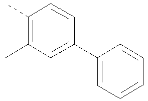 | 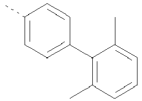 | 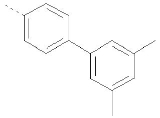 |
| Ar¹-238 | Ar¹-239 | Ar¹-240 |
| 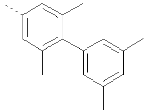 | 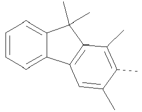 | 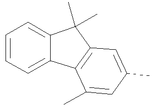 |
| Ar¹-241 | Ar¹-242 | Ar¹-243 |
| 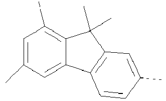 | 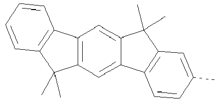 | 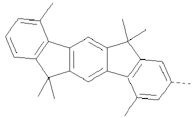 |
| Ar¹-244 | Ar¹-245 | Ar¹-246 |

Die oben gezeigten Gruppen können an ihren unsubstituiert gezeichneten Positionen jeweils mit Resten R¹ substituiert sein.

Besonders bevorzugt sind unter den oben genannten Gruppen Ar¹ die Gruppen Ar¹-1, Ar¹-2, Ar¹-3, Ar¹-4, Ar¹-5, Ar¹-6, Ar¹-15, Ar¹-16, Ar¹-46, Ar¹-47, Ar¹-48, Ar¹-55, Ar¹-59, Ar¹-60, Ar¹-61, Ar¹-62, Ar¹-63, Ar¹-64, Ar¹-65, Ar¹-66, Ar¹-67, Ar¹-70, Ar¹-74, Ar¹-78, Ar¹-82, Ar¹-89, Ar¹-92, Ar¹-100, Ar¹-101, Ar¹-102, Ar¹-104, Ar¹-107, Ar¹-110, Ar¹-113, Ar¹-127, Ar¹-132, Ar¹-133, Ar¹-134, Ar¹-135, Ar¹-136, Ar¹-137, Ar¹-143, Ar¹-145, Ar¹-147, Ar¹-163, Ar¹-164, Ar¹-165, Ar¹-166, Ar¹-167, Ar¹-168, Ar¹-188, Ar¹-189, Ar¹-200, Ar¹-201, Ar¹-202, Ar¹-203, und Ar¹-232. Ganz besonders bevorzugt sind unter den oben genannten Gruppen Ar¹ die Gruppen Ar¹-1, Ar¹-74, Ar¹-132, Ar¹-134, Ar¹-136, Ar¹-137, Ar¹-165, Ar¹-200, und Ar¹-201.

Weiterhin ist in Formel (A) die Gruppe Y bevorzugt gewählt aus einer Einfachbindung, C(R¹)₂, O, S und NR¹. Besonders bevorzugt ist Y eine Einfachbindung.

Wenn die Gruppe A eine Carbazol enthaltende Gruppe ist, ist sie bevorzugt eine Carbazolgruppe als solche und im engeren Sinne, oder eine Indenocarbazolgruppe als solche und im engeren Sinne. Die Carbazolgruppe kann über ihr Stickstoffatom an den Rest der Verbindung gebunden sein, oder über einen ihrer Benzolringe.

Besonders bevorzugte Gruppen A entsprechen den folgenden Formeln:

| | |
|---|---|
| 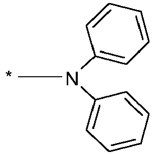 | 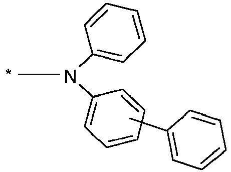 |
| Formel (A-1) | Formel (A-2) |
| 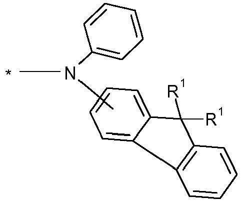 | 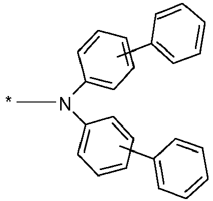 |
| Formel (A-3) | Formel (A-4) |
| 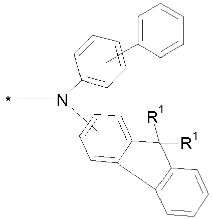 | 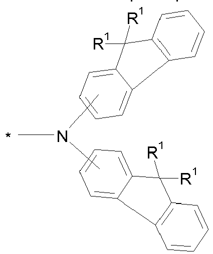 |
| Formel (A-5) | Formel (A-6) |
| 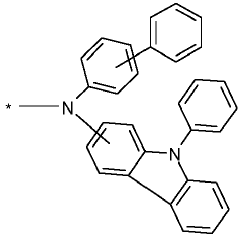 | 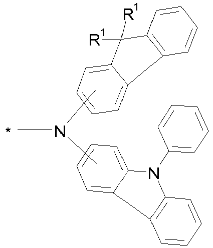 |
| Formel (A-7) | Formel (A-8) |
| 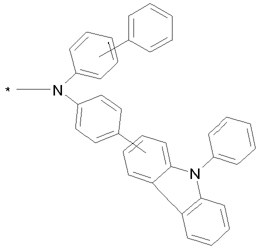 | 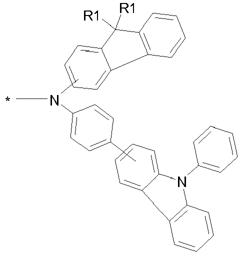 |
| Formel (A-9) | Formel (A-10) |
| 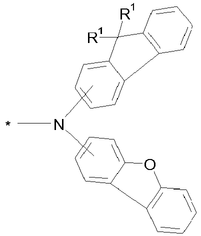 | 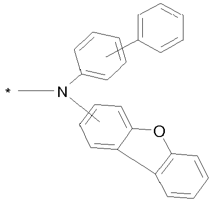 |
| Formel (A-11) | Formel (A-12) |
| 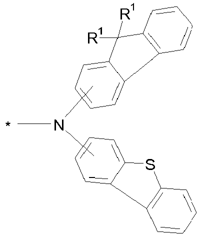 | 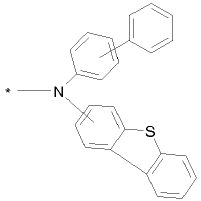 |
| Formel (A-13) | Formel (A-14) |
| 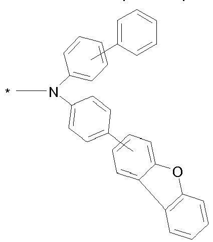 | 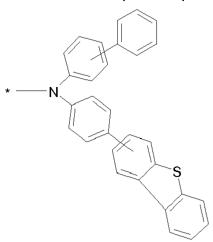 |
| Formel (A-15) | Formel (A-16) |
| 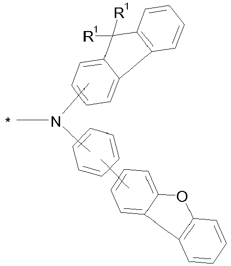 | 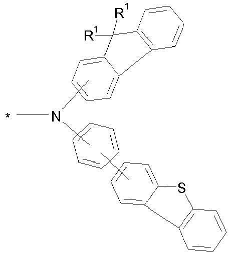 |
| Formel (A-17) | Formel (A-18) |
| 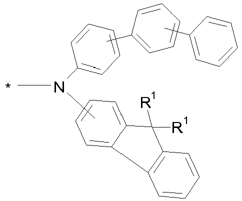 | 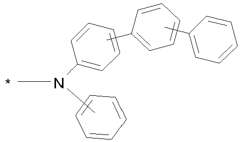 |
| Formel (A-19) | Formel (A-20) |
| 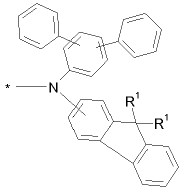 | 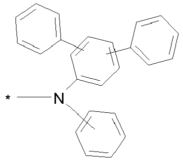 |
| Formel (A-21) | Formel (A-22) |
| 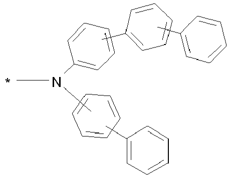 | 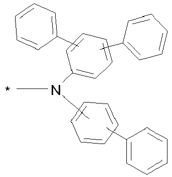 |
| Formel (A-23) | Formel (A-24) |
| 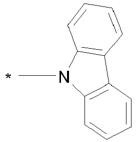 | 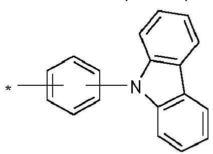 |
| Formel (A-24) | Formel (A-25) |
| 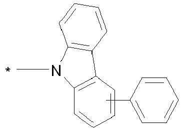 | 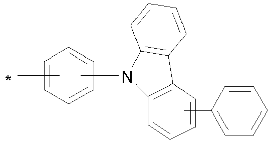 |
| Formel (A-26) | Formel (A-27) |
| 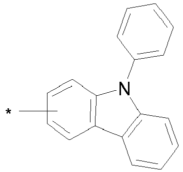 | 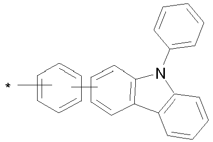 |
| Formel (A-28) | Formel (A-29) |
| 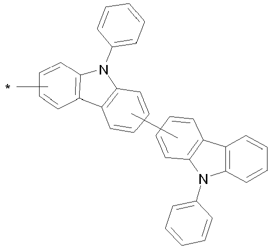 | 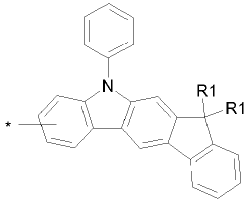 |
| Formel (A-30) | Formel (A-31) |
| 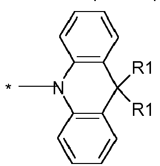 | 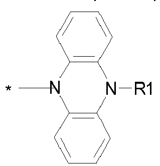 |
| Formel (A-32) | Formel (A-33) |
| 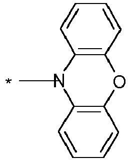 | 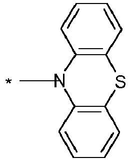 |
| Formel (A-34) | Formel (A-35) |
| 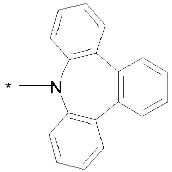 | 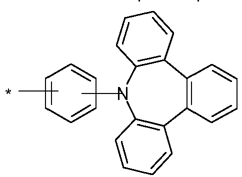 |
| Formel (A-36) | Formel (A-37) |
| 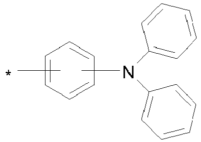 | 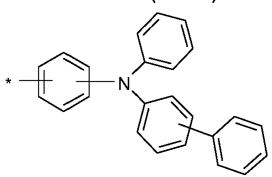 |
| Formel (A-38) | Formel (A-39) |
| 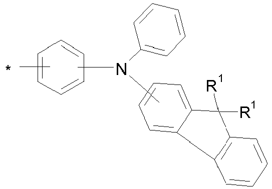 | 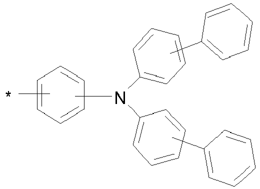 |
| Formel (A-40) | Formel (A-41) |
| 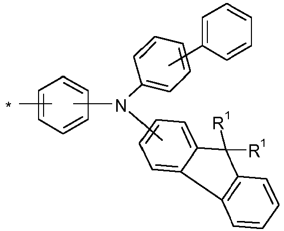 | 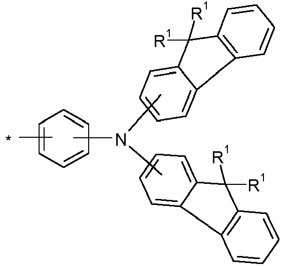 |
| Formel (A-42) | Formel (A-43) |
| 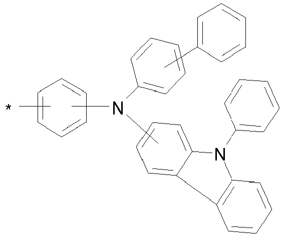 | 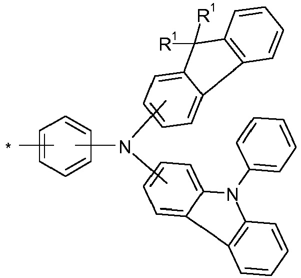 |
| Formel (A-44) | Formel (A-45) |
| 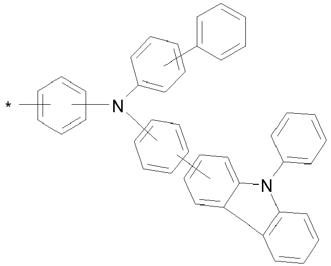 | 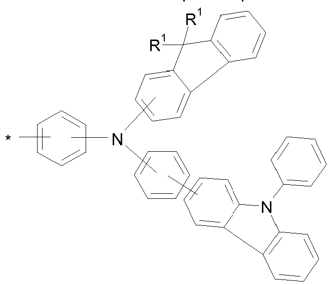 |
| Formel (A-46) | Formel (A-47) |
| 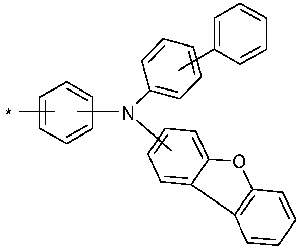 | 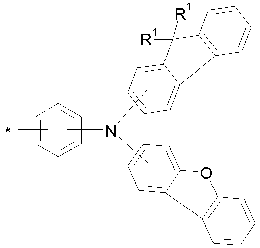 |
| Formel (A-48) | Formel (A-49) |
| 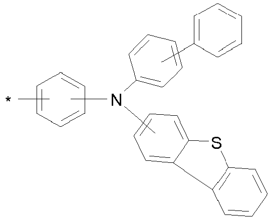 | 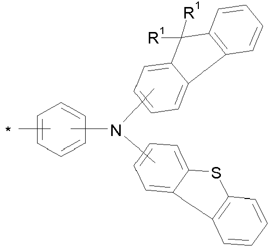 |
| Formel (A-50) | Formel (A-51) |
| 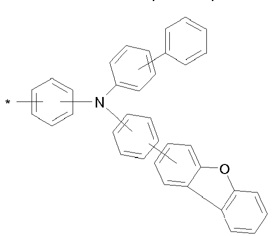 | 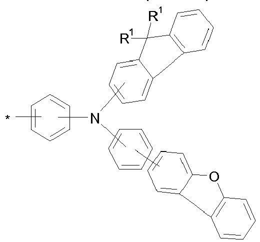 |
| Formel (A-52) | Formel (A-53) |
| 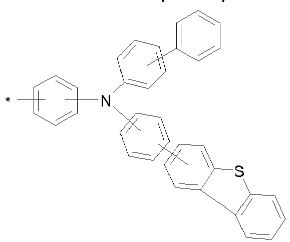 | 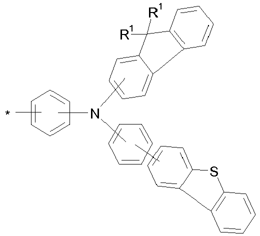 |
| Formel (A-54) | Formel (A-55) |
| 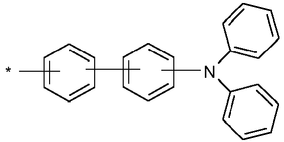 | 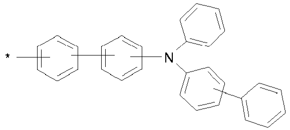 |
| Formel (A-56) | Formel (A-57) |
| 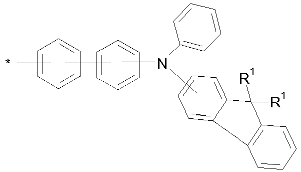 | 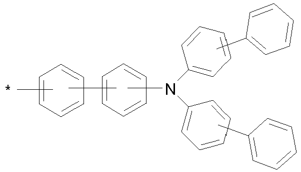 |
| Formel (A-58) | Formel (A-59) |
| 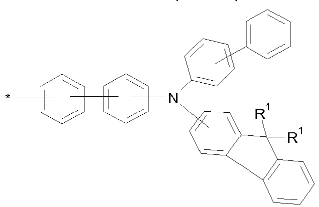 | 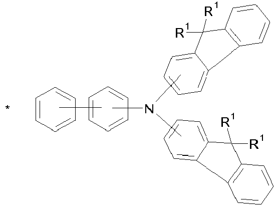 |
| Formel (A-60) | Formel (A-61) |
| 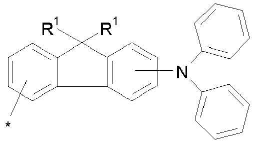 | 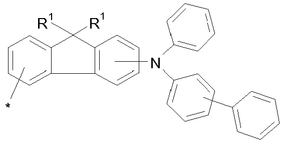 |
| Formel (A-62) | Formel (A-63) |
| 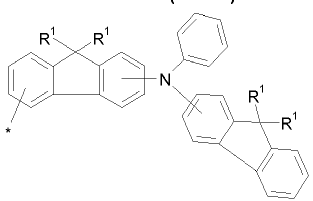 | 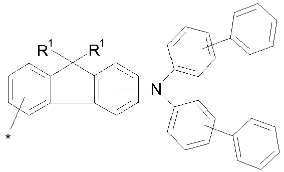 |
| Formel (A-64) | Formel (A-65) |
| 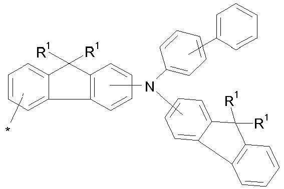 | 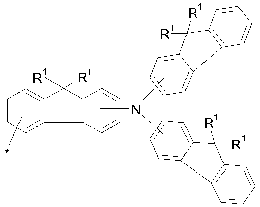 |
| Formel (A-66) | Formel (A-67) |

wobei die Gruppen an allen freien Positionen mit einem oder mehreren Resten R¹, wie oben definiert, substituiert sein können. Bevorzugt ist, dass Reste R¹ dabei definiert sind gemäß ihren bevorzugten Ausführungsformen. Bevorzugt sind die Verbindungen an ihren freien Positionen unsubstituiert.

Eine bevorzugte Ausführungsform der Verbindung der Formel (I) entspricht der folgenden Formel (I-1) 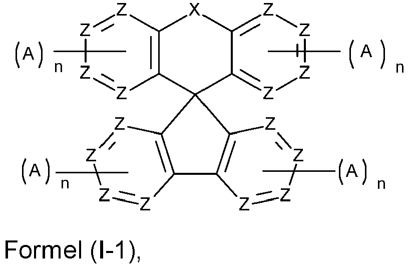 wobei die auftretenden Variablen definiert sind wie oben. Bevorzugt entsprechen die auftretenden Variablen ihren oben genannten bevorzugten Ausführungsformen.

Besonders bevorzugte Ausführungsformen der Verbindungen der Formel (I) entsprechen den folgenden Formeln. Die im Folgenden mit # markierten Formeln sind nicht anspruchsgemäß. 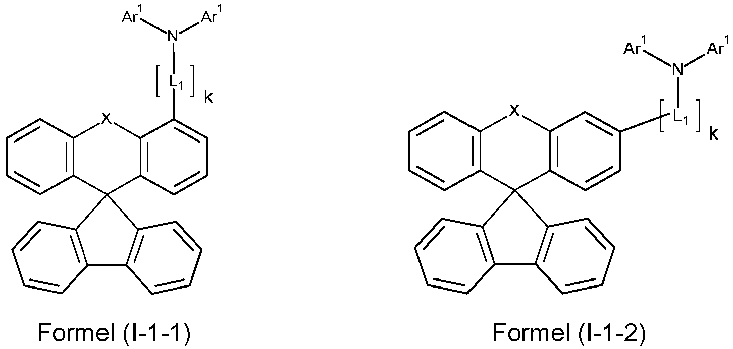 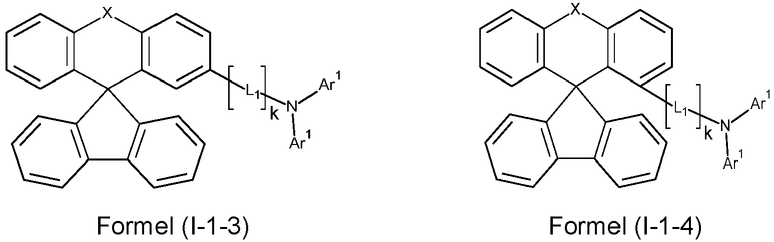 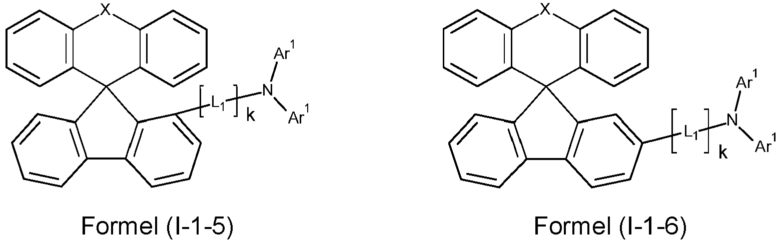 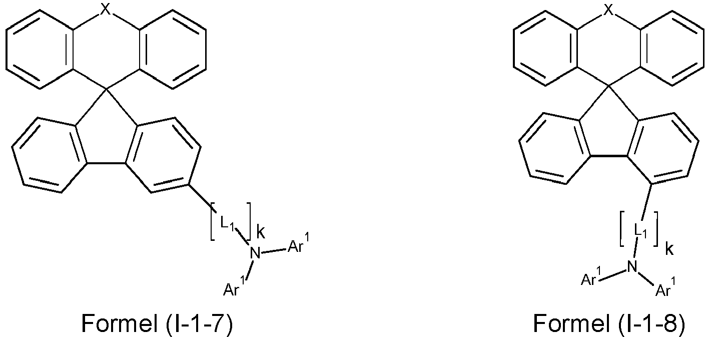 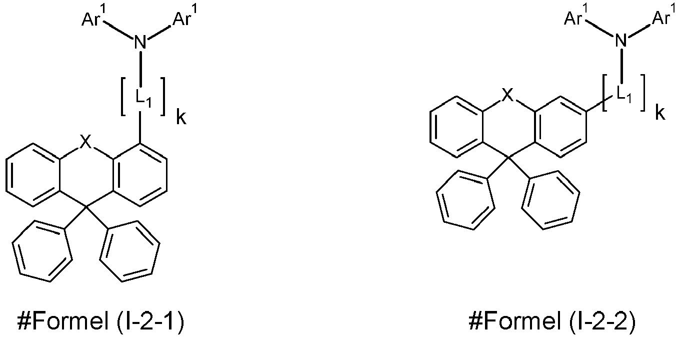 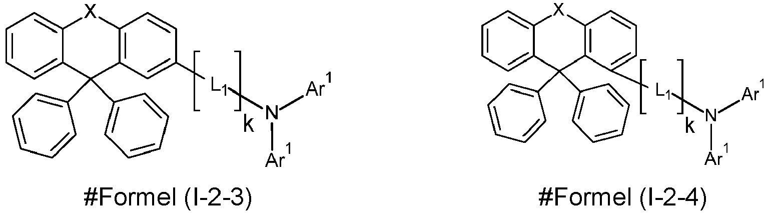 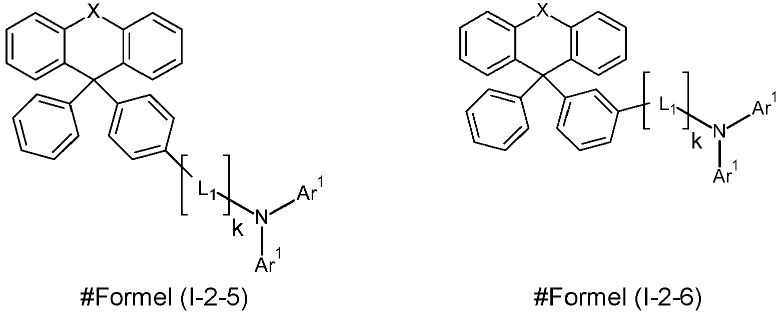 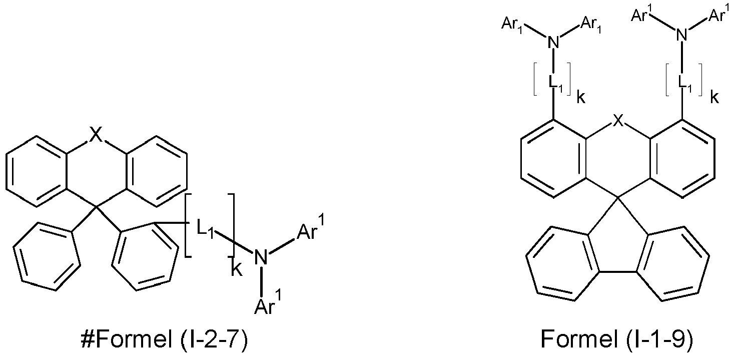 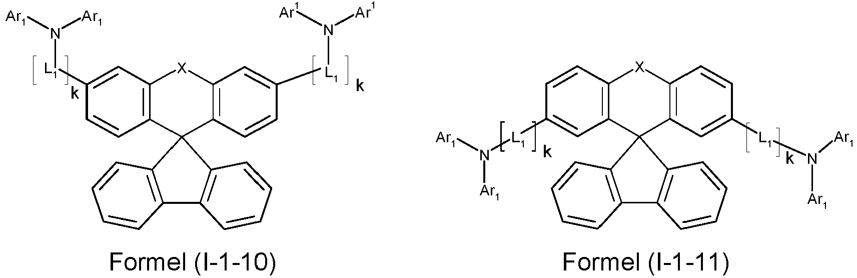 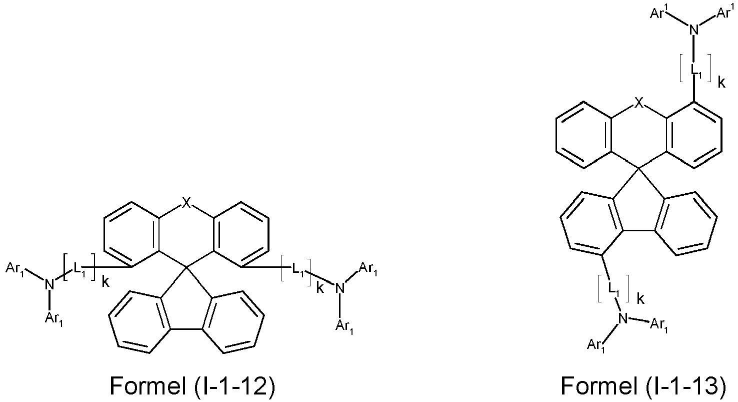 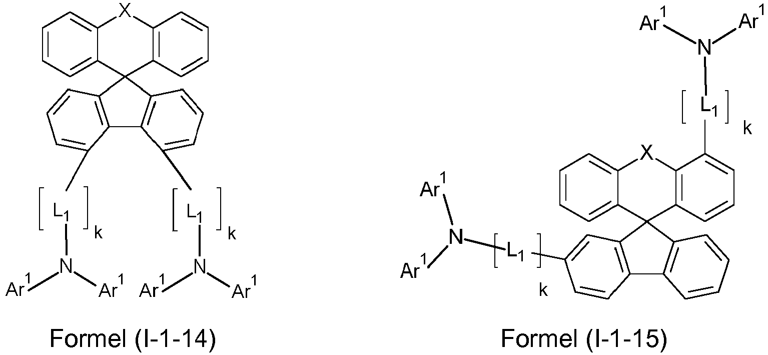 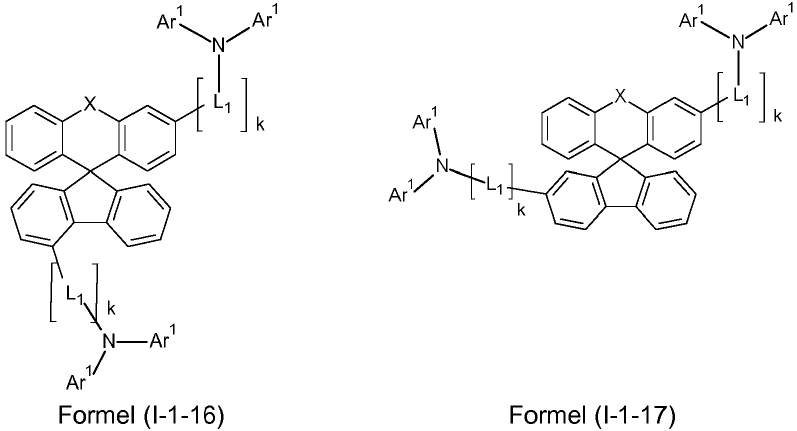 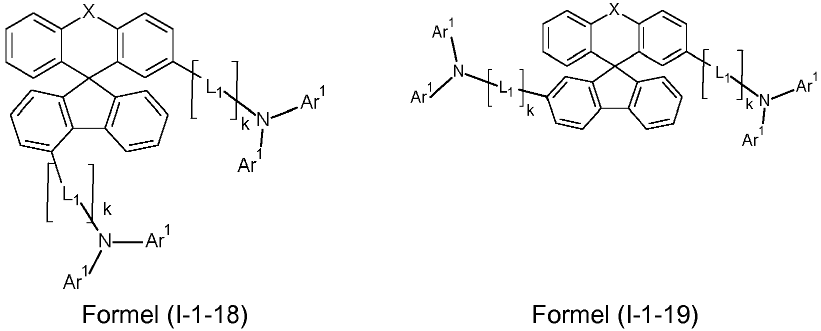 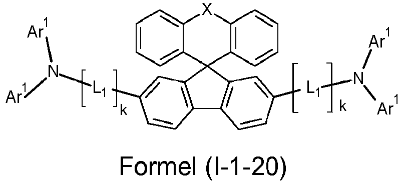
wobei die auftretenden Variablen wie obenstehend definiert sind, und
wobei die Verbindungen an den unsubstituiert gezeichneten Positionen an den Benzolringen jeweils mit Resten R² substituiert sein können. Bevorzugt sind die Verbindungen an den unsubstituiert gezeichneten Positionen an den Benzolringen jeweils unsubstituiert.

Ganz besonders bevorzugt entspricht die Verbindung einer der Formeln (I-1-1) bis (I-1-8), am stärksten bevorzugt einer der Formeln (I-1-1) bis (I-1-3). Für derartige Verbindungen wurden besonders gute Leistungsdaten bei Verwendung in der erfindungsgemäßen Vorrichtung festgestellt.

Bevorzugt ist in den obenstehenden Formeln L₁ gewählt aus aromatischen und heteroaromatischen Ringsystemen mit 6 bis 24 aromatischen Ringatomen, die mit einem oder mehreren Resten R¹ substituiert sein können.

Bevorzugt ist in den obenstehenden Formeln k gleich 0 oder 1. Besonders bevorzugt ist die Kombination der Formeln (I-1-1) bis (I-1-20) mit den bevorzugten Ausführungsformen von Ar¹. Weitere nicht anspruchsgemäße Verbindungen entsprechen der Kombination der Formeln (I-2-1) bis (I-2-7) mit den bevorzugten Ausführungsformen von Ar¹.

Besonders bevorzugte Ausführungsformen der Verbindungen der Formel (I) sind in der folgenden Tabelle abgebildet, wobei die Variablen definiert sind wie oben und bevorzugt keine weiteren Substituenten als die genannten vorliegen.

| | **Grundkörper** | **L¹ oder k=0** | **Ar¹** | **Ar¹** | | **Grundkörper** | **L¹ oder k=0** | **Ar¹** | **Ar¹** |
|---|---|---|---|---|---|---|---|---|---|
| **1** | I-1-2-O | k=0 | Ar¹-1 | Ar¹-1 | **721** | I-1-2-S | k=0 | Ar¹-1 | Ar¹-1 |
| **2** | s.o. | s.o. | s.o. | Ar¹-74 | **722** | s.o. | s.o. | s.o. | Ar¹-74 |
| **3** | s.o. | s.o. | s.o. | Ar¹-132 | **723** | s.o. | s.o. | s.o. | Ar¹-132 |
| **4** | s.o. | s.o. | s.o. | Ar¹-134 | **724** | s.o. | s.o. | s.o. | Ar¹-134 |
| **5** | s.o. | s.o. | s.o. | Ar¹-136 | **725** | s.o. | s.o. | s.o. | Ar¹-136 |
| **6** | s.o. | s.o. | s.o. | Ar¹-137 | **726** | s.o. | s.o. | s.o. | Ar¹-137 |
| **7** | s.o. | s.o. | s.o. | Ar¹-165 | **727** | s.o. | s.o. | s.o. | Ar¹-165 |
| **8** | S.O. | S.O. | S.O. | Ar¹-200 | **728** | S.O. | S.O. | S.O. | Ar¹-200 |
| **9** | S.O. | S.O. | S.O. | Ar¹-201 | **729** | S.O. | S.O. | S.O. | Ar¹-201 |
| **10** | S.O. | S.O. | Ar¹-74 | Ar¹-74 | **730** | S.O. | S.O. | Ar¹-74 | Ar¹-74 |
| **11** | S.O. | S.O. | S.O. | Ar¹-132 | **731** | S.O. | S.O. | S.O. | Ar¹-132 |
| **12** | S.O. | S.O. | S.O. | Ar¹-134 | **732** | S.O. | S.O. | S.O. | Ar¹-134 |
| **13** | S.O. | S.O. | S.O. | Ar¹-136 | **733** | S.O. | S.O. | S.O. | Ar¹-136 |
| **14** | S.O. | S.O. | S.O. | Ar¹-137 | **734** | S.O. | S.O. | S.O. | Ar¹-137 |
| **15** | S.O. | S.O. | S.O. | Ar¹-165 | **735** | S.O. | S.O. | S.O. | Ar¹-165 |
| **16** | S.O. | S.O. | S.O. | Ar¹-200 | **736** | S.O. | S.O. | S.O. | Ar¹-200 |
| **17** | S.O. | S.O. | S.O. | Ar¹-201 | **737** | S.O. | S.O. | S.O. | Ar¹-201 |
| **18** | S.O. | S.O. | Ar¹-132 | Ar¹-132 | **738** | S.O. | S.O. | Ar¹-132 | Ar¹-132 |
| **19** | S.O. | S.O. | S.O. | Ar¹-134 | **739** | S.O. | S.O. | S.O. | Ar¹-134 |
| **20** | S.O. | S.O. | S.O. | Ar¹-136 | **740** | S.O. | S.O. | S.O. | Ar¹-136 |
| **21** | S.O. | S.O. | S.O. | Ar¹-137 | **741** | S.O. | S.O. | S.O. | Ar¹-137 |
| **22** | S.O. | S.O. | S.O. | Ar¹-165 | **742** | S.O. | S.O. | S.O. | Ar¹-165 |
| **23** | S.O. | S.O. | S.O. | Ar¹-200 | **743** | S.O. | S.O. | S.O. | Ar¹-200 |
| **24** | S.O. | S.O. | S.O. | Ar¹-201 | **744** | S.O. | S.O. | S.O. | Ar¹-201 |
| **25** | S.O. | S.O. | Ar¹-134 | Ar¹-134 | **745** | S.O. | S.O. | Ar¹-134 | Ar¹-134 |
| **26** | S.O. | S.O. | S.O. | Ar¹-136 | **746** | S.O. | S.O. | S.O. | Ar¹-136 |
| **27** | S.O. | S.O. | S.O. | Ar¹-137 | **747** | S.O. | S.O. | S.O. | Ar¹-137 |
| **28** | S.O. | S.O. | S.O. | Ar¹-165 | **748** | S.O. | S.O. | S.O. | Ar¹-165 |
| **29** | S.O. | S.O. | S.O. | Ar¹-200 | **749** | S.O. | S.O. | S.O. | Ar¹-200 |
| **30** | S.O. | S.O. | S.O. | Ar¹-201 | **750** | S.O. | S.O. | S.O. | Ar¹-201 |
| **31** | S.O. | S.O. | Ar¹-136 | Ar¹-136 | **751** | S.O. | S.O. | Ar¹-136 | Ar¹-136 |
| **32** | S.O. | S.O. | S.O. | Ar¹-137 | **752** | S.O. | S.O. | S.O. | Ar¹-137 |
| **33** | S.O. | S.O. | S.O. | Ar¹-165 | **753** | S.O. | S.O. | S.O. | Ar¹-165 |
| **34** | S.O. | S.O. | S.O. | Ar¹-200 | **754** | S.O. | S.O. | S.O. | Ar¹-200 |
| **35** | S.O. | S.O. | S.O. | Ar¹-201 | **755** | S.O. | S.O. | S.O. | Ar¹-201 |
| **36** | S.O. | S.O. | Ar¹-137 | Ar¹-137 | **756** | S.O. | S.O. | Ar¹-137 | Ar¹-137 |
| **37** | S.O. | S.O. | S.O. | Ar¹-165 | **757** | S.O. | S.O. | S.O. | Ar¹-165 |
| **38** | S.O. | S.O. | S.O. | Ar¹-200 | **758** | S.O. | S.O. | S.O. | Ar¹-200 |
| **39** | S.O. | S.O. | S.O. | Ar¹-201 | **759** | S.O. | S.O. | S.O. | Ar¹-201 |
| **40** | S.O. | S.O. | Ar¹-165 | Ar¹-165 | **760** | S.O. | S.O. | Ar¹-165 | Ar¹-165 |
| **41** | S.O. | S.O. | S.O. | Ar¹-200 | **761** | S.O. | S.O. | S.O. | Ar¹-200 |
| **42** | S.O. | S.O. | S.O. | Ar¹-201 | **762** | S.O. | S.O. | S.O. | Ar¹-201 |
| **43** | S.O. | S.O. | Ar¹-200 | Ar¹-200 | **763** | S.O. | S.O. | Ar¹-200 | Ar¹-200 |
| **44** | S.O. | S.O. | S.O. | Ar¹-201 | **764** | S.O. | S.O. | S.O. | Ar¹-201 |
| **45** | S.O. | S.O. | Ar¹-201 | Ar¹-201 | **765** | S.O. | S.O. | Ar¹-201 | Ar¹-201 |
| **46** | S.O. | Ar^{L}-1 | Ar¹-1 | Ar¹-1 | **766** | S.O. | Ar^{L}-1 | Ar¹-1 | Ar¹-1 |
| **47** | S.O. | S.O. | S.O. | Ar¹-74 | **767** | S.O. | S.O. | S.O. | Ar¹-74 |
| **48** | S.O. | S.O. | S.O. | Ar¹-132 | **768** | S.O. | S.O. | S.O. | Ar¹-132 |
| **49** | S.O. | S.O. | S.O. | Ar¹-134 | **769** | S.O. | S.O. | S.O. | Ar¹-134 |
| **50** | S.O. | S.O. | S.O. | Ar¹-136 | **770** | S.O. | S.O. | S.O. | Ar¹-136 |
| **51** | S.O. | S.O. | S.O. | Ar¹-137 | **771** | S.O. | S.O. | S.O. | Ar¹-137 |
| **52** | S.O. | S.O. | S.O. | Ar¹-165 | **772** | S.O. | S.O. | S.O. | Ar¹-165 |
| **53** | S.O. | S.O. | S.O. | Ar¹-200 | **773** | S.O. | S.O. | S.O. | Ar¹-200 |
| **54** | S.O. | S.O. | S.O. | Ar¹-201 | **774** | S.O. | S.O. | S.O. | Ar¹-201 |
| **55** | S.O. | S.O. | Ar¹-74 | Ar¹-74 | **775** | S.O. | S.O. | Ar¹-74 | Ar¹-74 |
| **56** | S.O. | S.O. | S.O. | Ar¹-132 | **776** | S.O. | S.O. | S.O. | Ar¹-132 |
| **57** | S.O. | S.O. | S.O. | Ar¹-134 | **777** | S.O. | S.O. | S.O. | Ar¹-134 |
| **58** | S.O. | S.O. | S.O. | Ar¹-136 | **778** | S.O. | S.O. | S.O. | Ar¹-136 |
| **59** | S.O. | S.O. | S.O. | Ar¹-137 | **779** | S.O. | S.O. | S.O. | Ar¹-137 |
| **60** | S.O. | S.O. | S.O. | Ar¹-165 | **780** | S.O. | S.O. | S.O. | Ar¹-165 |
| **61** | S.O. | S.O. | S.O. | Ar¹-200 | **781** | S.O. | S.O. | S.O. | Ar¹-200 |
| **62** | S.O. | S.O. | S.O. | Ar¹-201 | **782** | S.O. | S.O. | S.O. | Ar¹**-**201 |
| **63** | S.O. | S.O. | Ar¹-132 | Ar¹-132 | **783** | S.O. | S.O. | Ar¹-132 | Ar¹-132 |
| **64** | S.O. | S.O. | S.O. | Ar¹-134 | **784** | S.O. | S.O. | S.O. | Ar¹-134 |
| **65** | S.O. | S.O. | S.O. | Ar¹-136 | **785** | S.O. | S.O. | S.O. | Ar¹-136 |
| **66** | S.O. | S.O. | S.O. | Ar¹-137 | **786** | S.O. | S.O. | S.O. | Ar¹-137 |
| **67** | S.O. | S.O. | S.O. | Ar¹-165 | **787** | S.O. | S.O. | S.O. | Ar¹-165 |
| **68** | S.O. | S.O. | S.O. | Ar¹-200 | **788** | S.O. | S.O. | S.O. | Ar¹-200 |
| **69** | S.O. | S.O. | S.O. | Ar¹-201 | **789** | S.O. | S.O. | S.O. | Ar¹-201 |
| **70** | S.O. | S.O. | Ar¹-134 | Ar¹-134 | **790** | S.O. | S.O. | Ar¹-134 | Ar¹-134 |
| **71** | S.O. | S.O. | S.O. | Ar¹-136 | **791** | S.O. | S.O. | S.O. | Ar¹-136 |
| **72** | S.O. | S.O. | S.O. | Ar¹-137 | **792** | S.O. | S.O. | S.O. | Ar¹-137 |
| **73** | S.O. | S.O. | S.O. | Ar¹-165 | **793** | S.O. | S.O. | S.O. | Ar¹-165 |
| **74** | S.O. | S.O. | S.O. | Ar¹-200 | **794** | S.O. | S.O. | S.O. | Ar¹-200 |
| **75** | S.O. | S.O. | S.O. | Ar¹-201 | **795** | S.O. | S.O. | S.O. | Ar¹-201 |
| **76** | S.O. | S.O. | Ar¹-136 | Ar¹-136 | **796** | S.O. | S.O. | Ar¹-136 | Ar¹-136 |
| **77** | S.O. | S.O. | S.O. | Ar¹-137 | **797** | S.O. | S.O. | S.O. | Ar¹-137 |
| **78** | S.O. | S.O. | S.O. | Ar¹-165 | **798** | S.O. | S.O. | S.O. | Ar¹-165 |
| **79** | S.O. | S.O. | S.O. | Ar¹-200 | **799** | S.O. | S.O. | S.O. | Ar¹-200 |
| **80** | S.O. | S.O. | S.O. | Ar¹-201 | **800** | S.O. | S.O. | S.O. | Ar¹-201 |
| **81** | S.O. | S.O. | Ar¹-137 | Ar¹-137 | **801** | S.O. | S.O. | Ar¹-137 | Ar¹-137 |
| **82** | S.O. | S.O. | S.O. | Ar¹-165 | **802** | S.O. | S.O. | S.O. | Ar¹-165 |
| **83** | S.O. | S.O. | S.O. | Ar¹-200 | **803** | S.O. | S.O. | S.O. | Ar¹-200 |
| **84** | S.O. | S.O. | S.O. | Ar¹-201 | **804** | S.O. | S.O. | S.O. | Ar¹-201 |
| **85** | S.O. | S.O. | Ar¹-165 | Ar¹-165 | **805** | S.O. | S.O. | Ar¹-165 | Ar¹-165 |
| **86** | S.O. | S.O. | S.O. | Ar¹-200 | **806** | S.O. | S.O. | S.O. | Ar¹-200 |
| **87** | S.O. | S.O. | S.O. | Ar¹-201 | **807** | S.O. | S.O. | S.O. | Ar¹-201 |
| **88** | S.O. | S.O. | Ar¹-200 | Ar¹-200 | **808** | S.O. | S.O. | Ar¹-200 | Ar¹-200 |
| **89** | S.O. | S.O. | S.O. | Ar¹-201 | **809** | S.O. | S.O. | S.O. | Ar¹-201 |
| **90** | S.O. | S.O. | Ar¹-201 | Ar¹-201 | **810** | S.O. | S.O. | Ar¹-201 | Ar¹-201 |
| **91** | S.O. | Ar^{L}-2 | Ar¹-1 | Ar¹-1 | **811** | S.O. | Ar^{L}-2 | Ar¹-1 | Ar¹-1 |
| **92** | S.O. | S.O. | S.O. | Ar¹-74 | **812** | S.O. | S.O. | S.O. | Ar¹-74 |
| **93** | S.O. | S.O. | S.O. | Ar¹-132 | **813** | S.O. | S.O. | S.O. | Ar¹-132 |
| **94** | S.O. | S.O. | S.O. | Ar¹-134 | **814** | S.O. | S.O. | S.O. | Ar¹-134 |
| **95** | S.O. | S.O. | S.O. | Ar¹-136 | **815** | S.O. | S.O. | S.O. | Ar¹-136 |
| **96** | S.O. | S.O. | S.O. | Ar¹-137 | **816** | S.O. | S.O. | S.O. | Ar¹-137 |
| **97** | S.O. | S.O. | S.O. | Ar¹-165 | **817** | S.O. | S.O. | S.O. | Ar¹-165 |
| **98** | S.O. | S.O. | S.O. | Ar¹-200 | **818** | S.O. | S.O. | S.O. | Ar¹-200 |
| **99** | S.O. | S.O. | S.O. | Ar¹-201 | **819** | S.O. | S.O. | S.O. | Ar¹-201 |
| **100** | S.O. | S.O. | Ar¹-74 | Ar¹-74 | **820** | S.O. | S.O. | Ar¹-74 | Ar¹-74 |
| **101** | S.O. | S.O. | S.O. | Ar¹-132 | **821** | S.O. | S.O. | S.O. | Ar¹-132 |
| **102** | S.O. | S.O. | S.O. | Ar¹-134 | **822** | S.O. | S.O. | S.O. | Ar¹-134 |
| **103** | S.O. | S.O. | S.O. | Ar¹-136 | **823** | S.O. | S.O. | S.O. | Ar¹-136 |
| **104** | S.O. | S.O. | S.O. | Ar¹-137 | **824** | S.O. | S.O. | S.O. | Ar¹-137 |
| **105** | S.O. | S.O. | S.O. | Ar¹-165 | **825** | S.O. | S.O. | S.O. | Ar¹-165 |
| **106** | S.O. | S.O. | S.O. | Ar¹-200 | **826** | S.O. | S.O. | S.O. | Ar¹-200 |
| **107** | S.O. | S.O. | S.O. | Ar¹-201 | **827** | S.O. | S.O. | S.O. | Ar¹-201 |
| **108** | S.O. | S.O. | Ar¹-132 | Ar¹-132 | **828** | S.O. | S.O. | Ar¹-132 | Ar¹-132 |
| **109** | S.O. | S.O. | S.O. | Ar¹-134 | **829** | S.O. | S.O. | S.O. | Ar¹-134 |
| **110** | S.O. | S.O. | S.O. | Ar¹-136 | **830** | S.O. | S.O. | S.O. | Ar¹-136 |
| **111** | S.O. | S.O. | S.O. | Ar¹-137 | **831** | S.O. | S.O. | S.O. | Ar¹-137 |
| **112** | S.O. | S.O. | S.O. | Ar¹-165 | **832** | S.O. | S.O. | S.O. | Ar¹-165 |
| **113** | S.O. | S.O. | S.O. | Ar¹-200 | **833** | S.O. | S.O. | S.O. | Ar¹-200 |
| **114** | S.O. | S.O. | S.O. | Ar¹-201 | **834** | S.O. | S.O. | S.O. | Ar¹-201 |
| **115** | S.O. | S.O. | Ar¹-134 | Ar¹-134 | **835** | S.O. | S.O. | Ar¹-134 | Ar¹-134 |
| **116** | S.O. | S.O. | S.O. | Ar¹-136 | **836** | S.O. | S.O. | S.O. | Ar¹-136 |
| **117** | S.O. | S.O. | S.O. | Ar¹-137 | **837** | S.O. | S.O. | S.O. | Ar¹-137 |
| **118** | S.O. | S.O. | S.O. | Ar¹-165 | **838** | S.O. | S.O. | S.O. | Ar¹-165 |
| **119** | S.O. | S.O. | S.O. | Ar¹-200 | **839** | S.O. | S.O. | S.O. | Ar¹-200 |
| **120** | S.O. | S.O. | S.O. | Ar¹-201 | **840** | S.O. | S.O. | S.O. | Ar¹-201 |
| **121** | S.O. | S.O. | Ar¹-136 | Ar¹-136 | **841** | S.O. | S.O. | Ar¹-136 | Ar¹-136 |
| **122** | S.O. | S.O. | S.O. | Ar¹-137 | **842** | S.O. | S.O. | S.O. | Ar¹-137 |
| **123** | S.O. | S.O. | S.O. | Ar¹-165 | **843** | S.O. | S.O. | S.O. | Ar¹-165 |
| **124** | S.O. | S.O. | S.O. | Ar¹-200 | **844** | S.O. | S.O. | S.O. | Ar¹-200 |
| **125** | S.O. | S.O. | S.O. | Ar¹-201 | **845** | S.O. | S.O. | S.O. | Ar¹-201 |
| **126** | S.O. | S.O. | Ar¹-137 | Ar¹-137 | **846** | S.O. | S.O. | Ar¹-137 | Ar¹-137 |
| **127** | S.O. | S.O. | S.O. | Ar¹-165 | **847** | S.O. | S.O. | S.O. | Ar¹-165 |
| **128** | S.O. | S.O. | S.O. | Ar¹-200 | **848** | S.O. | S.O. | S.O. | Ar¹-200 |
| **129** | S.O. | S.O. | S.O. | Ar¹-201 | **849** | S.O. | S.O. | S.O. | Ar¹-201 |
| **130** | S.O. | S.O. | Ar¹-165 | Ar¹-165 | **850** | S.O. | S.O. | Ar¹-165 | Ar¹-165 |
| **131** | S.O. | S.O. | S.O. | Ar¹-200 | **851** | S.O. | S.O. | S.O. | Ar¹-200 |
| **132** | S.O. | S.O. | S.O. | Ar¹-201 | **852** | S.O. | S.O. | S.O. | Ar¹-201 |
| **133** | S.O. | S.O. | Ar¹-200 | Ar¹-200 | **853** | S.O. | S.O. | Ar¹-200 | Ar¹-200 |
| **134** | S.O. | S.O. | S.O. | Ar¹-201 | **854** | S.O. | S.O. | S.O. | Ar¹-201 |
| **135** | S.O. | S.O. | Ar¹-201 | Ar¹-201 | **855** | S.O. | S.O. | Ar¹-201 | Ar¹-201 |
| **136** | S.O. | Ar^{L}-3 | Ar¹-1 | Ar¹-1 | **856** | S.O. | Ar^{L}-3 | Ar¹-1 | Ar¹-1 |
| **137** | S.O. | S.O. | S.O. | Ar¹-74 | **857** | S.O. | S.O. | S.O. | Ar¹-74 |
| **138** | S.O. | S.O. | S.O. | Ar¹-132 | **858** | S.O. | S.O. | S.O. | Ar¹-132 |
| **139** | S.O. | S.O. | S.O. | Ar¹-134 | **859** | S.O. | S.O. | S.O. | Ar¹-134 |
| **140** | S.O. | S.O. | S.O. | Ar¹-136 | **860** | S.O. | S.O. | S.O. | Ar¹-136 |
| **141** | S.O. | S.O. | S.O. | Ar¹-137 | **861** | S.O. | S.O. | S.O. | Ar¹-137 |
| **142** | S.O. | S.O. | S.O. | Ar¹-165 | **862** | S.O. | S.O. | S.O. | Ar¹-165 |
| **143** | S.O. | S.O. | S.O. | Ar¹-200 | **863** | S.O. | S.O. | S.O. | Ar¹-200 |
| **144** | S.O. | S.O. | S.O. | Ar¹-201 | **864** | S.O. | S.O. | S.O. | Ar¹-201 |
| **145** | S.O. | S.O. | Ar¹-74 | Ar¹-74 | **865** | S.O. | S.O. | Ar¹-74 | Ar¹-74 |
| **146** | S.O. | S.O. | S.O. | Ar¹-132 | **866** | S.O. | S.O. | S.O. | Ar¹-132 |
| **147** | S.O. | S.O. | S.O. | Ar¹-134 | **867** | S.O. | S.O. | S.O. | Ar¹-134 |
| **148** | S.O. | S.O. | S.O. | Ar¹-136 | **868** | S.O. | S.O. | S.O. | Ar¹-136 |
| **149** | S.O. | S.O. | S.O. | Ar¹-137 | **869** | S.O. | S.O. | S.O. | Ar¹-137 |
| **150** | S.O. | S.O. | S.O. | Ar¹-165 | **870** | S.O. | S.O. | S.O. | Ar¹-165 |
| **151** | S.O. | S.O. | S.O. | Ar¹-200 | **871** | S.O. | S.O. | S.O. | Ar¹-200 |
| **152** | S.O. | S.O. | S.O. | Ar¹-201 | **872** | S.O. | S.O. | S.O. | Ar¹-201 |
| **153** | S.O. | S.O. | Ar¹-132 | Ar¹-132 | **873** | S.O. | S.O. | Ar¹-132 | Ar¹-132 |
| **154** | S.O. | S.O. | S.O. | Ar¹-134 | **874** | S.O. | S.O. | S.O. | Ar¹-134 |
| **155** | S.O. | S.O. | S.O. | Ar¹-136 | **875** | S.O. | S.O. | S.O. | Ar¹-136 |
| **156** | S.O. | S.O. | S.O. | Ar¹-137 | **876** | S.O. | S.O. | S.O. | Ar¹-137 |
| **157** | S.O. | S.O. | S.O. | Ar¹-165 | **877** | S.O. | S.O. | S.O. | Ar¹-165 |
| **158** | S.O. | S.O. | S.O. | Ar¹-200 | **878** | S.O. | S.O. | S.O. | Ar¹-200 |
| **159** | S.O. | S.O. | S.O. | Ar¹-201 | **879** | S.O. | S.O. | S.O. | Ar¹-201 |
| **160** | S.O. | S.O. | Ar¹-134 | Ar¹-134 | **880** | S.O. | S.O. | Ar¹-134 | Ar¹-134 |
| **161** | S.O. | S.O. | S.O. | Ar¹-136 | **881** | S.O. | S.O. | S.O. | Ar¹-136 |
| **162** | S.O. | S.O. | S.O. | Ar¹-137 | **882** | S.O. | S.O. | S.O. | Ar¹-137 |
| **163** | S.O. | S.O. | S.O. | Ar¹-165 | **883** | S.O. | S.O. | S.O. | Ar¹-165 |
| **164** | S.O. | S.O. | S.O. | Ar¹-200 | **884** | S.O. | S.O. | S.O. | Ar¹-200 |
| **165** | S.O. | S.O. | S.O. | Ar¹-201 | **885** | S.O. | S.O. | S.O. | Ar¹-201 |
| **166** | S.O. | S.O. | Ar¹-136 | Ar¹-136 | **886** | S.O. | S.O. | Ar¹-136 | Ar¹-136 |
| **167** | S.O. | S.O. | S.O. | Ar¹-137 | **887** | S.O. | S.O. | S.O. | Ar¹-137 |
| **168** | S.O. | S.O. | S.O. | Ar¹-165 | **888** | S.O. | S.O. | S.O. | Ar¹-165 |
| **169** | S.O. | S.O. | S.O. | Ar¹-200 | **889** | S.O. | S.O. | S.O. | Ar¹-200 |
| **170** | S.O. | S.O. | S.O. | Ar¹-201 | **890** | S.O. | S.O. | S.O. | Ar¹-201 |
| **171** | S.O. | S.O. | Ar¹-137 | Ar¹-137 | **891** | S.O. | S.O. | Ar¹-137 | Ar¹-137 |
| **172** | S.O. | S.O. | S.O. | Ar¹-165 | **892** | S.O. | S.O. | S.O. | Ar¹-165 |
| **173** | S.O. | S.O. | S.O. | Ar¹-200 | **893** | S.O. | S.O. | S.O. | Ar¹-200 |
| **174** | S.O. | S.O. | S.O. | Ar¹-201 | **894** | S.O. | S.O. | S.O. | Ar¹-201 |
| **175** | S.O. | S.O. | Ar¹-165 | Ar¹-165 | **895** | S.O. | S.O. | Ar¹-165 | Ar¹-165 |
| **176** | S.O. | S.O. | S.O. | Ar¹-200 | **896** | S.O. | S.O. | S.O. | Ar¹-200 |
| **177** | S.O. | S.O. | S.O. | Ar¹-201 | **897** | S.O. | S.O. | S.O. | Ar¹-201 |
| **178** | S.O. | S.O. | Ar¹-200 | Ar¹-200 | **898** | S.O. | S.O. | Ar¹-200 | Ar¹-200 |
| **179** | S.O. | S.O. | S.O. | Ar¹-201 | **899** | S.O. | S.O. | S.O. | Ar¹-201 |
| **180** | S.O. | S.O. | Ar¹-201 | Ar¹-201 | **900** | S.O. | S.O. | Ar¹-201 | Ar¹-201 |
| **181** | I-1-4-O | k=0 | Ar¹-1 | Ar¹-1 | **901** | I-1-4-S | k=0 | Ar¹-1 | Ar¹-1 |
| **182** | S.O. | S.O. | S.O. | Ar¹-74 | **902** | S.O. | S.O. | S.O. | Ar¹-74 |
| **183** | S.O. | S.O. | S.O. | Ar¹-132 | **903** | S.O. | S.O. | S.O. | Ar¹-132 |
| **184** | S.O. | S.O. | S.O. | Ar¹-134 | **904** | S.O. | S.O. | S.O. | Ar¹-134 |
| **185** | S.O. | S.O. | S.O. | Ar¹-136 | **905** | S.O. | S.O. | S.O. | Ar¹-136 |
| **186** | S.O. | S.O. | S.O. | Ar¹-137 | **906** | S.O. | S.O. | S.O. | Ar¹-137 |
| **187** | S.O. | S.O. | S.O. | Ar¹-165 | **907** | S.O. | S.O. | S.O. | Ar¹-165 |
| **188** | S.O. | S.O. | S.O. | Ar¹-200 | **908** | S.O. | S.O. | S.O. | Ar¹-200 |
| **189** | S.O. | S.O. | S.O. | Ar¹-201 | **909** | S.O. | S.O. | S.O. | Ar¹-201 |
| **190** | S.O. | S.O. | Ar¹-74 | Ar¹-74 | **910** | S.O. | S.O. | Ar¹-74 | Ar¹-74 |
| **191** | S.O. | S.O. | S.O. | Ar¹-132 | **911** | S.O. | S.O. | S.O. | Ar¹-132 |
| **192** | S.O. | S.O. | S.O. | Ar¹-134 | **912** | S.O. | S.O. | S.O. | Ar¹-134 |
| **193** | S.O. | S.O. | S.O. | Ar¹-136 | **913** | S.O. | S.O. | S.O. | Ar¹-136 |
| **194** | S.O. | S.O. | S.O. | Ar¹-137 | **914** | S.O. | S.O. | S.O. | Ar¹-137 |
| **195** | S.O. | S.O. | S.O. | Ar¹-165 | **915** | S.O. | S.O. | S.O. | Ar¹-165 |
| **196** | S.O. | S.O. | S.O. | Ar¹-200 | **916** | S.O. | S.O. | S.O. | Ar¹-200 |
| **197** | S.O. | S.O. | S.O. | Ar¹-201 | **917** | S.O. | S.O. | S.O. | Ar¹-201 |
| **198** | S.O. | S.O. | Ar¹-132 | Ar¹-132 | **918** | S.O. | S.O. | Ar¹-132 | Ar¹-132 |
| **199** | S.O. | S.O. | S.O. | Ar¹-134 | **919** | S.O. | S.O. | S.O. | Ar¹-134 |
| **200** | S.O. | S.O. | S.O. | Ar¹-136 | **920** | S.O. | S.O. | S.O. | Ar¹-136 |
| **201** | S.O. | S.O. | S.O. | Ar¹-137 | **921** | S.O. | S.O. | S.O. | Ar¹-137 |
| **202** | S.O. | S.O. | S.O. | Ar¹-165 | **922** | S.O. | S.O. | S.O. | Ar¹-165 |
| **203** | S.O. | S.O. | S.O. | Ar¹-200 | **923** | S.O. | S.O. | S.O. | Ar¹-200 |
| **204** | S.O. | S.O. | S.O. | Ar¹-201 | **924** | S.O. | S.O. | S.O. | Ar¹-201 |
| **205** | S.O. | S.O. | Ar¹-134 | Ar¹-134 | **925** | S.O. | S.O. | Ar¹-134 | Ar¹-134 |
| **206** | S.O. | S.O. | S.O. | Ar¹-136 | **926** | S.O. | S.O. | S.O. | Ar¹-136 |
| **207** | S.O. | S.O. | S.O. | Ar¹-137 | **927** | S.O. | S.O. | S.O. | Ar¹-137 |
| **208** | S.O. | S.O. | S.O. | Ar¹-165 | **928** | S.O. | S.O. | S.O. | Ar¹-165 |
| **209** | S.O. | S.O. | S.O. | Ar¹-200 | **929** | S.O. | S.O. | S.O. | Ar¹-200 |
| **210** | S.O. | S.O. | S.O. | Ar¹-201 | **930** | S.O. | S.O. | S.O. | Ar¹-201 |
| **211** | S.O. | S.O. | Ar¹-136 | Ar¹-136 | **931** | S.O. | S.O. | Ar¹-136 | Ar¹-136 |
| **212** | S.O. | S.O. | S.O. | Ar¹-137 | **932** | S.O. | S.O. | S.O. | Ar¹-137 |
| **213** | S.O. | S.O. | S.O. | Ar¹-165 | **933** | S.O. | S.O. | S.O. | Ar¹-165 |
| **214** | S.O. | S.O. | S.O. | Ar¹-200 | **934** | S.O. | S.O. | S.O. | Ar¹-200 |
| **215** | S.O. | S.O. | S.O. | Ar¹-201 | **935** | S.O. | S.O. | S.O. | Ar¹-201 |
| **216** | S.O. | S.O. | Ar¹-137 | Ar¹-137 | **936** | S.O. | S.O. | Ar¹-137 | Ar¹-137 |
| **217** | S.O. | S.O. | S.O. | Ar¹-165 | **937** | S.O. | S.O. | S.O. | Ar¹-165 |
| **218** | S.O. | S.O. | S.O. | Ar¹-200 | **938** | S.O. | S.O. | S.O. | Ar¹-200 |
| **219** | S.O. | S.O. | S.O. | Ar¹-201 | **939** | S.O. | S.O. | S.O. | Ar¹-201 |
| **220** | S.O. | S.O. | Ar¹-165 | Ar¹-165 | **940** | S.O. | S.O. | Ar¹-165 | Ar¹-165 |
| **221** | S.O. | S.O. | S.O. | Ar¹-200 | **941** | S.O. | S.O. | S.O. | Ar¹-200 |
| **222** | S.O. | S.O. | S.O. | Ar¹-201 | **942** | S.O. | S.O. | S.O. | Ar¹-201 |
| **223** | S.O. | S.O. | Ar¹-200 | Ar¹-200 | **943** | S.O. | S.O. | Ar¹-200 | Ar¹-200 |
| **224** | S.O. | S.O. | S.O. | Ar¹-201 | **944** | S.O. | S.O. | S.O. | Ar¹-201 |
| **225** | S.O. | S.O. | Ar¹-201 | Ar¹-201 | **945** | S.O. | S.O. | Ar¹-201 | Ar¹-201 |
| **226** | S.O. | Ar^{L}-1 | Ar¹-1 | Ar¹-1 | **946** | S.O. | Ar^{L}-1 | Ar¹-1 | Ar¹-1 |
| **227** | S.O. | S.O. | S.O. | Ar¹-74 | **947** | S.O. | S.O. | S.O. | Ar¹-74 |
| **228** | S.O. | S.O. | S.O. | Ar¹-132 | **948** | S.O. | S.O. | S.O. | Ar¹-132 |
| **229** | S.O. | S.O. | S.O. | Ar¹-134 | **949** | S.O. | S.O. | S.O. | Ar¹-134 |
| **230** | S.O. | S.O. | S.O. | Ar¹-136 | **950** | S.O. | S.O. | S.O. | Ar¹-136 |
| **231** | S.O. | S.O. | S.O. | Ar¹-137 | **951** | S.O. | S.O. | S.O. | Ar¹-137 |
| **232** | S.O. | S.O. | S.O. | Ar¹-165 | **952** | S.O. | S.O. | S.O. | Ar¹-165 |
| **233** | S.O. | S.O. | S.O. | Ar¹-200 | **953** | S.O. | S.O. | S.O. | Ar¹-200 |
| **234** | S.O. | S.O. | S.O. | Ar¹-201 | **954** | S.O. | S.O. | S.O. | Ar¹-201 |
| **235** | S.O. | S.O. | Ar¹-74 | Ar¹-74 | **955** | S.O. | S.O. | Ar¹-74 | Ar¹-74 |
| **236** | S.O. | S.O. | S.O. | Ar¹-132 | **956** | S.O. | S.O. | S.O. | Ar¹-132 |
| **237** | S.O. | S.O. | S.O. | Ar¹-134 | **957** | S.O. | S.O. | S.O. | Ar¹-134 |
| **238** | S.O. | S.O. | S.O. | Ar¹-136 | **958** | S.O. | S.O. | S.O. | Ar¹-136 |
| **239** | S.O. | S.O. | S.O. | Ar¹-137 | **959** | S.O. | S.O. | S.O. | Ar¹-137 |
| **240** | S.O. | S.O. | S.O. | Ar¹-165 | **960** | S.O. | S.O. | S.O. | Ar¹-165 |
| **241** | S.O. | S.O. | S.O. | Ar¹-200 | **961** | S.O. | S.O. | S.O. | Ar¹-200 |
| **242** | S.O. | S.O. | S.O. | Ar¹-201 | **962** | S.O. | S.O. | S.O. | Ar¹-201 |
| **243** | S.O. | S.O. | Ar¹-132 | Ar¹-132 | **963** | S.O. | S.O. | Ar¹-132 | Ar¹-132 |
| **244** | S.O. | S.O. | S.O. | Ar¹-134 | **964** | S.O. | S.O. | S.O. | Ar¹-134 |
| **245** | S.O. | S.O. | S.O. | Ar¹-136 | **965** | S.O. | S.O. | S.O. | Ar¹-136 |
| **246** | S.O. | S.O. | S.O. | Ar¹-137 | **966** | S.O. | S.O. | S.O. | Ar¹-137 |
| **247** | S.O. | S.O. | S.O. | Ar¹-165 | **967** | S.O. | S.O. | S.O. | Ar¹-165 |
| **248** | S.O. | S.O. | S.O. | Ar¹-200 | **968** | S.O. | S.O. | S.O. | Ar¹-200 |
| **249** | S.O. | S.O. | S.O. | Ar¹-201 | **969** | S.O. | S.O. | S.O. | Ar¹-201 |
| **250** | S.O. | S.O. | Ar¹-134 | Ar¹-134 | **970** | S.O. | S.O. | Ar¹-134 | Ar¹-134 |
| **251** | S.O. | S.O. | S.O. | Ar¹-136 | **971** | S.O. | S.O. | S.O. | Ar¹-136 |
| **252** | S.O. | S.O. | S.O. | Ar¹-137 | **972** | S.O. | S.O. | S.O. | Ar¹-137 |
| **253** | S.O. | S.O. | S.O. | Ar¹-165 | **973** | S.O. | S.O. | S.O. | Ar¹-165 |
| **254** | S.O. | S.O. | S.O. | Ar¹-200 | **974** | S.O. | S.O. | S.O. | Ar¹-200 |
| **255** | S.O. | S.O. | S.O. | Ar¹-201 | **975** | S.O. | S.O. | S.O. | Ar¹-201 |
| **256** | S.O. | S.O. | Ar¹-136 | Ar¹-136 | **976** | S.O. | S.O. | Ar¹-136 | Ar¹-136 |
| **257** | S.O. | S.O. | S.O. | Ar¹-137 | **977** | S.O. | S.O. | S.O. | Ar¹-137 |
| **258** | S.O. | S.O. | S.O. | Ar¹-165 | **978** | S.O. | S.O. | S.O. | Ar¹-165 |
| **259** | S.O. | S.O. | S.O. | Ar¹-200 | **979** | S.O. | S.O. | S.O. | Ar¹-200 |
| **260** | S.O. | S.O. | S.O. | Ar¹-201 | **980** | S.O. | S.O. | S.O. | Ar¹-201 |
| **261** | S.O. | S.O. | Ar¹-137 | Ar¹-137 | **981** | S.O. | S.O. | Ar¹-137 | Ar¹-137 |
| **262** | S.O. | S.O. | S.O. | Ar¹-165 | **982** | S.O. | S.O. | S.O. | Ar¹-165 |
| **263** | S.O. | S.O. | S.O. | Ar¹-200 | **983** | S.O. | S.O. | S.O. | Ar¹-200 |
| **264** | S.O. | S.O. | S.O. | Ar¹-201 | **984** | S.O. | S.O. | S.O. | Ar¹-201 |
| **265** | S.O. | S.O. | Ar¹-165 | Ar¹-165 | **985** | S.O. | S.O. | Ar¹-165 | Ar¹-165 |
| **266** | S.O. | S.O. | S.O. | Ar¹-200 | **986** | S.O. | S.O. | S.O. | Ar¹-200 |
| **267** | S.O. | S.O. | S.O. | Ar¹-201 | **987** | S.O. | S.O. | S.O. | Ar¹-201 |
| **268** | S.O. | S.O. | Ar¹-200 | Ar¹-200 | **988** | S.O. | S.O. | Ar¹-200 | Ar¹-200 |
| **269** | S.O. | S.O. | S.O. | Ar¹-201 | **989** | S.O. | S.O. | S.O. | Ar¹-201 |
| **270** | S.O. | S.O. | Ar¹-201 | Ar¹-201 | **990** | S.O. | S.O. | Ar¹-201 | Ar¹-201 |
| **271** | S.O. | Ar^{L}-2 | Ar¹-1 | Ar¹-1 | **991** | S.O. | Ar^{L}-2 | Ar¹-1 | Ar¹-1 |
| **272** | S.O. | S.O. | S.O. | Ar¹-74 | **992** | S.O. | S.O. | S.O. | Ar¹-74 |
| **273** | S.O. | S.O. | S.O. | Ar¹-132 | **993** | S.O. | S.O. | S.O. | Ar¹-132 |
| **274** | S.O. | S.O. | S.O. | Ar¹-134 | **994** | S.O. | S.O. | S.O. | Ar¹-134 |
| **275** | S.O. | S.O. | S.O. | Ar¹-136 | **995** | S.O. | S.O. | S.O. | Ar¹-136 |
| **276** | S.O. | S.O. | S.O. | Ar¹-137 | **996** | S.O. | S.O. | S.O. | Ar¹-137 |
| **277** | S.O. | S.O. | S.O. | Ar¹-165 | **997** | S.O. | S.O. | S.O. | Ar¹-165 |
| **278** | S.O. | S.O. | S.O. | Ar¹-200 | **998** | S.O. | S.O. | S.O. | Ar¹-200 |
| **279** | S.O. | S.O. | S.O. | Ar¹-201 | **999** | S.O. | S.O. | S.O. | Ar¹-201 |
| **280** | S.O. | S.O. | Ar¹-74 | Ar¹-74 | **1000** | S.O. | S.O. | Ar¹-74 | Ar¹-74 |
| **281** | S.O. | S.O. | S.O. | Ar¹-132 | **1001** | S.O. | S.O. | S.O. | Ar¹-132 |
| **282** | S.O. | S.O. | S.O. | Ar¹-134 | **1002** | S.O. | S.O. | S.O. | Ar¹-134 |
| **283** | S.O. | S.O. | S.O. | Ar¹-136 | **1003** | S.O. | S.O. | S.O. | Ar¹-136 |
| **284** | S.O. | S.O. | S.O. | Ar¹-137 | **1004** | S.O. | S.O. | S.O. | Ar¹-137 |
| **285** | S.O. | S.O. | S.O. | Ar¹-165 | **1005** | S.O. | S.O. | S.O. | Ar¹-165 |
| **286** | S.O. | S.O. | S.O. | Ar¹-200 | **1006** | S.O. | S.O. | S.O. | Ar¹-200 |
| **287** | S.O. | S.O. | S.O. | Ar¹-201 | **1007** | S.O. | S.O. | S.O. | Ar¹-201 |
| **288** | S.O. | S.O. | Ar¹-132 | Ar¹-132 | **1008** | S.O. | S.O. | Ar¹-132 | Ar¹-132 |
| **289** | S.O. | S.O. | S.O. | Ar¹-134 | **1009** | S.O. | S.O. | S.O. | Ar¹-134 |
| **290** | S.O. | S.O. | S.O. | Ar¹-136 | **1010** | S.O. | S.O. | S.O. | Ar¹-136 |
| **291** | S.O. | S.O. | S.O. | Ar¹-137 | **1011** | S.O. | S.O. | S.O. | Ar¹-137 |
| **292** | S.O. | S.O. | S.O. | Ar¹-165 | **1012** | S.O. | S.O. | S.O. | Ar¹-165 |
| **293** | S.O. | S.O. | S.O. | Ar¹-200 | **1013** | S.O. | S.O. | S.O. | Ar¹-200 |
| **294** | S.O. | S.O. | S.O. | Ar¹-201 | **1014** | S.O. | S.O. | S.O. | Ar¹-201 |
| **295** | S.O. | S.O. | Ar¹-134 | Ar¹-134 | **1015** | S.O. | S.O. | Ar¹-134 | Ar¹-134 |
| **296** | S.O. | S.O. | S.O. | Ar¹-136 | **1016** | S.O. | S.O. | S.O. | Ar¹-136 |
| **297** | S.O. | S.O. | S.O. | Ar¹-137 | **1017** | S.O. | S.O. | S.O. | Ar¹-137 |
| **298** | S.O. | S.O. | S.O. | Ar¹-165 | **1018** | S.O. | S.O. | S.O. | Ar¹-165 |
| **299** | S.O. | S.O. | S.O. | Ar¹-200 | **1019** | S.O. | S.O. | S.O. | Ar¹-200 |
| **300** | S.O. | S.O. | S.O. | Ar¹-201 | **1020** | S.O. | S.O. | S.O. | Ar¹-201 |
| **301** | S.O. | S.O. | Ar¹-136 | Ar¹-136 | **1021** | S.O. | S.O. | Ar¹-136 | Ar¹-136 |
| **302** | S.O. | S.O. | S.O. | Ar¹-137 | **1022** | S.O. | S.O. | S.O. | Ar¹-137 |
| **303** | S.O. | S.O. | S.O. | Ar¹-165 | **1023** | S.O. | S.O. | S.O. | Ar¹-165 |
| **304** | S.O. | S.O. | S.O. | Ar¹-200 | **1024** | S.O. | S.O. | S.O. | Ar¹-200 |
| **305** | S.O. | S.O. | S.O. | Ar¹-201 | **1025** | S.O. | S.O. | S.O. | Ar¹-201 |
| **306** | S.O. | S.O. | Ar¹-137 | Ar¹-137 | **1026** | S.O. | S.O. | Ar¹-137 | Ar¹-137 |
| **307** | S.O. | S.O. | S.O. | Ar¹-165 | **1027** | S.O. | S.O. | S.O. | Ar¹-165 |
| **308** | S.O. | S.O. | S.O. | Ar¹-200 | **1028** | S.O. | S.O. | S.O. | Ar¹-200 |
| **309** | S.O. | S.O. | S.O. | Ar¹-201 | **1029** | S.O. | S.O. | S.O. | Ar¹-201 |
| **310** | S.O. | S.O. | Ar¹-165 | Ar¹-165 | **1030** | S.O. | S.O. | Ar¹-165 | Ar¹-165 |
| **311** | S.O. | S.O. | S.O. | Ar¹-200 | **1031** | S.O. | S.O. | S.O. | Ar¹-200 |
| **312** | S.O. | S.O. | S.O. | Ar¹-201 | **1032** | S.O. | S.O. | S.O. | Ar¹-201 |
| **313** | S.O. | S.O. | Ar¹-200 | Ar¹-200 | **1033** | S.O. | S.O. | Ar¹-200 | Ar¹-200 |
| **314** | S.O. | S.O. | S.O. | Ar¹-201 | **1034** | S.O. | S.O. | S.O. | Ar¹-201 |
| **315** | S.O. | S.O. | Ar¹-201 | Ar¹-201 | **1035** | S.O. | S.O. | Ar¹-201 | Ar¹-201 |
| **316** | S.O. | Ar^{L}-3 | Ar¹-1 | Ar¹-1 | **1036** | S.O. | Ar^{L}-3 | Ar¹-1 | Ar¹-1 |
| **317** | S.O. | S.O. | S.O. | Ar¹-74 | **1037** | S.O. | S.O. | S.O. | Ar¹-74 |
| **318** | S.O. | S.O. | S.O. | Ar¹-132 | **1038** | S.O. | S.O. | S.O. | Ar¹-132 |
| **319** | S.O. | S.O. | S.O. | Ar¹-134 | **1039** | S.O. | S.O. | S.O. | Ar¹-134 |
| **320** | S.O. | S.O. | S.O. | Ar¹-136 | **1040** | S.O. | S.O. | S.O. | Ar¹-136 |
| **321** | S.O. | S.O. | S.O. | Ar¹-137 | **1041** | S.O. | S.O. | S.O. | Ar¹-137 |
| **322** | S.O. | S.O. | S.O. | Ar¹-165 | **1042** | S.O. | S.O. | S.O. | Ar¹-165 |
| **323** | S.O. | S.O. | S.O. | Ar¹-200 | **1043** | S.O. | S.O. | S.O. | Ar¹-200 |
| **324** | S.O. | S.O. | S.O. | Ar¹-201 | **1044** | S.O. | S.O. | S.O. | Ar¹-201 |
| **325** | S.O. | S.O. | Ar¹-74 | Ar¹-74 | **1045** | S.O. | S.O. | Ar¹-74 | Ar¹-74 |
| **326** | S.O. | S.O. | S.O. | Ar¹-132 | **1046** | S.O. | S.O. | S.O. | Ar¹-132 |
| **327** | S.O. | S.O. | S.O. | Ar¹-134 | **1047** | S.O. | S.O. | S.O. | Ar¹-134 |
| **328** | S.O. | S.O. | S.O. | Ar¹-136 | **1048** | S.O. | S.O. | S.O. | Ar¹-136 |
| **329** | S.O. | S.O. | S.O. | Ar¹-137 | **1049** | S.O. | S.O. | S.O. | Ar¹-137 |
| **330** | S.O. | S.O. | S.O. | Ar¹-165 | **1050** | S.O. | S.O. | S.O. | Ar¹-165 |
| **331** | S.O. | S.O. | S.O. | Ar¹-200 | **1051** | S.O. | S.O. | S.O. | Ar¹-200 |
| **332** | S.O. | S.O. | S.O. | Ar¹-201 | **1052** | S.O. | S.O. | S.O. | Ar¹-201 |
| **333** | S.O. | S.O. | Ar¹-132 | Ar¹-132 | **1053** | S.O. | S.O. | Ar¹-132 | Ar¹-132 |
| **334** | S.O. | S.O. | S.O. | Ar¹-134 | **1054** | S.O. | S.O. | S.O. | Ar¹-134 |
| **335** | S.O. | S.O. | S.O. | Ar¹-136 | **1055** | S.O. | S.O. | S.O. | Ar¹-136 |
| **336** | S.O. | S.O. | S.O. | Ar¹-137 | **1056** | S.O. | S.O. | S.O. | Ar¹-137 |
| **337** | S.O. | S.O. | S.O. | Ar¹-165 | **1057** | S.O. | S.O. | S.O. | Ar¹-165 |
| **338** | S.O. | S.O. | S.O. | Ar¹-200 | **1058** | S.O. | S.O. | S.O. | Ar¹-200 |
| **339** | S.O. | S.O. | S.O. | Ar¹-201 | **1059** | S.O. | S.O. | S.O. | Ar¹-201 |
| **340** | S.O. | S.O. | Ar¹-134 | Ar¹-134 | **1060** | S.O. | S.O. | Ar¹-134 | Ar¹-134 |
| **341** | S.O. | S.O. | S.O. | Ar¹-136 | **1061** | S.O. | S.O. | S.O. | Ar¹-136 |
| **342** | S.O. | S.O. | S.O. | Ar¹-137 | **1062** | S.O. | S.O. | S.O. | Ar¹-137 |
| **343** | S.O. | S.O. | S.O. | Ar¹-165 | **1063** | S.O. | S.O. | S.O. | Ar¹-165 |
| **344** | S.O. | S.O. | S.O. | Ar¹-200 | **1064** | S.O. | S.O. | S.O. | Ar¹-200 |
| **345** | S.O. | S.O. | S.O. | Ar¹-201 | **1065** | S.O. | S.O. | S.O. | Ar¹-201 |
| **346** | S.O. | S.O. | Ar¹-136 | Ar¹-136 | **1066** | S.O. | S.O. | Ar¹-136 | Ar¹-136 |
| **347** | S.O. | S.O. | S.O. | Ar¹-137 | **1067** | S.O. | S.O. | S.O. | Ar¹-137 |
| **348** | S.O. | S.O. | S.O. | Ar¹-165 | **1068** | S.O. | S.O. | S.O. | Ar¹-165 |
| **349** | S.O. | S.O. | S.O. | Ar¹-200 | **1069** | S.O. | S.O. | S.O. | Ar¹-200 |
| **350** | S.O. | S.O. | S.O. | Ar¹-201 | **1070** | S.O. | S.O. | S.O. | Ar¹-201 |
| **351** | S.O. | S.O. | Ar¹-137 | Ar¹-137 | **1071** | S.O. | S.O. | Ar¹-137 | Ar¹-137 |
| **352** | S.O. | S.O. | S.O. | Ar¹-165 | **1072** | S.O. | S.O. | S.O. | Ar¹-165 |
| **353** | S.O. | S.O. | S.O. | Ar¹-200 | **1073** | S.O. | S.O. | S.O. | Ar¹-200 |
| **354** | S.O. | S.O. | S.O. | Ar¹-201 | **1074** | S.O. | S.O. | S.O. | Ar¹-201 |
| **355** | S.O. | S.O. | Ar¹-165 | Ar¹-165 | **1075** | S.O. | S.O. | Ar¹-165 | Ar¹-165 |
| **356** | S.O. | S.O. | S.O. | Ar¹-200 | **1076** | S.O. | S.O. | S.O. | Ar¹-200 |
| **357** | S.O. | S.O. | S.O. | Ar¹-201 | **1077** | S.O. | S.O. | S.O. | Ar¹-201 |
| **358** | S.O. | S.O. | Ar¹-200 | Ar¹-200 | **1078** | S.O. | S.O. | Ar¹-200 | Ar¹-200 |
| **359** | S.O. | S.O. | S.O. | Ar¹-201 | **1079** | S.O. | S.O. | S.O. | Ar¹-201 |
| **360** | S.O. | S.O. | Ar¹-201 | Ar¹-201 | **1080** | S.O. | S.O. | Ar¹-201 | Ar¹-201 |
| **361** | 1-1-5-0 | k=0 | Ar¹-1 | Ar¹-1 | **1081** | I-1-5-S | k=0 | Ar¹-1 | Ar¹-1 |
| **362** | S.O. | S.O. | S.O. | Ar¹-74 | **1082** | S.O. | S.O. | S.O. | Ar¹-74 |
| **363** | S.O. | S.O. | S.O. | Ar¹-132 | **1083** | S.O. | S.O. | S.O. | Ar¹-132 |
| **364** | S.O. | S.O. | S.O. | Ar¹-134 | **1084** | S.O. | S.O. | S.O. | Ar¹-134 |
| **365** | S.O. | S.O. | S.O. | Ar¹-136 | **1085** | S.O. | S.O. | S.O. | Ar¹-136 |
| **366** | S.O. | S.O. | S.O. | Ar¹-137 | **1086** | S.O. | S.O. | S.O. | Ar¹-137 |
| **367** | S.O. | S.O. | S.O. | Ar¹-165 | **1087** | S.O. | S.O. | S.O. | Ar¹-165 |
| **368** | S.O. | S.O. | S.O. | Ar¹-200 | **1088** | S.O. | S.O. | S.O. | Ar¹-200 |
| **369** | S.O. | S.O. | S.O. | Ar¹-201 | **1089** | S.O. | S.O. | S.O. | Ar¹-201 |
| **370** | S.O. | S.O. | Ar¹-74 | Ar¹-74 | **1090** | S.O. | S.O. | Ar¹-74 | Ar¹-74 |
| **371** | S.O. | S.O. | S.O. | Ar¹-132 | **1091** | S.O. | S.O. | S.O. | Ar¹-132 |
| **372** | S.O. | S.O. | S.O. | Ar¹-134 | **1092** | S.O. | S.O. | S.O. | Ar¹-134 |
| **373** | S.O. | S.O. | S.O. | Ar¹-136 | **1093** | S.O. | S.O. | S.O. | Ar¹-136 |
| **374** | S.O. | S.O. | S.O. | Ar¹-137 | **1094** | S.O. | S.O. | S.O. | Ar¹-137 |
| **375** | S.O. | S.O. | S.O. | Ar¹-165 | **1095** | S.O. | S.O. | S.O. | Ar¹-165 |
| **376** | S.O. | S.O. | S.O. | Ar¹-200 | **1096** | S.O. | S.O. | S.O. | Ar¹-200 |
| **377** | S.O. | S.O. | S.O. | Ar¹-201 | **1097** | S.O. | S.O. | S.O. | Ar¹-201 |
| **378** | S.O. | S.O. | Ar¹-132 | Ar¹-132 | **1098** | S.O. | S.O. | Ar¹-132 | Ar¹-132 |
| **379** | S.O. | S.O. | S.O. | Ar¹-134 | **1099** | S.O. | S.O. | S.O. | Ar¹-134 |
| **380** | S.O. | S.O. | S.O. | Ar¹-136 | **1100** | S.O. | S.O. | S.O. | Ar¹-136 |
| **381** | S.O. | S.O. | S.O. | Ar¹-137 | **1101** | S.O. | S.O. | S.O. | Ar¹-137 |
| **382** | S.O. | S.O. | S.O. | Ar¹-165 | **1102** | S.O. | S.O. | S.O. | Ar¹-165 |
| **383** | S.O. | S.O. | S.O. | Ar¹-200 | **1103** | S.O. | S.O. | S.O. | Ar¹-200 |
| **384** | S.O. | S.O. | S.O. | Ar¹-201 | **1104** | S.O. | S.O. | S.O. | Ar¹-201 |
| **385** | S.O. | S.O. | Ar¹-134 | Ar¹-134 | **1105** | S.O. | S.O. | Ar¹-134 | Ar¹-134 |
| **386** | S.O. | S.O. | S.O. | Ar¹-136 | **1106** | S.O. | S.O. | S.O. | Ar¹-136 |
| **387** | S.O. | S.O. | S.O. | Ar¹-137 | **1107** | S.O. | S.O. | S.O. | Ar¹-137 |
| **388** | S.O. | S.O. | S.O. | Ar¹-165 | **1108** | S.O. | S.O. | S.O. | Ar¹-165 |
| **389** | S.O. | S.O. | S.O. | Ar¹-200 | **1109** | S.O. | S.O. | S.O. | Ar¹-200 |
| **390** | S.O. | S.O. | S.O. | Ar¹-201 | **1110** | S.O. | S.O. | S.O. | Ar¹-201 |
| **391** | S.O. | S.O. | Ar¹-136 | Ar¹-136 | **1111** | S.O. | S.O. | Ar¹-136 | Ar¹-136 |
| **392** | S.O. | S.O. | S.O. | Ar¹-137 | **1112** | S.O. | S.O. | S.O. | Ar¹-137 |
| **393** | S.O. | S.O. | S.O. | Ar¹-165 | **1113** | S.O. | S.O. | S.O. | Ar¹-165 |
| **394** | S.O. | S.O. | S.O. | Ar¹-200 | **1114** | S.O. | S.O. | S.O. | Ar¹-200 |
| **395** | S.O. | S.O. | S.O. | Ar¹-201 | **1115** | S.O. | S.O. | S.O. | Ar¹-201 |
| **396** | S.O. | S.O. | Ar¹-137 | Ar¹-137 | **1116** | S.O. | S.O. | Ar¹-137 | Ar¹-137 |
| **397** | S.O. | S.O. | S.O. | Ar¹-165 | **1117** | S.O. | S.O. | S.O. | Ar¹-165 |
| **398** | S.O. | S.O. | S.O. | Ar¹-200 | **1118** | S.O. | S.O. | S.O. | Ar¹-200 |
| **399** | S.O. | S.O. | S.O. | Ar¹-201 | **1119** | S.O. | S.O. | S.O. | Ar¹-201 |
| **400** | S.O. | S.O. | Ar¹-165 | Ar¹-165 | **1120** | S.O. | S.O. | Ar¹-165 | Ar¹-165 |
| **401** | S.O. | S.O. | S.O. | Ar¹-200 | **1121** | S.O. | S.O. | S.O. | Ar¹-200 |
| **402** | S.O. | S.O. | S.O. | Ar¹-201 | **1122** | S.O. | S.O. | S.O. | Ar¹-201 |
| **403** | S.O. | S.O. | Ar¹-200 | Ar¹-200 | **1123** | S.O. | S.O. | Ar¹-200 | Ar¹-200 |
| **404** | S.O. | S.O. | S.O. | Ar¹-201 | **1124** | S.O. | S.O. | S.O. | Ar¹-201 |
| **405** | S.O. | S.O. | Ar¹-201 | Ar¹-201 | **1125** | S.O. | S.O. | Ar¹-201 | Ar¹-201 |
| **406** | S.O. | Ar^{L}-1 | Ar¹-1 | Ar¹-1 | **1126** | S.O. | Ar^{L}-1 | Ar¹-1 | Ar¹-1 |
| **407** | S.O. | S.O. | S.O. | Ar¹-74 | **1127** | S.O. | S.O. | S.O. | Ar¹-74 |
| **408** | S.O. | S.O. | S.O. | Ar¹-132 | **1128** | S.O. | S.O. | S.O. | Ar¹-132 |
| **409** | S.O. | S.O. | S.O. | Ar¹-134 | **1129** | S.O. | S.O. | S.O. | Ar¹-134 |
| **410** | S.O. | S.O. | S.O. | Ar¹-136 | **1130** | S.O. | S.O. | S.O. | Ar¹-136 |
| **411** | S.O. | S.O. | S.O. | Ar¹-137 | **1131** | S.O. | S.O. | S.O. | Ar¹-137 |
| **412** | S.O. | S.O. | S.O. | Ar¹-165 | **1132** | S.O. | S.O. | S.O. | Ar¹-165 |
| **413** | S.O. | S.O. | S.O. | Ar¹-200 | **1133** | S.O. | S.O. | S.O. | Ar¹-200 |
| **414** | S.O. | S.O. | S.O. | Ar¹-201 | **1134** | S.O. | S.O. | S.O. | Ar¹-201 |
| **415** | S.O. | S.O. | Ar¹-74 | Ar¹-74 | **1135** | S.O. | S.O. | Ar¹-74 | Ar¹-74 |
| **416** | S.O. | S.O. | S.O. | Ar¹-132 | **1136** | S.O. | S.O. | S.O. | Ar¹-132 |
| **417** | S.O. | S.O. | S.O. | Ar¹-134 | **1137** | S.O. | S.O. | S.O. | Ar¹-134 |
| **418** | S.O. | S.O. | S.O. | Ar¹-136 | **1138** | S.O. | S.O. | S.O. | Ar¹-136 |
| **419** | S.O. | S.O. | S.O. | Ar¹-137 | **1139** | S.O. | S.O. | S.O. | Ar¹-137 |
| **420** | S.O. | S.O. | S.O. | Ar¹-165 | **1140** | S.O. | S.O. | S.O. | Ar¹-165 |
| **421** | S.O. | S.O. | S.O. | Ar¹-200 | **1141** | S.O. | S.O. | S.O. | Ar¹-200 |
| **422** | S.O. | S.O. | S.O. | Ar¹-201 | **1142** | S.O. | S.O. | S.O. | Ar¹-201 |
| **423** | S.O. | S.O. | Ar¹-132 | Ar¹-132 | **1143** | S.O. | S.O. | Ar¹-132 | Ar¹-132 |
| **424** | S.O. | S.O. | S.O. | Ar¹-134 | **1144** | S.O. | S.O. | S.O. | Ar¹-134 |
| **425** | S.O. | S.O. | S.O. | Ar¹-136 | **1145** | S.O. | S.O. | S.O. | Ar¹-136 |
| **426** | S.O. | S.O. | S.O. | Ar¹-137 | **1146** | S.O. | S.O. | S.O. | Ar¹-137 |
| **427** | S.O. | S.O. | S.O. | Ar¹-165 | **1147** | S.O. | S.O. | S.O. | Ar¹-165 |
| **428** | S.O. | S.O. | S.O. | Ar¹-200 | **1148** | S.O. | S.O. | S.O. | Ar¹-200 |
| **429** | S.O. | S.O. | S.O. | Ar¹-201 | **1149** | S.O. | S.O. | S.O. | Ar¹-201 |
| **430** | S.O. | S.O. | Ar¹-134 | Ar¹-134 | **1150** | S.O. | S.O. | Ar¹-134 | Ar¹-134 |
| **431** | S.O. | S.O. | S.O. | Ar¹-136 | **1151** | S.O. | S.O. | S.O. | Ar¹-136 |
| **432** | S.O. | S.O. | S.O. | Ar¹-137 | **1152** | S.O. | S.O. | S.O. | Ar¹-137 |
| **433** | S.O. | S.O. | S.O. | Ar¹-165 | **1153** | S.O. | S.O. | S.O. | Ar¹-165 |
| **434** | S.O. | S.O. | S.O. | Ar¹-200 | **1154** | S.O. | S.O. | S.O. | Ar¹-200 |
| **435** | S.O. | S.O. | S.O. | Ar¹-201 | **1155** | S.O. | S.O. | S.O. | Ar¹-201 |
| **436** | S.O. | S.O. | Ar¹-136 | Ar¹-136 | **1156** | S.O. | S.O. | Ar¹-136 | Ar¹-136 |
| **437** | S.O. | S.O. | S.O. | Ar¹-137 | **1157** | S.O. | S.O. | S.O. | Ar¹-137 |
| **438** | S.O. | S.O. | S.O. | Ar¹-165 | **1158** | S.O. | S.O. | S.O. | Ar¹-165 |
| **439** | S.O. | S.O. | S.O. | Ar¹-200 | **1159** | S.O. | S.O. | S.O. | Ar¹-200 |
| **440** | S.O. | S.O. | S.O. | Ar¹-201 | **1160** | S.O. | S.O. | S.O. | Ar¹-201 |
| **441** | S.O. | S.O. | Ar¹-137 | Ar¹-137 | **1161** | S.O. | S.O. | Ar¹-137 | Ar¹-137 |
| **442** | S.O. | S.O. | S.O. | Ar¹-165 | **1162** | S.O. | S.O. | S.O. | Ar¹-165 |
| **443** | S.O. | S.O. | S.O. | Ar¹-200 | **1163** | S.O. | S.O. | S.O. | Ar¹-200 |
| **444** | S.O. | S.O. | S.O. | Ar¹-201 | **1164** | S.O. | S.O. | S.O. | Ar¹-201 |
| **445** | S.O. | S.O. | Ar¹-165 | Ar¹-165 | **1165** | S.O. | S.O. | Ar¹-165 | Ar¹-165 |
| **446** | S.O. | S.O. | S.O. | Ar¹-200 | **1166** | S.O. | S.O. | S.O. | Ar¹-200 |
| **447** | S.O. | S.O. | S.O. | Ar¹-201 | **1167** | S.O. | S.O. | S.O. | Ar¹-201 |
| **448** | S.O. | S.O. | Ar¹-200 | Ar¹-200 | **1168** | S.O. | S.O. | Ar¹-200 | Ar¹-200 |
| **449** | S.O. | S.O. | S.O. | Ar¹-201 | **1169** | S.O. | S.O. | S.O. | Ar¹-201 |
| **450** | S.O. | S.O. | Ar¹-201 | Ar¹-201 | **1170** | S.O. | S.O. | Ar¹-201 | Ar¹-201 |
| **451** | S.O. | Ar^{L}-2 | Ar¹-1 | Ar¹-1 | **1171** | S.O. | Ar^{L}-2 | Ar¹-1 | Ar¹-1 |
| **452** | S.O. | S.O. | S.O. | Ar¹-74 | **1172** | S.O. | S.O. | S.O. | Ar¹-74 |
| **453** | S.O. | S.O. | S.O. | Ar¹-132 | **1173** | S.O. | S.O. | S.O. | Ar¹-132 |
| **454** | S.O. | S.O. | S.O. | Ar¹-134 | **1174** | S.O. | S.O. | S.O. | Ar¹-134 |
| **455** | S.O. | S.O. | S.O. | Ar¹-136 | **1175** | S.O. | S.O. | S.O. | Ar¹-136 |
| **456** | S.O. | S.O. | S.O. | Ar¹-137 | **1176** | S.O. | S.O. | S.O. | Ar¹-137 |
| **457** | S.O. | S.O. | S.O. | Ar¹-165 | **1177** | S.O. | S.O. | S.O. | Ar¹-165 |
| **458** | S.O. | S.O. | S.O. | Ar¹-200 | **1178** | S.O. | S.O. | S.O. | Ar¹-200 |
| **459** | S.O. | S.O. | S.O. | Ar¹-201 | **1179** | S.O. | S.O. | S.O. | Ar¹-201 |
| **460** | S.O. | S.O. | Ar¹-74 | Ar¹-74 | **1180** | S.O. | S.O. | Ar¹-74 | Ar¹-74 |
| **461** | S.O. | S.O. | S.O. | Ar¹-132 | **1181** | S.O. | S.O. | S.O. | Ar¹-132 |
| **462** | S.O. | S.O. | S.O. | Ar¹-134 | **1182** | S.O. | S.O. | S.O. | Ar¹-134 |
| **463** | S.O. | S.O. | S.O. | Ar¹-136 | **1183** | S.O. | S.O. | S.O. | Ar¹-136 |
| **464** | S.O. | S.O. | S.O. | Ar¹-137 | **1184** | S.O. | S.O. | S.O. | Ar¹-137 |
| **465** | S.O. | S.O. | S.O. | Ar¹-165 | **1185** | S.O. | S.O. | S.O. | Ar¹-165 |
| **466** | S.O. | S.O. | S.O. | Ar¹-200 | **1186** | S.O. | S.O. | S.O. | Ar¹-200 |
| **467** | S.O. | S.O. | S.O. | Ar¹-201 | **1187** | S.O. | S.O. | S.O. | Ar¹-201 |
| **468** | S.O. | S.O. | Ar¹-132 | Ar¹-132 | **1188** | S.O. | S.O. | Ar¹-132 | Ar¹-132 |
| **469** | S.O. | S.O. | S.O. | Ar¹-134 | **1189** | S.O. | S.O. | S.O. | Ar¹-134 |
| **470** | S.O. | S.O. | S.O. | Ar¹-136 | **1190** | S.O. | S.O. | S.O. | Ar¹-136 |
| **471** | S.O. | S.O. | S.O. | Ar¹-137 | **1191** | S.O. | S.O. | S.O. | Ar¹-137 |
| **472** | S.O. | S.O. | S.O. | Ar¹-165 | **1192** | S.O. | S.O. | S.O. | Ar¹-165 |
| **473** | S.O. | S.O. | S.O. | Ar¹-200 | **1193** | S.O. | S.O. | S.O. | Ar¹-200 |
| **474** | S.O. | S.O. | S.O. | Ar¹-201 | **1194** | S.O. | S.O. | S.O. | Ar¹-201 |
| **475** | S.O. | S.O. | Ar¹-134 | Ar¹-134 | **1195** | S.O. | S.O. | Ar¹-134 | Ar¹-134 |
| **476** | S.O. | S.O. | S.O. | Ar¹-136 | **1196** | S.O. | S.O. | S.O. | Ar¹-136 |
| **477** | S.O. | S.O. | S.O. | Ar¹-137 | **1197** | S.O. | S.O. | S.O. | Ar¹-137 |
| **478** | S.O. | S.O. | S.O. | Ar¹-165 | **1198** | S.O. | S.O. | S.O. | Ar¹-165 |
| **479** | S.O. | S.O. | S.O. | Ar¹-200 | **1199** | S.O. | S.O. | S.O. | Ar¹-200 |
| **480** | S.O. | S.O. | S.O. | Ar¹-201 | **1200** | S.O. | S.O. | S.O. | Ar¹-201 |
| **481** | S.O. | S.O. | Ar¹-136 | Ar¹-136 | **1201** | S.O. | S.O. | Ar¹-136 | Ar¹-136 |
| **482** | S.O. | S.O. | S.O. | Ar¹-137 | **1202** | S.O. | S.O. | S.O. | Ar¹-137 |
| **483** | S.O. | S.O. | S.O. | Ar¹-165 | **1203** | S.O. | S.O. | S.O. | Ar¹-165 |
| **484** | S.O. | S.O. | S.O. | Ar¹-200 | **1204** | S.O. | S.O. | S.O. | Ar¹-200 |
| **485** | S.O. | S.O. | S.O. | Ar¹-201 | **1205** | S.O. | S.O. | S.O. | Ar¹-201 |
| **486** | S.O. | S.O. | Ar¹-137 | Ar¹-137 | **1206** | S.O. | S.O. | Ar¹-137 | Ar¹-137 |
| **487** | S.O. | S.O. | S.O. | Ar¹-165 | **1207** | S.O. | S.O. | S.O. | Ar¹-165 |
| **488** | S.O. | S.O. | S.O. | Ar¹-200 | **1208** | S.O. | S.O. | S.O. | Ar¹-200 |
| **489** | S.O. | S.O. | S.O. | Ar¹-201 | **1209** | S.O. | S.O. | S.O. | Ar¹-201 |
| **490** | S.O. | S.O. | Ar¹-165 | Ar¹-165 | **1210** | S.O. | S.O. | Ar¹-165 | Ar¹-165 |
| **491** | S.O. | S.O. | S.O. | Ar¹-200 | **1211** | S.O. | S.O. | S.O. | Ar¹-200 |
| **492** | S.O. | S.O. | S.O. | Ar¹-201 | **1212** | S.O. | S.O. | S.O. | Ar¹-201 |
| **493** | S.O. | S.O. | Ar¹-200 | Ar¹-200 | **1213** | S.O. | S.O. | Ar¹-200 | Ar¹-200 |
| **494** | S.O. | S.O. | S.O. | Ar¹-201 | **1214** | S.O. | S.O. | S.O. | Ar¹-201 |
| **495** | S.O. | S.O. | Ar¹-201 | Ar¹-201 | **1215** | S.O. | S.O. | Ar¹-201 | Ar¹-201 |
| **496** | S.O. | Ar^{L}-3 | Ar¹-1 | Ar¹-1 | **1216** | S.O. | Ar^{L}-3 | Ar¹-1 | Ar¹-1 |
| **497** | S.O. | S.O. | S.O. | Ar¹-74 | **1217** | S.O. | S.O. | S.O. | Ar¹-74 |
| **498** | S.O. | S.O. | S.O. | Ar¹-132 | **1218** | S.O. | S.O. | S.O. | Ar¹-132 |
| **499** | S.O. | S.O. | S.O. | Ar¹-134 | **1219** | S.O. | S.O. | S.O. | Ar¹-134 |
| **500** | S.O. | S.O. | S.O. | Ar¹-136 | **1220** | S.O. | S.O. | S.O. | Ar¹-136 |
| **501** | S.O. | S.O. | S.O. | Ar¹-137 | **1221** | S.O. | S.O. | S.O. | Ar¹-137 |
| **502** | S.O. | S.O. | S.O. | Ar¹-165 | **1222** | S.O. | S.O. | S.O. | Ar¹-165 |
| **503** | S.O. | S.O. | S.O. | Ar¹-200 | **1223** | S.O. | S.O. | S.O. | Ar¹-200 |
| **504** | S.O. | S.O. | S.O. | Ar¹-201 | **1224** | S.O. | S.O. | S.O. | Ar¹-201 |
| **505** | S.O. | S.O. | Ar¹-74 | Ar¹-74 | **1225** | S.O. | S.O. | Ar¹-74 | Ar¹-74 |
| **506** | S.O. | S.O. | S.O. | Ar¹-132 | **1226** | S.O. | S.O. | S.O. | Ar¹-132 |
| **507** | S.O. | S.O. | S.O. | Ar¹-134 | **1227** | S.O. | S.O. | S.O. | Ar¹-134 |
| **508** | S.O. | S.O. | S.O. | Ar¹-136 | **1228** | S.O. | S.O. | S.O. | Ar¹-136 |
| **509** | S.O. | S.O. | S.O. | Ar¹-137 | **1229** | S.O. | S.O. | S.O. | Ar¹-137 |
| **510** | S.O. | S.O. | S.O. | Ar¹-165 | **1230** | S.O. | S.O. | S.O. | Ar¹-165 |
| **511** | S.O. | S.O. | S.O. | Ar¹-200 | **1231** | S.O. | S.O. | S.O. | Ar¹-200 |
| **512** | S.O. | S.O. | S.O. | Ar¹-201 | **1232** | S.O. | S.O. | S.O. | Ar¹-201 |
| **513** | S.O. | S.O. | Ar¹-132 | Ar¹-132 | **1233** | S.O. | S.O. | Ar¹-132 | Ar¹-132 |
| **514** | S.O. | S.O. | S.O. | Ar¹-134 | **1234** | S.O. | S.O. | S.O. | Ar¹-134 |
| **515** | S.O. | S.O. | S.O. | Ar¹-136 | **1235** | S.O. | S.O. | S.O. | Ar¹-136 |
| **516** | S.O. | S.O. | S.O. | Ar¹-137 | **1236** | S.O. | S.O. | S.O. | Ar¹-137 |
| **517** | S.O. | S.O. | S.O. | Ar¹-165 | **1237** | S.O. | S.O. | S.O. | Ar¹-165 |
| **518** | S.O. | S.O. | S.O. | Ar¹-200 | **1238** | S.O. | S.O. | S.O. | Ar¹-200 |
| **519** | S.O. | S.O. | S.O. | Ar¹-201 | **1239** | S.O. | S.O. | S.O. | Ar¹-201 |
| **520** | S.O. | S.O. | Ar¹-134 | Ar¹-134 | **1240** | S.O. | S.O. | Ar¹-134 | Ar¹-134 |
| **521** | S.O. | S.O. | S.O. | Ar¹-136 | **1241** | S.O. | S.O. | S.O. | Ar¹-136 |
| **522** | S.O. | S.O. | S.O. | Ar¹-137 | **1242** | S.O. | S.O. | S.O. | Ar¹-137 |
| **523** | S.O. | S.O. | S.O. | Ar¹-165 | **1243** | S.O. | S.O. | S.O. | Ar¹-165 |
| **524** | S.O. | S.O. | S.O. | Ar¹-200 | **1244** | S.O. | S.O. | S.O. | Ar¹-200 |
| **525** | S.O. | S.O. | S.O. | Ar¹-201 | **1245** | S.O. | S.O. | S.O. | Ar¹-201 |
| **526** | S.O. | S.O. | Ar¹-136 | Ar¹-136 | **1246** | S.O. | S.O. | Ar¹-136 | Ar¹-136 |
| **527** | S.O. | S.O. | S.O. | Ar¹-137 | **1247** | S.O. | S.O. | S.O. | Ar¹-137 |
| **528** | S.O. | S.O. | S.O. | Ar¹-165 | **1248** | S.O. | S.O. | S.O. | Ar¹-165 |
| **529** | S.O. | S.O. | S.O. | Ar¹-200 | **1249** | S.O. | S.O. | S.O. | Ar¹-200 |
| **530** | S.O. | S.O. | S.O. | Ar¹-201 | **1250** | S.O. | S.O. | S.O. | Ar¹-201 |
| **531** | S.O. | S.O. | Ar¹-137 | Ar¹-137 | **1251** | S.O. | S.O. | Ar¹-137 | Ar¹-137 |
| **532** | S.O. | S.O. | S.O. | Ar¹-165 | **1252** | S.O. | S.O. | S.O. | Ar¹-165 |
| **533** | S.O. | S.O. | S.O. | Ar¹-200 | **1253** | S.O. | S.O. | S.O. | Ar¹-200 |
| **534** | S.O. | S.O. | S.O. | Ar¹-201 | **1254** | S.O. | S.O. | S.O. | Ar¹-201 |
| **535** | S.O. | S.O. | Ar¹-165 | Ar¹-165 | **1255** | S.O. | S.O. | Ar¹-165 | Ar¹-165 |
| **536** | S.O. | S.O. | S.O. | Ar¹-200 | **1256** | S.O. | S.O. | S.O. | Ar¹-200 |
| **537** | S.O. | S.O. | S.O. | Ar¹-201 | **1257** | S.O. | S.O. | S.O. | Ar¹-201 |
| **538** | S.O. | S.O. | Ar¹-200 | Ar¹-200 | **1258** | S.O. | S.O. | Ar¹-200 | Ar¹-200 |
| **539** | S.O. | S.O. | S.O. | Ar¹-201 | **1259** | S.O. | S.O. | S.O. | Ar¹-201 |
| **540** | S.O. | S.O. | Ar¹-201 | Ar¹-201 | **1260** | S.O. | S.O. | Ar¹-201 | Ar¹-201 |
| **541** | I-1-7-O | k=0 | Ar¹-1 | Ar¹-1 | **1261** | I-1-7-O | k=0 | Ar¹-1 | Ar¹-1 |
| **542** | S.O. | S.O. | S.O. | Ar¹-74 | **1262** | S.O. | S.O. | S.O. | Ar¹-74 |
| **543** | S.O. | S.O. | S.O. | Ar¹-132 | **1263** | S.O. | S.O. | S.O. | Ar¹-132 |
| **544** | S.O. | S.O. | S.O. | Ar¹-134 | **1264** | S.O. | S.O. | S.O. | Ar¹-134 |
| **545** | S.O. | S.O. | S.O. | Ar¹-136 | **1265** | S.O. | S.O. | S.O. | Ar¹-136 |
| **546** | S.O. | S.O. | S.O. | Ar¹-137 | **1266** | S.O. | S.O. | S.O. | Ar¹-137 |
| **547** | S.O. | S.O. | S.O. | Ar¹-165 | **1267** | S.O. | S.O. | S.O. | Ar¹-165 |
| **548** | S.O. | S.O. | S.O. | Ar¹-200 | **1268** | S.O. | S.O. | S.O. | Ar¹-200 |
| **549** | S.O. | S.O. | S.O. | Ar¹-201 | **1269** | S.O. | S.O. | S.O. | Ar¹-201 |
| **550** | S.O. | S.O. | Ar¹-74 | Ar¹-74 | **1270** | S.O. | S.O. | Ar¹-74 | Ar¹-74 |
| **551** | S.O. | S.O. | S.O. | Ar¹-132 | **1271** | S.O. | S.O. | S.O. | Ar¹-132 |
| **552** | S.O. | S.O. | S.O. | Ar¹-134 | **1272** | S.O. | S.O. | S.O. | Ar¹-134 |
| **553** | S.O. | S.O. | S.O. | Ar¹-136 | **1273** | S.O. | S.O. | S.O. | Ar¹-136 |
| **554** | S.O. | S.O. | S.O. | Ar¹-137 | **1274** | S.O. | S.O. | S.O. | Ar¹-137 |
| **555** | S.O. | S.O. | S.O. | Ar¹-165 | **1275** | S.O. | S.O. | S.O. | Ar¹-165 |
| **556** | S.O. | S.O. | S.O. | Ar¹-200 | **1276** | S.O. | S.O. | S.O. | Ar¹-200 |
| **557** | S.O. | S.O. | S.O. | Ar¹-201 | **1277** | S.O. | S.O. | S.O. | Ar¹-201 |
| **558** | S.O. | S.O. | Ar¹-132 | Ar¹-132 | **1278** | S.O. | S.O. | Ar¹-132 | Ar¹-132 |
| **559** | S.O. | S.O. | S.O. | Ar¹-134 | **1279** | S.O. | S.O. | S.O. | Ar¹-134 |
| **560** | S.O. | S.O. | S.O. | Ar¹-136 | **1280** | S.O. | S.O. | S.O. | Ar¹-136 |
| **561** | S.O. | S.O. | S.O. | Ar¹-137 | **1281** | S.O. | S.O. | S.O. | Ar¹-137 |
| **562** | S.O. | S.O. | S.O. | Ar¹-165 | **1282** | S.O. | S.O. | S.O. | Ar¹-165 |
| **563** | S.O. | S.O. | S.O. | Ar¹-200 | **1283** | S.O. | S.O. | S.O. | Ar¹-200 |
| **564** | S.O. | S.O. | S.O. | Ar¹-201 | **1284** | S.O. | S.O. | S.O. | Ar¹-201 |
| **565** | S.O. | S.O. | Ar¹-134 | Ar¹-134 | **1285** | S.O. | S.O. | Ar¹-134 | Ar¹-134 |
| **566** | S.O. | S.O. | S.O. | Ar¹-136 | **1286** | S.O. | S.O. | S.O. | Ar¹-136 |
| **567** | S.O. | S.O. | S.O. | Ar¹-137 | **1287** | S.O. | S.O. | S.O. | Ar¹-137 |
| **568** | S.O. | S.O. | S.O. | Ar¹-165 | **1288** | S.O. | S.O. | S.O. | Ar¹-165 |
| **569** | S.O. | S.O. | S.O. | Ar¹-200 | **1289** | S.O. | S.O. | S.O. | Ar¹-200 |
| **570** | S.O. | S.O. | S.O. | Ar¹-201 | **1290** | S.O. | S.O. | S.O. | Ar¹-201 |
| **571** | S.O. | S.O. | Ar¹-136 | Ar¹-136 | **1291** | S.O. | S.O. | Ar¹-136 | Ar¹-136 |
| **572** | S.O. | S.O. | S.O. | Ar¹-137 | **1292** | S.O. | S.O. | S.O. | Ar¹-137 |
| **573** | S.O. | S.O. | S.O. | Ar¹-165 | **1293** | S.O. | S.O. | S.O. | Ar¹-165 |
| **574** | S.O. | S.O. | S.O. | Ar¹-200 | **1294** | S.O. | S.O. | S.O. | Ar¹-200 |
| **575** | S.O. | S.O. | S.O. | Ar¹-201 | **1295** | S.O. | S.O. | S.O. | Ar¹-201 |
| **576** | S.O. | S.O. | Ar¹-137 | Ar¹-137 | **1296** | S.O. | S.O. | Ar¹-137 | Ar¹-137 |
| **577** | S.O. | S.O. | S.O. | Ar¹-165 | **1297** | S.O. | S.O. | S.O. | Ar¹-165 |
| **578** | S.O. | S.O. | S.O. | Ar¹-200 | **1298** | S.O. | S.O. | S.O. | Ar¹-200 |
| **579** | S.O. | S.O. | S.O. | Ar¹-201 | **1299** | S.O. | S.O. | S.O. | Ar¹-201 |
| **580** | S.O. | S.O. | Ar¹-165 | Ar¹-165 | **1300** | S.O. | S.O. | Ar¹-165 | Ar¹-165 |
| **581** | S.O. | S.O. | S.O. | Ar¹-200 | **1301** | S.O. | S.O. | S.O. | Ar¹-200 |
| **582** | S.O. | S.O. | S.O. | Ar¹-201 | **1302** | S.O. | S.O. | S.O. | Ar¹-201 |
| **583** | S.O. | S.O. | Ar¹-200 | Ar¹-200 | **1303** | S.O. | S.O. | Ar¹-200 | Ar¹-200 |
| **584** | S.O. | S.O. | S.O. | Ar¹-201 | **1304** | S.O. | S.O. | S.O. | Ar¹-201 |
| **585** | S.O. | S.O. | Ar¹-201 | Ar¹-201 | **1305** | S.O. | S.O. | Ar¹-201 | Ar¹-201 |
| **586** | S.O. | Ar^{L}-1 | Ar¹-1 | Ar¹-1 | **1306** | S.O. | Ar^{L}-1 | Ar¹-1 | Ar¹-1 |
| **587** | S.O. | S.O. | S.O. | Ar¹-74 | **1307** | S.O. | S.O. | S.O. | Ar¹-74 |
| **588** | S.O. | S.O. | S.O. | Ar¹-132 | **1308** | S.O. | S.O. | S.O. | Ar¹-132 |
| **589** | S.O. | S.O. | S.O. | Ar¹-134 | **1309** | S.O. | S.O. | S.O. | Ar¹-134 |
| **590** | S.O. | S.O. | S.O. | Ar¹-136 | **1310** | S.O. | S.O. | S.O. | Ar¹-136 |
| **591** | S.O. | S.O. | S.O. | Ar¹-137 | **1311** | S.O. | S.O. | S.O. | Ar¹-137 |
| **592** | S.O. | S.O. | S.O. | Ar¹-165 | **1312** | S.O. | S.O. | S.O. | Ar¹-165 |
| **593** | S.O. | S.O. | S.O. | Ar¹-200 | **1313** | S.O. | S.O. | S.O. | Ar¹-200 |
| **594** | S.O. | S.O. | S.O. | Ar¹-201 | **1314** | S.O. | S.O. | S.O. | Ar¹-201 |
| **595** | S.O. | S.O. | Ar¹-74 | Ar¹-74 | **1315** | S.O. | S.O. | Ar¹-74 | Ar¹-74 |
| **596** | S.O. | S.O. | S.O. | Ar¹-132 | **1316** | S.O. | S.O. | S.O. | Ar¹-132 |
| **597** | S.O. | S.O. | S.O. | Ar¹-134 | **1317** | S.O. | S.O. | S.O. | Ar¹-134 |
| **598** | S.O. | S.O. | S.O. | Ar¹-136 | **1318** | S.O. | S.O. | S.O. | Ar¹-136 |
| **599** | S.O. | S.O. | S.O. | Ar¹-137 | **1319** | S.O. | S.O. | S.O. | Ar¹-137 |
| **600** | S.O. | S.O. | S.O. | Ar¹-165 | **1320** | S.O. | S.O. | S.O. | Ar¹-165 |
| **601** | S.O. | S.O. | S.O. | Ar¹-200 | **1321** | S.O. | S.O. | S.O. | Ar¹-200 |
| **602** | S.O. | S.O. | S.O. | Ar¹-201 | **1322** | S.O. | S.O. | S.O. | Ar¹-201 |
| **603** | S.O. | S.O. | Ar¹-132 | Ar¹-132 | **1323** | S.O. | S.O. | Ar¹-132 | Ar¹-132 |
| **604** | S.O. | S.O. | S.O. | Ar¹-134 | **1324** | S.O. | S.O. | S.O. | Ar¹-134 |
| **605** | S.O. | S.O. | S.O. | Ar¹-136 | **1325** | S.O. | S.O. | S.O. | Ar¹-136 |
| **606** | S.O. | S.O. | S.O. | Ar¹-137 | **1326** | S.O. | S.O. | S.O. | Ar¹-137 |
| **607** | S.O. | S.O. | S.O. | Ar¹-165 | **1327** | S.O. | S.O. | S.O. | Ar¹-165 |
| **608** | S.O. | S.O. | S.O. | Ar¹-200 | **1328** | S.O. | S.O. | S.O. | Ar¹-200 |
| **609** | S.O. | S.O. | S.O. | Ar¹-201 | **1329** | S.O. | S.O. | S.O. | Ar¹-201 |
| **610** | S.O. | S.O. | Ar¹-134 | Ar¹-134 | **1330** | S.O. | S.O. | Ar¹-134 | Ar¹-134 |
| **611** | S.O. | S.O. | S.O. | Ar¹-136 | **1331** | S.O. | S.O. | S.O. | Ar¹-136 |
| **612** | S.O. | S.O. | S.O. | Ar¹-137 | **1332** | S.O. | S.O. | S.O. | Ar¹-137 |
| **613** | S.O. | S.O. | S.O. | Ar¹-165 | **1333** | S.O. | S.O. | S.O. | Ar¹-165 |
| **614** | S.O. | S.O. | S.O. | Ar¹-200 | **1334** | S.O. | S.O. | S.O. | Ar¹-200 |
| **615** | S.O. | S.O. | S.O. | Ar¹-201 | **1335** | S.O. | S.O. | S.O. | Ar¹-201 |
| **616** | S.O. | S.O. | Ar¹-136 | Ar¹-136 | **1336** | S.O. | S.O. | Ar¹-136 | Ar¹-136 |
| **617** | S.O. | S.O. | S.O. | Ar¹-137 | **1337** | S.O. | S.O. | S.O. | Ar¹-137 |
| **618** | S.O. | S.O. | S.O. | Ar¹-165 | **1338** | S.O. | S.O. | S.O. | Ar¹-165 |
| **619** | S.O. | S.O. | S.O. | Ar¹-200 | **1339** | S.O. | S.O. | S.O. | Ar¹-200 |
| **620** | S.O. | S.O. | S.O. | Ar¹-201 | **1340** | S.O. | S.O. | S.O. | Ar¹-201 |
| **621** | S.O. | S.O. | Ar¹-137 | Ar¹-137 | **1341** | S.O. | S.O. | Ar¹-137 | Ar¹-137 |
| **622** | S.O. | S.O. | S.O. | Ar¹-165 | **1342** | S.O. | S.O. | S.O. | Ar¹-165 |
| **623** | S.O. | S.O. | S.O. | Ar¹-200 | **1343** | S.O. | S.O. | S.O. | Ar¹-200 |
| **624** | S.O. | S.O. | S.O. | Ar¹-201 | **1344** | S.O. | S.O. | S.O. | Ar¹-201 |
| **625** | S.O. | S.O. | Ar¹-165 | Ar¹-165 | **1345** | S.O. | S.O. | Ar¹-165 | Ar¹-165 |
| **626** | S.O. | S.O. | S.O. | Ar¹-200 | **1346** | S.O. | S.O. | S.O. | Ar¹-200 |
| **627** | S.O. | S.O. | S.O. | Ar¹-201 | **1347** | S.O. | S.O. | S.O. | Ar¹-201 |
| **628** | S.O. | S.O. | Ar¹-200 | Ar¹-200 | **1348** | S.O. | S.O. | Ar¹-200 | Ar¹-200 |
| **629** | S.O. | S.O. | S.O. | Ar¹-201 | **1349** | S.O. | S.O. | S.O. | Ar¹-201 |
| **630** | S.O. | S.O. | Ar¹-201 | Ar¹-201 | **1350** | S.O. | S.O. | Ar¹-201 | Ar¹-201 |
| **631** | S.O. | Ar^{L}-2 | Ar¹-1 | Ar¹-1 | **1351** | S.O. | Ar^{L}-2 | Ar¹-1 | Ar¹-1 |
| **632** | S.O. | S.O. | S.O. | Ar¹-74 | **1352** | S.O. | S.O. | S.O. | Ar¹-74 |
| **633** | S.O. | S.O. | S.O. | Ar¹-132 | **1353** | S.O. | S.O. | S.O. | Ar¹-132 |
| **634** | S.O. | S.O. | S.O. | Ar¹-134 | **1354** | S.O. | S.O. | S.O. | Ar¹-134 |
| **635** | S.O. | S.O. | S.O. | Ar¹-136 | **1355** | S.O. | S.O. | S.O. | Ar¹-136 |
| **636** | S.O. | S.O. | S.O. | Ar¹-137 | **1356** | S.O. | S.O. | S.O. | Ar¹-137 |
| **637** | S.O. | S.O. | S.O. | Ar¹-165 | **1357** | S.O. | S.O. | S.O. | Ar¹-165 |
| **638** | S.O. | S.O. | S.O. | Ar¹-200 | **1358** | S.O. | S.O. | S.O. | Ar¹-200 |
| **639** | S.O. | S.O. | S.O. | Ar¹-201 | **1359** | S.O. | S.O. | S.O. | Ar¹-201 |
| **640** | S.O. | S.O. | Ar¹-74 | Ar¹-74 | **1360** | S.O. | S.O. | Ar¹-74 | Ar¹-74 |
| **641** | S.O. | S.O. | S.O. | Ar¹-132 | **1361** | S.O. | S.O. | S.O. | Ar¹-132 |
| **642** | S.O. | S.O. | S.O. | Ar¹-134 | **1362** | S.O. | S.O. | S.O. | Ar¹-134 |
| **643** | S.O. | S.O. | S.O. | Ar¹-136 | **1363** | S.O. | S.O. | S.O. | Ar¹-136 |
| **644** | S.O. | S.O. | S.O. | Ar¹-137 | **1364** | S.O. | S.O. | S.O. | Ar¹-137 |
| **645** | S.O. | S.O. | S.O. | Ar¹-165 | **1365** | S.O. | S.O. | S.O. | Ar¹-165 |
| **646** | S.O. | S.O. | S.O. | Ar¹-200 | **1366** | S.O. | S.O. | S.O. | Ar¹-200 |
| **647** | S.O. | S.O. | S.O. | Ar¹-201 | **1367** | S.O. | S.O. | S.O. | Ar¹-201 |
| **648** | S.O. | S.O. | Ar¹-132 | Ar¹-132 | **1368** | S.O. | S.O. | Ar¹-132 | Ar¹-132 |
| **649** | S.O. | S.O. | S.O. | Ar¹-134 | **1369** | S.O. | S.O. | S.O. | Ar¹-134 |
| **650** | S.O. | S.O. | S.O. | Ar¹-136 | **1370** | S.O. | S.O. | S.O. | Ar¹-136 |
| **651** | S.O. | S.O. | S.O. | Ar¹-137 | **1371** | S.O. | S.O. | S.O. | Ar¹-137 |
| **652** | S.O. | S.O. | S.O. | Ar¹-165 | **1372** | S.O. | S.O. | S.O. | Ar¹-165 |
| **653** | S.O. | S.O. | S.O. | Ar¹-200 | **1373** | S.O. | S.O. | S.O. | Ar¹-200 |
| **654** | S.O. | S.O. | S.O. | Ar¹-201 | **1374** | S.O. | S.O. | S.O. | Ar¹-201 |
| **655** | S.O. | S.O. | Ar¹-134 | Ar¹-134 | **1375** | S.O. | S.O. | Ar¹-134 | Ar¹-134 |
| **656** | S.O. | S.O. | S.O. | Ar¹-136 | **1376** | S.O. | S.O. | S.O. | Ar¹-136 |
| **657** | S.O. | S.O. | S.O. | Ar¹-137 | **1377** | S.O. | S.O. | S.O. | Ar¹-137 |
| **658** | S.O. | S.O. | S.O. | Ar¹-165 | **1378** | S.O. | S.O. | S.O. | Ar¹-165 |
| **659** | S.O. | S.O. | S.O. | Ar¹-200 | **1379** | S.O. | S.O. | S.O. | Ar¹-200 |
| **660** | S.O. | S.O. | S.O. | Ar¹-201 | **1380** | S.O. | S.O. | S.O. | Ar¹-201 |
| **661** | S.O. | S.O. | Ar¹-136 | Ar¹-136 | **1381** | S.O. | S.O. | Ar¹-136 | Ar¹-136 |
| **662** | S.O. | S.O. | S.O. | Ar¹-137 | **1382** | S.O. | S.O. | S.O. | Ar¹-137 |
| **663** | S.O. | S.O. | S.O. | Ar¹-165 | **1383** | S.O. | S.O. | S.O. | Ar¹-165 |
| **664** | S.O. | S.O. | S.O. | Ar¹-200 | **1384** | S.O. | S.O. | S.O. | Ar¹-200 |
| **665** | S.O. | S.O. | S.O. | Ar¹-201 | **1385** | S.O. | S.O. | S.O. | Ar¹-201 |
| **666** | S.O. | S.O. | Ar¹-137 | Ar¹-137 | **1386** | S.O. | S.O. | Ar¹-137 | Ar¹-137 |
| **667** | S.O. | S.O. | S.O. | Ar¹-165 | **1387** | S.O. | S.O. | S.O. | Ar¹-165 |
| **668** | S.O. | S.O. | S.O. | Ar¹-200 | **1388** | S.O. | S.O. | S.O. | Ar¹-200 |
| **669** | S.O. | S.O. | S.O. | Ar¹-201 | **1389** | S.O. | S.O. | S.O. | Ar¹-201 |
| **670** | S.O. | S.O. | Ar¹-165 | Ar¹-165 | **1390** | S.O. | S.O. | Ar¹-165 | Ar¹-165 |
| **671** | S.O. | S.O. | S.O. | Ar¹-200 | **1391** | S.O. | S.O. | S.O. | Ar¹-200 |
| **672** | S.O. | S.O. | S.O. | Ar¹-201 | **1392** | S.O. | S.O. | S.O. | Ar¹-201 |
| **673** | S.O. | S.O. | Ar¹-200 | Ar¹-200 | **1393** | S.O. | S.O. | Ar¹-200 | Ar¹-200 |
| **674** | S.O. | S.O. | S.O. | Ar¹-201 | **1394** | S.O. | S.O. | S.O. | Ar¹-201 |
| **675** | S.O. | S.O. | Ar¹-201 | Ar¹-201 | **1395** | S.O. | S.O. | Ar¹-201 | Ar¹-201 |
| **676** | S.O. | Ar^{L}-3 | Ar¹-1 | Ar¹-1 | **1396** | S.O. | Ar^{L}-3 | Ar¹-1 | Ar¹-1 |
| **677** | S.O. | S.O. | S.O. | Ar¹-74 | **1397** | S.O. | S.O. | S.O. | Ar¹-74 |
| **678** | S.O. | S.O. | S.O. | Ar¹-132 | **1398** | S.O. | S.O. | S.O. | Ar¹-132 |
| **679** | S.O. | S.O. | S.O. | Ar¹-134 | **1399** | S.O. | S.O. | S.O. | Ar¹-134 |
| **680** | S.O. | S.O. | S.O. | Ar¹-136 | **1400** | S.O. | S.O. | S.O. | Ar¹-136 |
| **681** | S.O. | S.O. | S.O. | Ar¹-137 | **1401** | S.O. | S.O. | S.O. | Ar¹-137 |
| **682** | S.O. | S.O. | S.O. | Ar¹-165 | **1402** | S.O. | S.O. | S.O. | Ar¹-165 |
| **683** | S.O. | S.O. | S.O. | Ar¹-200 | **1403** | S.O. | S.O. | S.O. | Ar¹-200 |
| **684** | S.O. | S.O. | S.O. | Ar¹-201 | **1404** | S.O. | S.O. | S.O. | Ar¹-201 |
| **685** | S.O. | S.O. | Ar¹-74 | Ar¹-74 | **1405** | S.O. | S.O. | Ar¹-74 | Ar¹-74 |
| **686** | S.O. | S.O. | S.O. | Ar¹-132 | **1406** | S.O. | S.O. | S.O. | Ar¹-132 |
| **687** | S.O. | S.O. | S.O. | Ar¹-134 | **1407** | S.O. | S.O. | S.O. | Ar¹-134 |
| **688** | S.O. | S.O. | S.O. | Ar¹-136 | **1408** | S.O. | S.O. | S.O. | Ar¹-136 |
| **689** | S.O. | S.O. | S.O. | Ar¹-137 | **1409** | S.O. | S.O. | S.O. | Ar¹-137 |
| **690** | S.O. | S.O. | S.O. | Ar¹-165 | **1410** | S.O. | S.O. | S.O. | Ar¹-165 |
| **691** | S.O. | S.O. | S.O. | Ar¹-200 | **1411** | S.O. | S.O. | S.O. | Ar¹-200 |
| **692** | S.O. | S.O. | S.O. | Ar¹-201 | **1412** | S.O. | S.O. | S.O. | Ar¹-201 |
| **693** | S.O. | S.O. | Ar¹-132 | Ar¹-132 | **1413** | S.O. | S.O. | Ar¹-132 | Ar¹-132 |
| **694** | S.O. | S.O. | S.O. | Ar¹-134 | **1414** | S.O. | S.O. | S.O. | Ar¹-134 |
| **695** | S.O. | S.O. | S.O. | Ar¹-136 | **1415** | S.O. | S.O. | S.O. | Ar¹-136 |
| **696** | S.O. | S.O. | S.O. | Ar¹-137 | **1416** | S.O. | S.O. | S.O. | Ar¹-137 |
| **697** | S.O. | S.O. | S.O. | Ar¹-165 | **1417** | S.O. | S.O. | S.O. | Ar¹-165 |
| **698** | S.O. | S.O. | S.O. | Ar¹-200 | **1418** | S.O. | S.O. | S.O. | Ar¹-200 |
| **699** | S.O. | S.O. | S.O. | Ar¹-201 | **1419** | S.O. | S.O. | S.O. | Ar¹-201 |
| **700** | S.O. | S.O. | Ar¹-134 | Ar¹-134 | **1420** | S.O. | S.O. | Ar¹-134 | Ar¹-134 |
| **701** | S.O. | S.O. | S.O. | Ar¹-136 | **1421** | S.O. | S.O. | S.O. | Ar¹-136 |
| **702** | S.O. | S.O. | S.O. | Ar¹-137 | **1422** | S.O. | S.O. | S.O. | Ar¹-137 |
| **703** | S.O. | S.O. | S.O. | Ar¹-165 | **1423** | S.O. | S.O. | S.O. | Ar¹-165 |
| **704** | S.O. | S.O. | S.O. | Ar¹-200 | **1424** | S.O. | S.O. | S.O. | Ar¹-200 |
| **705** | S.O. | S.O. | S.O. | Ar¹-201 | **1425** | S.O. | S.O. | S.O. | Ar¹-201 |
| **706** | S.O. | S.O. | Ar¹-136 | Ar¹-136 | **1426** | S.O. | S.O. | Ar¹-136 | Ar¹-136 |
| **707** | S.O. | S.O. | S.O. | Ar¹-137 | **1427** | S.O. | S.O. | S.O. | Ar¹-137 |
| **708** | S.O. | S.O. | S.O. | Ar¹-165 | **1428** | S.O. | S.O. | S.O. | Ar¹-165 |
| **709** | S.O. | S.O. | S.O. | Ar¹-200 | **1429** | S.O. | S.O. | S.O. | Ar¹-200 |
| **710** | S.O. | S.O. | S.O. | Ar¹-201 | **1430** | S.O. | S.O. | S.O. | Ar¹-201 |
| **711** | S.O. | S.O. | Ar¹-137 | Ar¹-137 | **1431** | S.O. | S.O. | Ar¹-137 | Ar¹-137 |
| **712** | S.O. | S.O. | S.O. | Ar¹-165 | **1432** | S.O. | S.O. | S.O. | Ar¹-165 |
| **713** | S.O. | S.O. | S.O. | Ar¹-200 | **1433** | S.O. | S.O. | S.O. | Ar¹-200 |
| **714** | S.O. | S.O. | S.O. | Ar¹-201 | **1434** | S.O. | S.O. | S.O. | Ar¹-201 |
| **715** | S.O. | S.O. | Ar¹-165 | Ar¹-165 | **1435** | S.O. | S.O. | Ar¹-165 | Ar¹-165 |
| **716** | S.O. | S.O. | S.O. | Ar¹-200 | **1436** | S.O. | S.O. | S.O. | Ar¹-200 |
| **717** | S.O. | S.O. | S.O. | Ar¹-201 | **1437** | S.O. | S.O. | S.O. | Ar¹-201 |
| **718** | S.O. | S.O. | Ar¹-200 | Ar¹-200 | **1438** | S.O. | S.O. | Ar¹-200 | Ar¹-200 |
| **719** | S.O. | S.O. | S.O. | Ar¹-201 | **1439** | S.O. | S.O. | S.O. | Ar¹-201 |
| **720** | S.O. | S.O. | Ar¹-201 | Ar¹-201 | **1440** | S.O. | S.O. | Ar¹-201 | Ar¹-201 |

Die in der Tabelle genannten Grundkörper, die auch allgemein besonders bevorzugte Ausführungsformen von Verbindungen der Formel (I) sind, sind dabei die Folgenden:

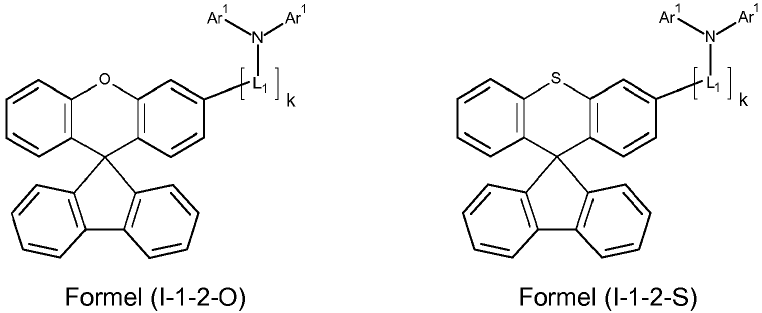

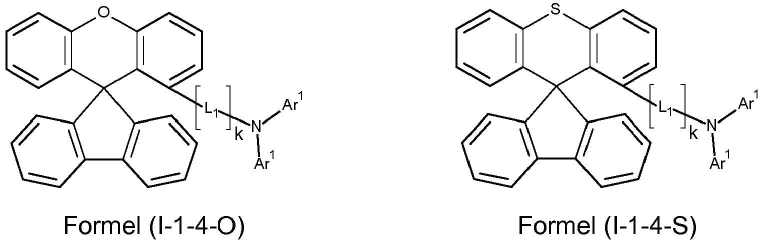

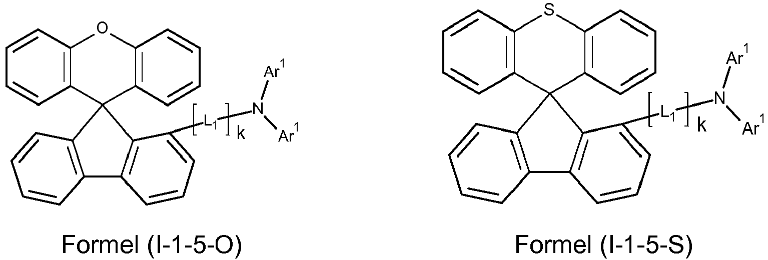

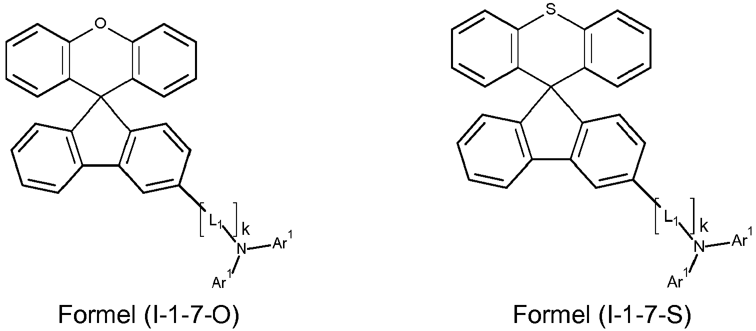

Bevorzugte Verbindungen der Formel (I) sind weiterhin in der folgenden Tabelle abgebildet:

| | | |
|---|---|---|
| 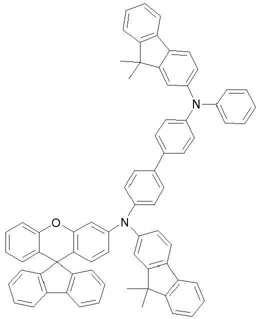 | 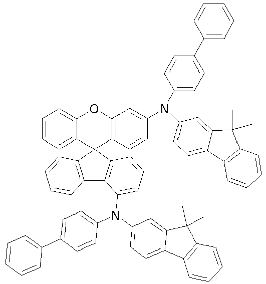 | 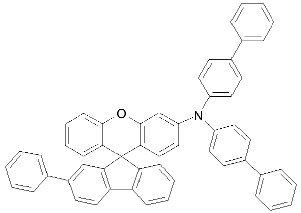 |
| 1 | 2 | 3 |
| 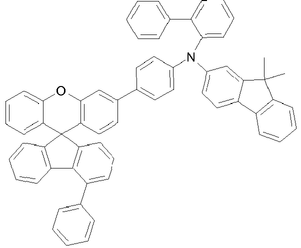 | 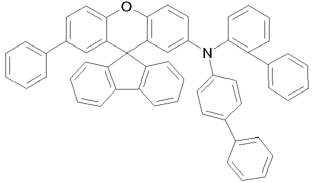 | 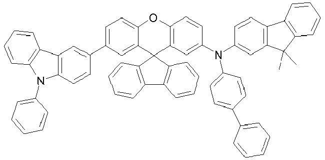 |
| 4 | 5 | 6 |
| 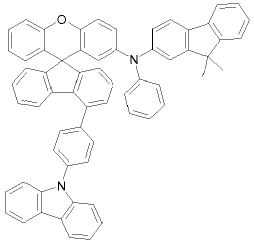 | 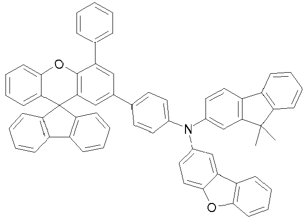 | 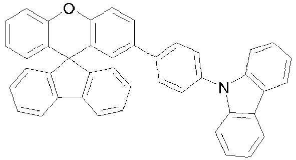 |
| 7 | 8 | 9 |
| 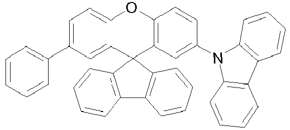 | 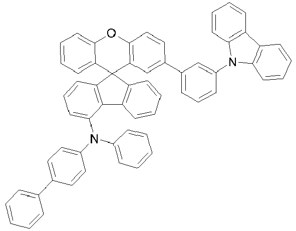 | 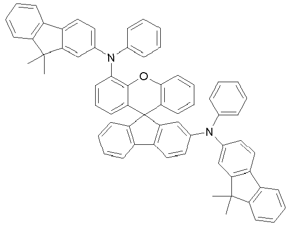 |
| 10 | 11 | 12 |
| 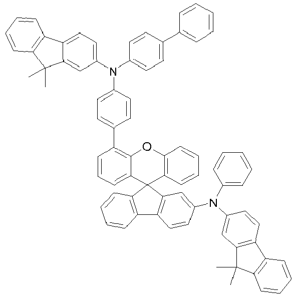 | 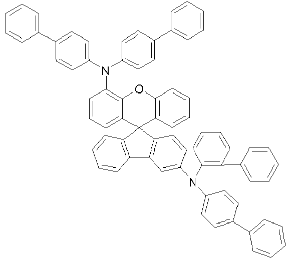 | 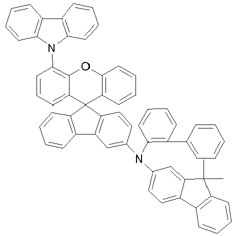 |
| 13 | 14 | 15 |
| 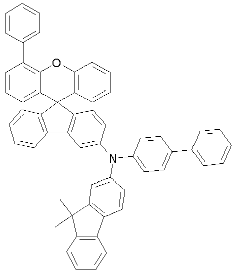 | 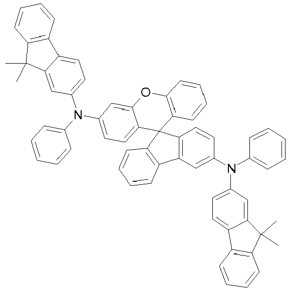 | 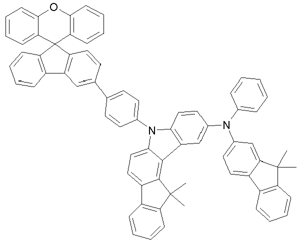 |
| 16 | 17 | 18 |
| 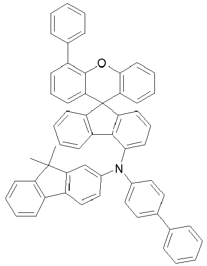 | 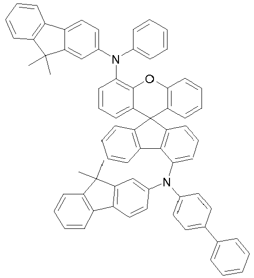 | 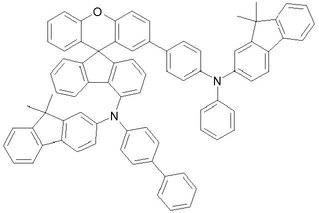 |
| 19 | 20 | 21 |
| 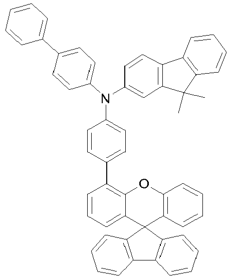 | 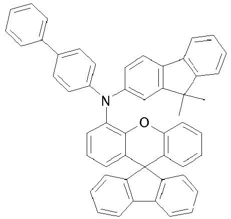 | 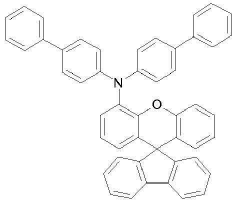 |
| 22 | 23 | 24 |
| 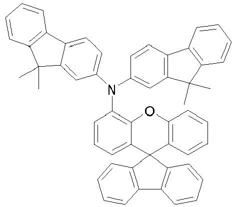 | 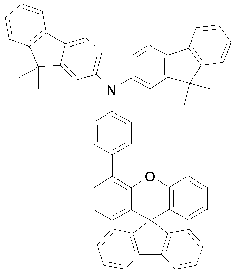 | 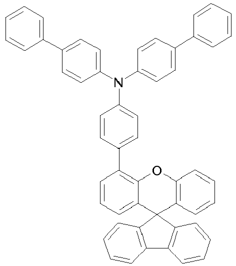 |
| 25 | 26 | 27 |
| 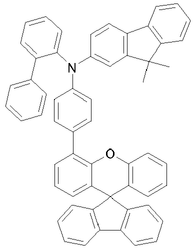 | 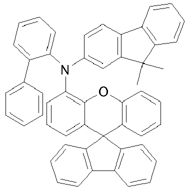 | 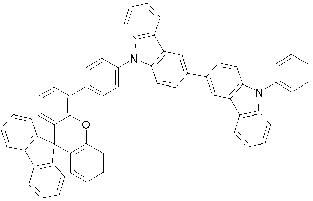 |
| 28 | 29 | 30 |
| 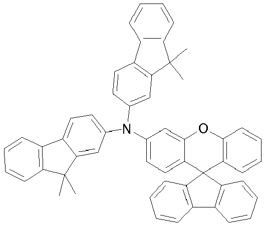 | 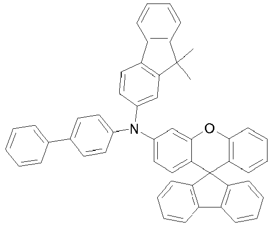 | 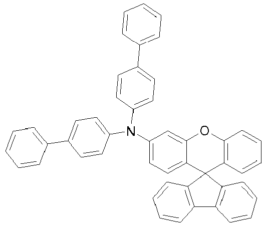 |
| 31 | 32 | 33 |
| 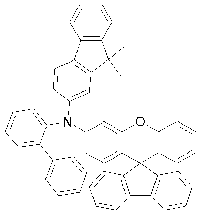 | 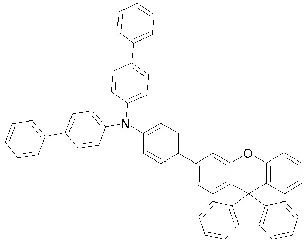 | 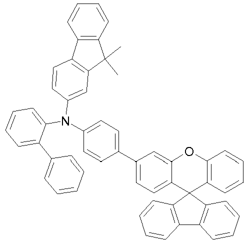 |
| 34 | 35 | 36 |
| 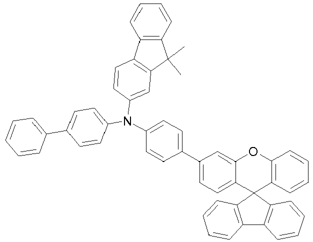 | 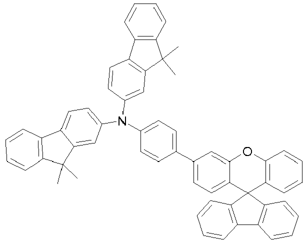 | 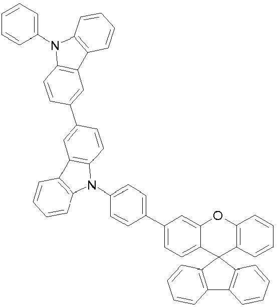 |
| 37 | 38 | 39 |
| 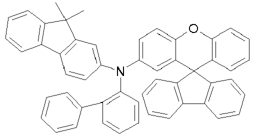 | 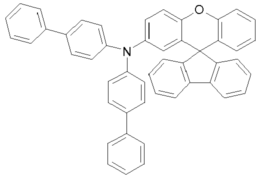 | 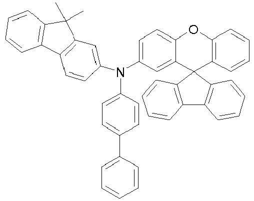 |
| 40 | 41 | 42 |
| 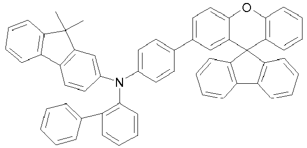 | 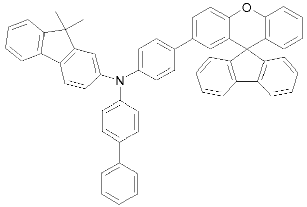 | 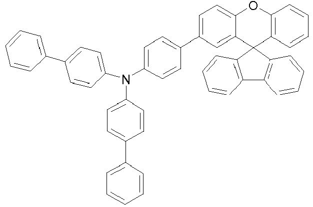 |
| 43 | 44 | 45 |
| 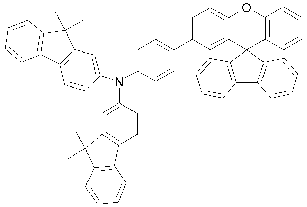 | 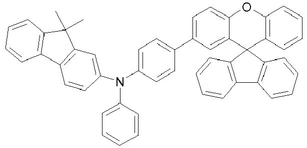 | 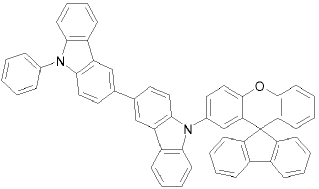 |
| 46 | 47 | 48 |
| 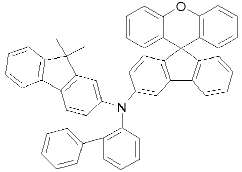 | 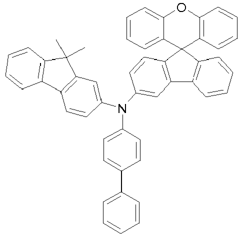 | 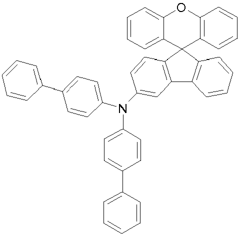 |
| 49 | 50 | 51 |
| 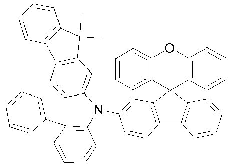 | 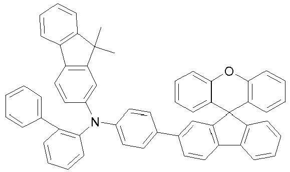 | 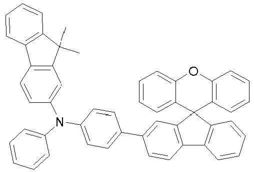 |
| 52 | 53 | 54 |
| 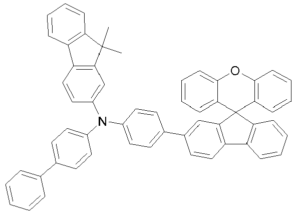 | 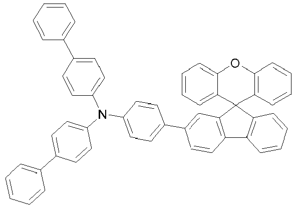 | 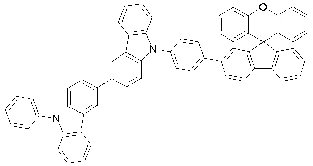 |
| 55 | 56 | 57 |
| 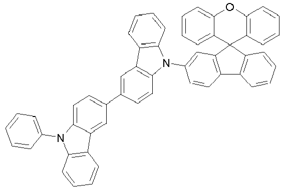 | 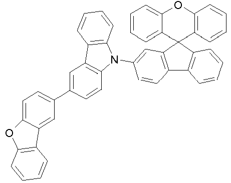 | 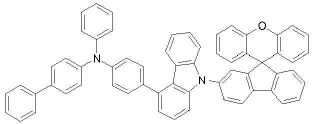 |
| 58 | 59 | 60 |
| 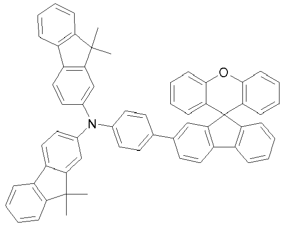 | 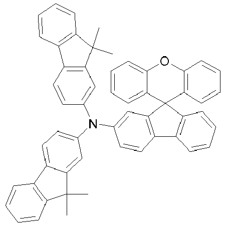 | 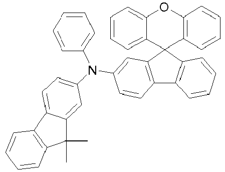 |
| 61 | 62 | 63 |
| 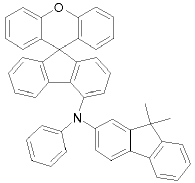 | 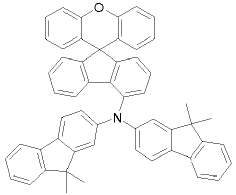 | 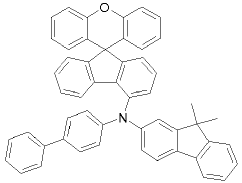 |
| 64 | 65 | 66 |
| 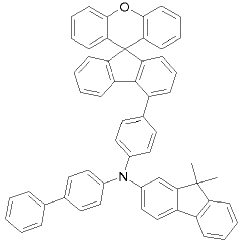 | 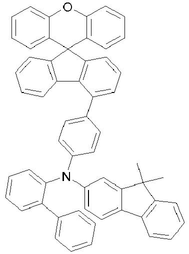 | 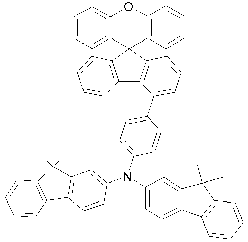 |
| 67 | 68 | 69 |
| 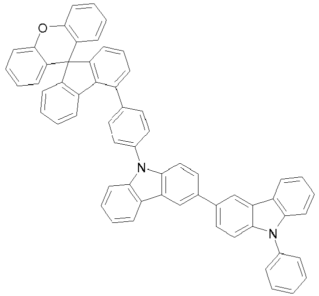 | 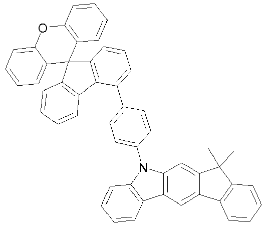 | |
| 70 | 71 | 72 |

Für die Synthese der Verbindungen der Formel (I) können im Stand der Technik bekannte Verfahren genutzt werden, insbesondere Verfahren, die in der Offenlegungsschrift WO 2014/072017 offenbart sind.

Die erfindungsgemäße Vorrichtung ist bevorzugt gewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und organischen Elektrolumineszenzvorrichtungen (OLEDs). Besonders bevorzugt ist sie eine organische Elektrolumineszenzvorrichtung.

In der erfindungsgemäßen elektronischen Vorrichtung ist die Verbindung der Formel (I) bevorzugt in einer Schicht enthalten, die an die Anode angrenzend angeordnet ist. Diese Schicht enthält bevorzugt einen p-Dotanden. Als p-Dotanden werden gemäß der vorliegenden Erfindung bevorzugt solche organischen Elektronenakzeptorverbindungen eingesetzt, die eine oder mehrere der anderen Verbindungen der Mischung oxidieren können.

Besonders bevorzugte Ausführungsformen von p-Dotanden sind die in WO 2011/073149, EP 1968131, EP 2276085, EP 2213662, EP 1722602, EP 2045848, DE 102007031220, US 8044390, US 8057712, WO 2009/003455, WO 2010/094378, WO 2011/120709, US 2010/0096600, WO 2012/095143 und DE 102012209523 offenbarten Verbindungen.

Besonders bevorzugt als p-Dotanden sind Chinodimethanverbindungen, Azaindenofluorendione, Azaphenalene, Azatriphenylene, I₂, Metallhalogenide, bevorzugt Übergangsmetallhalogenide, Metalloxide, bevorzugt Metalloxide enthaltend mindestens ein Übergangsmetall oder ein Metall der 3. Hauptgruppe, und Übergangsmetallkomplexe, bevorzugt Komplexe von Cu, Co, Ni, Pd und Pt mit Liganden enthaltend mindestens ein Sauerstoffatom als Bindungsstelle. Bevorzugt sind weiterhin Übergangsmetalloxide als Dotanden, bevorzugt Oxide von Rhenium, Molybdän und Wolfram, besonders bevorzugt Re₂O₇, MoOs, WO₃ und ReOs.

Die p-Dotanden liegen bevorzugt weitgehend gleichmäßig verteilt in den p-dotierten Schichten vor. Dies kann beispielsweise durch Co-Verdampfung des p-Dotanden und der Lochtransportmaterial-Matrix erreicht werden. Bevorzugt liegen p-Dotanden in einem Anteil von insgesamt 0.5% bis 10 Vol.-%, bevorzugt 0.8 bis 8 % Vol.-% in der betreffenden Schicht vor.

Bevorzugt sind als p-Dotanden insbesondere die folgenden Verbindungen:

| | | |
|---|---|---|
| 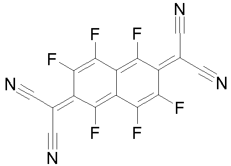 | 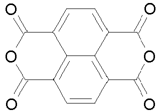 | 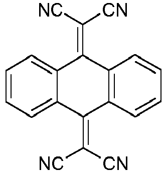 |
| (D-1) | (D-2) | (D-3) |
| 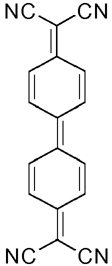 | 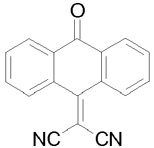 | 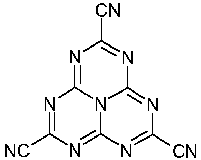 |
| (D-4) | (D-5) | (D-6) |
| 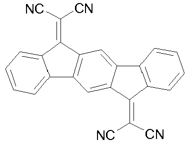 | 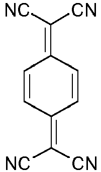 | 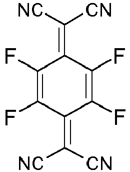 |
| (D-7) | (D-8) | (D-9) |
| 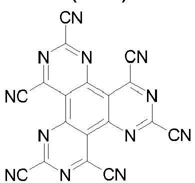 | 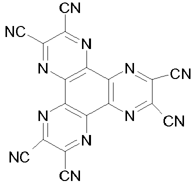 | 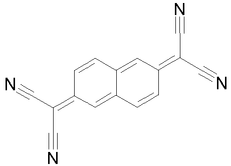 |
| (D-10) | (D-11) | (D-12) |
| 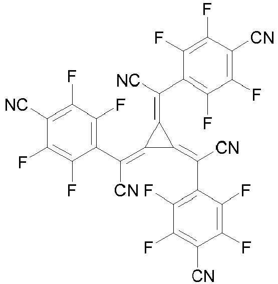 | | |
| (D-13) | | |

Weiterhin ist es bevorzugt, dass als weiteres Merkmal der erfindungsgemäßen elektronischen Vorrichtung zwischen der Schicht enthaltend die Verbindung der Formel (I) und der anodennächsten emittierenden Schicht mindestens eine weitere Schicht vorhanden ist, welche keine Verbindung der Formel (I) aufweist.

Es ist bevorzugt, dass die Schicht, welche anodenseitig an die anodennächste emittierende Schicht angrenzt, keine Verbindung der Formel (I) aufweist.

Nach dem Gegenstand der vorliegenden Erfindung, erfüllt die Vorrichtung die folgende Bedingung: zwischen der lochtransportierenden Schicht und der emittierenden Schicht sind mindestens zwei weitere Schichten angeordnet, und es sind keine weiteren emittierenden Schichten zwischen der emittierenden Schicht und der Anode angeordnet.

Bevorzugt ist eine elektronische Vorrichtung, welche in der genannten Reihenfolge die folgenden Schichten enthält: Anode, lochtransportierende Schicht HTL1, lochtransportierende Schicht HTL2, lochtransportierende Schicht HTL3, emittierende Schicht EML, elektronentransportierende Schicht ETL, und Kathode, wobei weitere Schichten vorhanden sein können, wobei die Schicht HTL1 an die Anode angrenzt, wobei die Schicht HTL3 an die emittierende Schicht angrenzt, und wobei die Schicht HTL1 eine Verbindung der Formel (I) enthält. Bevorzugt enthält dabei die Schicht HTL3 keine Verbindung der Formel (I).

Eine besonders bevorzugte Ausführungsform der Vorrichtung weist die folgende Schichtabfolge zwischen Anode und anodennächster emittierender Schicht auf: Anode, lochtransportierende Schicht HTL1 enthaltend eine Verbindung der Formel (I), lochtransportierende Schicht HTL2, lochtransportierende Schicht HTL3 enthaltend keine Verbindung der Formel (I), anodennächste emittierende Schicht. Dabei sind bevorzugt keine weiteren Schichten zwischen Anode und anodennächster emittierender Schicht vorhanden. Die Schicht HTL1 weist dabei bevorzugt eine Dicke von 5 bis 50 nm auf. Die Schicht HTL2 weist dabei bevorzugt eine Dicke von 5 bis 250 nm auf. Die Schicht HTL3 weist dabei bevorzugt eine Dicke von 5 bis 120 nm auf.

Eine alternative besonders bevorzugte Ausführungsform der Vorrichtung weist die folgende Schichtabfolge zwischen Anode und anodennächster emittierender Schicht auf: Anode, lochtransportierende Schicht HTL1 enthaltend eine Verbindung der Formel (I) und einen p-Dotanden, lochtransportierende Schicht HTL2, lochtransportierende Schicht HTL3 enthaltend keine Verbindung der Formel (I), anodennächste emittierende Schicht. Dabei sind bevorzugt keine weiteren Schichten zwischen Anode und anodennächster emittierender Schicht vorhanden. Die Schicht HTL1 weist dabei bevorzugt eine Dicke von 5 bis 250 nm auf. Die Schicht HTL2 weist dabei bevorzugt eine Dicke von 5 bis 250 nm auf. Die Schicht HTL3 weist dabei bevorzugt eine Dicke von 5 bis 120 nm auf.

Eine alternative besonders bevorzugte Ausführungform der Vorrichtung weist die folgende Schichtabfolge zwischen Anode und anodennächster emittierender Schicht auf: Anode, lochtransportierende Schicht HTL1 enthaltend eine Verbindung der Formel (I) und einen p-Dotanden, lochtransportierende Schicht HTL2a, lochtransportierende Schicht HTL2b enthaltend einen p-Dotanden, lochtransportierende Schicht HTL3 enthaltend keine Verbindung der Formel (I), anodennächste emittierende Schicht. Dabei sind bevorzugt keine weiteren Schichten zwischen Anode und anodennächster emittierender Schicht vorhanden.

Die lochtransportierende Schicht, die anodenseitig an die anodennächste emittierende Schicht angrenzt, enthält bevorzugt eine Monoamin-Verbindung. Unter einer Monoamin-Verbindung wird dabei eine Verbindung verstanden, die nur eine Aminogruppe enthält. Bevorzugt ist diese Aminogruppe eine Diarylaminogruppe. Unter einer Diarylaminogruppe wird eine Gruppe verstanden, in der an das Amino-Stickstoffatom zwei Gruppen gewählt aus Arylgruppen und Heteroarylgruppen gebunden sind.

Besonders bevorzugt enthält die lochtransportierende Schicht, die anodenseitig an die anodennächste emittierende Schicht angrenzt, eine Monoamin-Verbindung, die mindestens eine Gruppe gewählt aus Spirobifluorenylgruppen, Phenanthrenylgruppen, Fluorenylgruppen, Carbazolylgruppen, Dibenzofuranylgruppen und Dibenzothiophenylgruppen enthält. Besonders bevorzugt sind darunter Spirobifluorenyl-Monoamine, welche an einer der Positionen 1, 3 und 4 am Spirobifluoren-Grundgerüst eine Diarylaminogruppe tragen, darunter insbesondere die Verbindungen, die in der Offenlegungsschrift WO 2013/120577 auf den Seiten 36-51 und 88-122 offenbart sind. Ganz besonders bevorzugt sind Spirobifluorenyl-Monoamine, die an Position 4 am Spirobifluoren-Grundgerüst eine Diarylaminogruppe tragen, darunter insbesondere die Verbindungen, die in der Offenlegungsschrift WO 2013/120577 auf den Seiten 36-51 und 88-122 offenbart sind.

Es ist bevorzugt, dass die Monoamin-Verbindung, die in der lochtransportierenden Schicht enthalten ist, welche anodenseitig an die anodennächste emittierende Schicht angrenzt, ein HOMO-Energieniveau von 5.0 bis 5.6 eV aufweist, besonders bevorzugt 5.1 bis 5.5 eV aufweist. Das HOMO-Energieniveau wird dabei mittels Cyclovoltammetrie (CV) bestimmt, nach dem auf S. 28, Z.1 bis S. 29, Z. 21 der Offenlegungsschrift WO 2011/032624 beschriebenen Verfahren.

Die Vorrichtung kann zusätzlich zu den genannten Schichten weitere Schichten aufweisen, darunter insbesondere Schichten gewählt aus Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) und organischen oder anorganischen p/n-Übergängen.

Die Vorrichtung enthält bevorzugt nur eine emittierende Schicht. Sie kann jedoch auch mehrere emittierende Schichten enthalten. In diesem Fall weisen diese mehreren emittierenden Schichten bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues, grünes, gelbes, orangefarbenes oder rotes Licht emittieren. Insbesondere bevorzugt sind Dreischichtsysteme, also Systeme mit drei emittierenden Schichten, wobei die drei Schichten blaue, grüne und orangefarbene oder rote Emission zeigen.

Die emittierende Schicht der Vorrichtung kann eine fluoreszierende emittierende Schicht sein, oder sie kann eine phosphoreszierende emittierende Schicht sein.

Unter phosphoreszierenden emittierenden Schichten werden insbesondere Schichten verstanden, welche mindestens einen phosphoreszierenden Emitter enthalten. Vom Begriff phosphoreszierende Emitter sind Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Emitter (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende Emitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten. Dabei werden im Sinne der vorliegenden Erfindung alle lumineszierenden Iridium-, Platin- oder Kupferkomplexe als phosphoreszierende Emitter angesehen.

Bevorzugt ist die phosphoreszierende emittierende Schicht der Vorrichtung eine grün oder rot phosphoreszierende Schicht. Weiterhin bevorzugt ist die fluoreszierende emittierende Schicht der Vorrichtung eine blau fluoreszierende Schicht.

Die emittierenden Schichten enthalten bevorzugt mindestens ein Matrixmaterial und mindestens einen Emitter.

Insbesondere im Fall von phosphoreszierenden emittierenden Schichten ist es bevorzugt, dass die betreffende Schicht zwei oder mehr unterschiedliche Matrixmaterialien enthält, bevorzugt zwei oder drei, ganz besonders bevorzugt zwei (Mixed-Matrix-Systeme). Bevorzugt stellt dabei eines der beiden Matrixmaterialien ein Material mit lochtransportierenden Eigenschaften und das andere Matrixmaterial ein Material mit elektronentransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen.

Im Folgenden ist offenbart, welche Materialklassen bevorzugt in den betreffenden Funktionsschichten der Vorrichtung verwendet werden.

Bevorzugte phosphoreszierende Emitter zur Verwendung in der emittierenden Schicht können den Anmeldungen WO 00/70655, WO 01/41512, WO 02/02714, WO 02/15645, EP 1191613, EP 1191612, EP 1191614, WO 05/033244, WO 05/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind.

Bevorzugte fluoreszierende Emitter sind ausgewählt aus der Klasse der Arylamine. Unter einem Arylamin bzw. einem aromatischen Amin im Sinne dieser Erfindung wird dabei eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte emittierende Verbindungen sind Indenofluorenamine bzw. - diamine, beispielsweise gemäß WO 2006/108497 oder WO 2006/122630, Benzoindenofluorenamine bzw. -diamine, beispielsweise gemäß WO 2008/006449, und Dibenzoindenofluorenamine bzw. -diamine, beispielsweise gemäß WO 2007/140847, sowie die in WO 2010/012328 offenbarten Indenofluorenderivate mit kondensierten Arylgruppen. Ebenfalls bevorzugt sind die in WO 2012/048780 und die in WO 2013/185871 offenbarten Pyren-Arylamine. Ebenfalls bevorzugt sind die in WO 2014/037077 offenbarten Benzoindenofluoren-Amine, die in WO 2014/106522 offenbarten Benzofluoren-Amine, die in WO 2014/111269 und in WO 2017/036574 offenbarten erweiterten Benzoindenofluorene, die in WO 2017/028940 und WO 2017/028941 offenbarten Phenoxazine, und die in WO 2016/150544 offenbarten Fluoren-Derivate, die mit Furan-Einheiten oder mit Thiophen-Einheiten verbunden sind.

Als Matrixmaterialien, bevorzugt für fluoreszierende emittierende Schichten, kommen Materialien verschiedener Stoffklassen in Frage. Bevorzugte Matrixmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind. Bevorzugt sind weiterhin die in WO 2006/097208, WO 2006/131192, WO 2007/065550, WO 2007/110129, WO 2007/065678, WO 2008/145239, WO 2009/100925, WO 2011/054442, und EP 1553154 offenbarten Anthracenderivate, die in EP 1749809, EP 1905754 und US 2012/0187826 offenbarten Pyren-Verbindungen, die in WO 2015/158409 offenbarten Benzanthracenyl-Anthracen-Verbindungen, die in WO 2017/025165 offenbarten Indeno-Benzofurane, und die in WO 2017/036573 offenbarten Phenanthryl-Anthracene.

Bevorzugte Matrixmaterialien für phosphoreszierende emittierende Verbindungen sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109, WO 2011/000455 oder WO 2013/041176, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte Carbazol-Derivate, z. B. gemäß US 2009/0136779, WO 2010/050778, WO 2011 /042107, WO 2011 /088877 oder WO 2012/143080, Triphenylenderivaten, z. B. gemäß WO 2012/048781, oder Lactame, z. B. gemäß WO 2011/116865 oder WO 2011/137951.

Weitere Verbindungen, die neben den Verbindungen der Formel (I) bevorzugt in lochtransportierenden Schichten der erfindungsgemäßen OLEDs eingesetzt werden, sind insbesondere Indenofluorenamin-Derivate (z. B. gemäß WO 06/122630 oder WO 06/100896), die in EP 1661888 offenbarten Aminderivate, Hexaazatriphenylenderivate (z. B. gemäß WO 01/049806), Aminderivate mit kondensierten Aromaten (z. B. gemäß US 5,061,569), die in WO 95/09147 offenbarten Aminderivate, Monobenzoindenofluorenamine (z. B. gemäß WO 08/006449), Dibenzoindenofluorenamine (z. B. gemäß WO 07/140847), Spirobifluoren-Amine (z. B. gemäß WO 2012/034627 oder WO 2013/120577), Fluoren-Amine (z. B. gemäß WO 2014/015937, WO 2014/015938, WO 2014/015935 und WO 2015/082056), Spiro-Dibenzopyran-Amine (z. B. gemäß WO 2013/083216), Dihydroacridin-Derivate (z. B. gemäß WO 2012/150001), Spirodibenzofurane und Spirodibenzothiophene, z.B. gemäß WO 2015/022051 und WO 2016/102048 und WO 2016/131521, Phenanthren-Diarylamine, z.B. gemäß WO 2015/131976, Spiro-Tribenzotropolone, z.B. gemäß WO 2016/087017, Spirobifluorene mit meta-Phenyldiamingruppen, z.B. gemäß WO 2016/078738, Spiro-Bisacridine, zB. gemäß WO 2015/158411, Xanthen-Diarylamine, z.B. gemäß WO 2014/072017, und 9,10-Dihydroanthracen-Spiroverbindungen mit Diarylaminogruppen gemäß WO 2015/086108.

Als Materialien für die Elektronentransportschicht können alle Materialien verwendet werden, wie sie gemäß dem Stand der Technik als Elektronentransportmaterialien in der Elektronentransportschicht verwendet werden. Insbesondere eignen sich Aluminiumkomplexe, beispielsweise Alq₃, Zirkoniumkomplexe, beispielsweise Zrq₄, Lithiumkomplexe, beispielsweise Liq, Benzimidazolderivate, Triazinderivate, Pyrimidinderivate, Pyridinderivate, Pyrazinderivate, Chinoxalinderivate, Chinolinderivate, Oxadiazolderivate, aromatische Ketone, Lactame, Borane, Diazaphospholderivate und Phosphinoxidderivate. Weiterhin geeignete Materialien sind Derivate der oben genannten Verbindungen, wie sie in JP 2000/053957, WO 2003/060956, WO 2004/028217, WO 2004/080975 und WO 2010/072300 offenbart werden.

Als Kathode der elektronischen Vorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere. Weiterhin kann die Anode auch aus mehreren Schichten bestehen, beispielsweise aus einer inneren Schicht aus ITO und einer äußeren Schicht aus einem Metalloxid, bevorzugt Wolframoxid, Molybdänoxid oder Vanadiumoxid.

Die Vorrichtung wird (je nach Anwendung) strukturiert, kontaktiert und schließlich versiegelt, um schädigende Effekte von Wasser und Luft auszuschließen.

In einer bevorzugten Ausführungsform ist die elektronische Vorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine elektronische Vorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden.

Weiterhin bevorzugt ist eine elektronische Vorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (I) nötig. Hohe Löslichkeit lässt sich durch geeignete Substitution der Verbindungen erreichen.

Weiterhin bevorzugt ist es, dass zur Herstellung einer erfindungsgemäßen elektronischen Vorrichtung eine oder mehrere Schichten aus Lösung und eine oder mehrere Schichten durch ein Sublimationsverfahren aufgetragen werden.

Erfindungsgemäß können die elektronischen Vorrichtungen in Displays, als Lichtquellen in Beleuchtungsanwendungen sowie als Lichtquellen in medizinischen und/oder kosmetischen Anwendungen (z.B. Lichttherapie) eingesetzt werden.

Weiterer Gegenstand der Erfindung ist eine Verbindung als solche, die einer Formel (S) entspricht 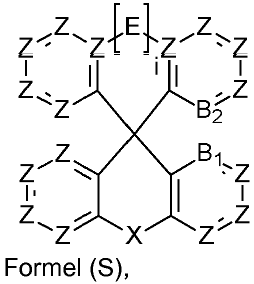 wobei an B₁ eine Gruppe A gebunden sein muss, und wobei für die auftretenden Variablen gilt:
- B₂ ist: N oder CR² oder
- C, und B₁: ist C;
- Z: ist bei jedem Auftreten gleich oder verschieden CR² oder N oder C, wobei eine Gruppe Z genau dann gleich C ist, wenn eine Gruppe E an die betreffende Gruppe Z gebunden ist;
- A: ist eine Arylaminogruppe, wahlweise substituiert mit einem oder mehreren Resten R¹, oder eine Carbazol enthaltende Gruppe, wahlweise substituiert mit einem oder mehreren Resten R¹;
- E: ist eine Einfachbindung;
- X: ist O oder S;
- R¹: ist bei jedem Auftreten gleich oder verschieden gewählt aus H, D, F, C(=O)R³, CN, Si(R³)₃, N(R³)₂, P(=O)(R³)₂, OR³, S(=O)R³, S(=O)₂R³, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R¹ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R³ substituiert sein können; und wobei eine oder mehrere CH₂-Gruppen in den genannten Alkyl-, Alkoxy-, Alkenyl- und Alkinylgruppen durch -R³C=CR³-, -C=C-, Si(R³)₂, C=O, C=NR³, -C(=O)O-, -C(=O)NR³-, NR³, P(=O)(R³), -O-, -S-, SO oder SO₂ ersetzt sein können;
- R²: ist bei jedem Auftreten gleich oder verschieden gewählt aus H, D, F, C(=O)R³, CN, Si(R³)₃, P(=O)(R³)₂, OR³, S(=O)R³, S(=O)₂R³, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R² miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R³ substituiert sein können; und wobei eine oder mehrere CH₂-Gruppen in den genannten Alkyl-, Alkoxy-, Alkenyl- und Alkinylgruppen durch -R³C=CR³-, -C=C-, Si(R³)₂, C=O, -C(=O)O-, -C(=O)NR³-, P(=O)(R³), -O-, -S-, SO oder SO₂ ersetzt sein können;
- R³: ist bei jedem Auftreten gleich oder verschieden gewählt aus H, D, F, C(=O)R⁴, CN, Si(R⁴)₃, N(R⁴)₂, P(=O)(R⁴)₂, OR⁴, S(=O)R⁴, S(=O)₂R⁴, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R³ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁴ substituiert sein können; und wobei eine oder mehrere CH₂-Gruppen in den genannten Alkyl-, Alkoxy-, Alkenyl- und Alkinylgruppen durch -R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können;
- R⁴: ist bei jedem Auftreten gleich oder verschieden gewählt aus H, D, F, CN, Alkyl- oder Alkoxygruppen mit 1 bis 20 C-Atomen, Alkenyl- oder Alkinylgruppen mit 2 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R⁴ miteinander verknüpft sein können und einen Ring bilden können; und wobei die genannten Alkyl-, Alkoxy-, Alkenyl- und Alkinylgruppen, aromatischen Ringsysteme und heteroaromatischen Ringsysteme mit F oder CN substituiert sein können;
- i: ist gleich 0 oder 1.

Unter dem Begriff Arylaminogruppe und Carbazolgruppe als Gruppe A werden dabei Gruppen gemäß der obenstehenden Definition verstanden.

In der Verbindung der Formel (S) ist X bevorzugt gleich O.

Weiterhin ist i bevorzugt gleich 1.

Weiterhin sind bevorzugt höchstens 2 Gruppen Z pro Ring gleich N. Weiterhin bevorzugt sind höchstens 4 Gruppen Z pro Verbindung der Formel (S), ganz besonders bevorzugt höchstens 2 Gruppen Z pro Verbindung der Formel (S) gleich Z.

Besonders bevorzugt ist Z gleich CR², wobei in dem Fall, dass eine Gruppe E an die betreffende Gruppe Z gebunden ist, diese Gruppe Z gleich C ist.

Betreffend die Variablen R¹ bis R³ gelten die oben angegebenen bevorzugten Ausführungsformen.

Bevorzugt ist die Gruppe A eine Arylaminogruppe, die wahlweise mit einem oder mehreren Resten R¹ substituiert ist. Die Gruppe A als Arylaminogruppe ist bevorzugt definiert wie oben angegeben und entspricht bevorzugt der Formel (A), wie oben angegeben.

Bevorzugte Ausführungsformen der Verbindung der Formel (S) sind Verbindungen der Formel (S-2) 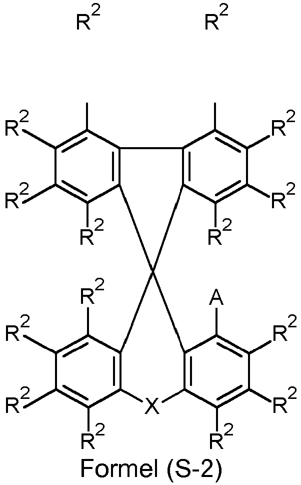 wobei die auftretenden Variablen wie oben definiert sind.

Verschiedene Verbindungen sind im Folgenden abgebildet. Jedoch entsprechen nur die Verbindungen S-70 bis S-84 der Formel (S) von Anspruch 13.

| | | |
|---|---|---|
| 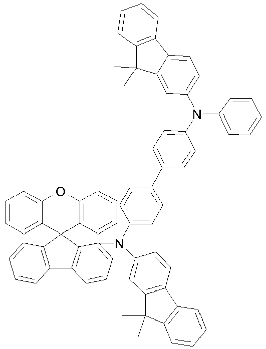 | 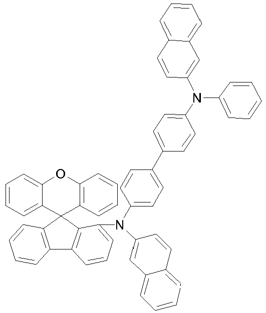 | 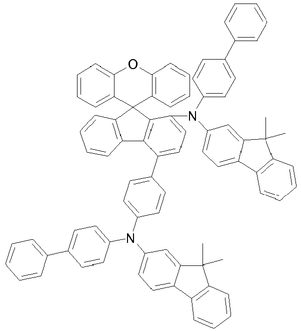 |
| Formel (S-1) | Formel (S-2) | Formel (S-3) |
| 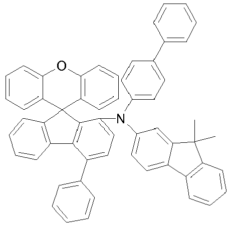 | 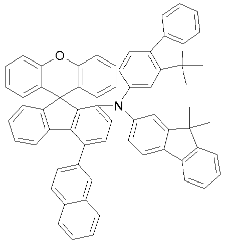 | 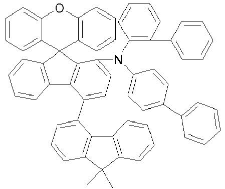 |
| Formel (S-4) | Formel (S-5) | Formel (S-6) |
| 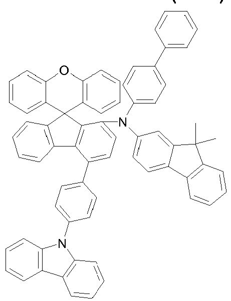 | 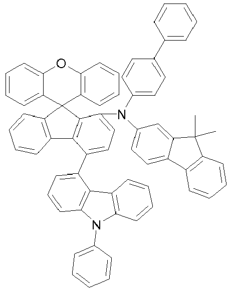 | 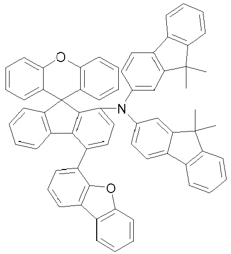 |
| Formel (S-7) | Formel (S-8) | Formel (S-9) |
| 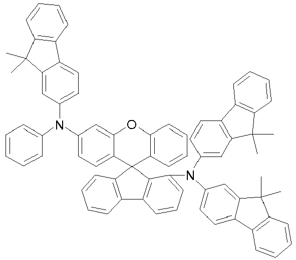 | 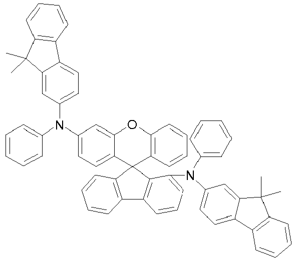 | 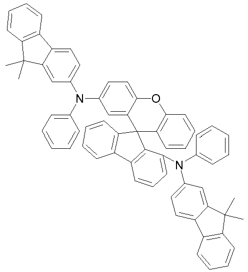 |
| Formel (S-10) | Formel (S-11) | Formel (S-12) |
| 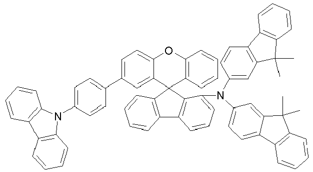 | 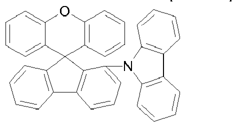 | 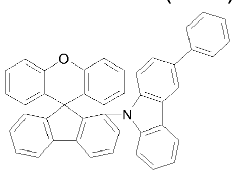 |
| Formel (S-13) | Formel (S-14) | Formel (S-15) |
| 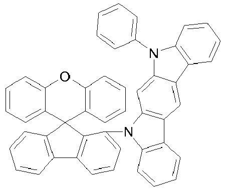 | 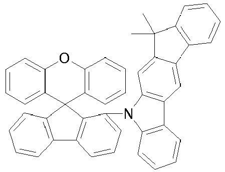 | 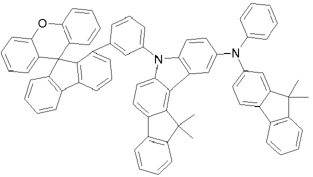 |
| Formel (S-16) | Formel (S-17) | Formel (S-18) |
| 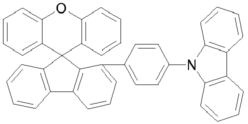 | 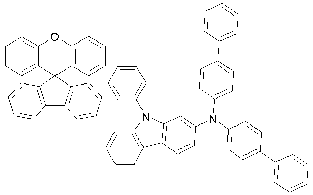 | 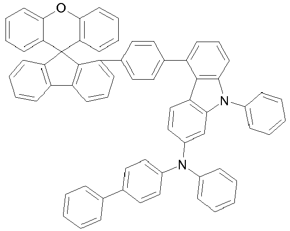 |
| Formel (S-19) | Formel (S-20) | Formel (S-21) |
| 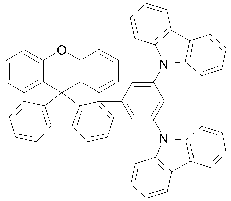 | 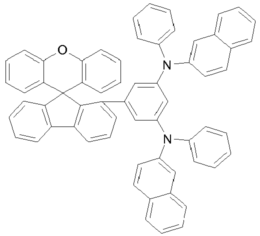 | 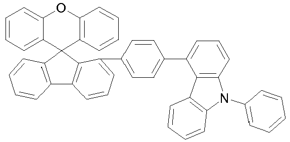 |
| Formel (S-22) | Formel (S-23) | Formel (S-24) |
| 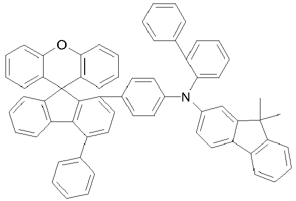 | 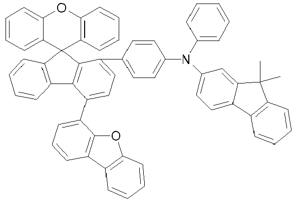 | 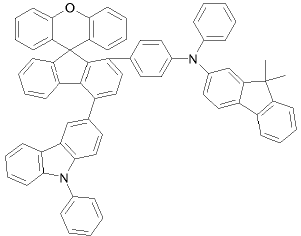 |
| Formel (S-25) | Formel (S-26) | Formel (S-27) |
| 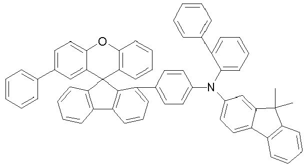 | 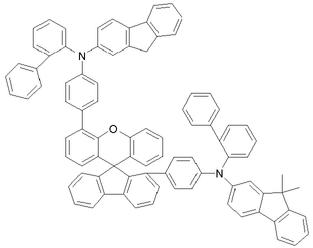 | 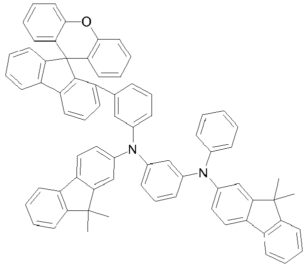 |
| Formel (S-28) | Formel (S-29) | Formel (S-30) |
| 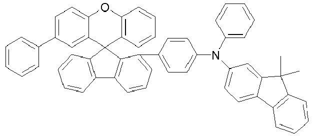 | 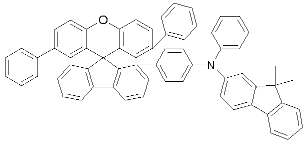 | 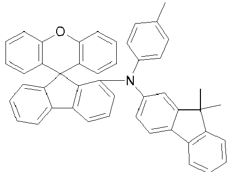 |
| Formel (S-31) | Formel (S-32) | Formel (S-33) |
| 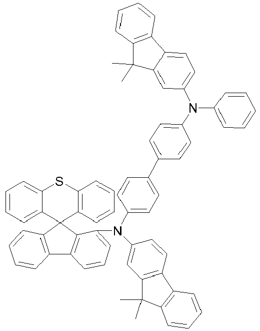 | 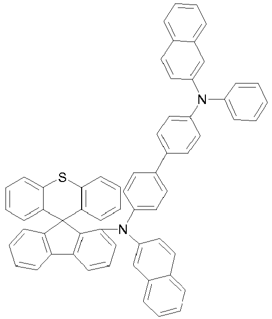 | 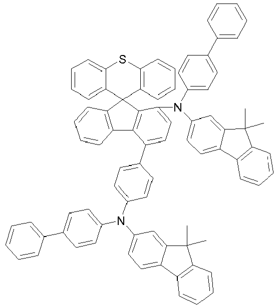 |
| Formel (S-34) | Formel (S-35) | Formel (S-36) |
| 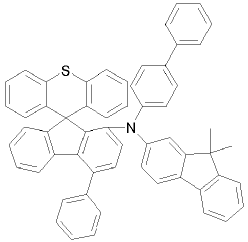 | 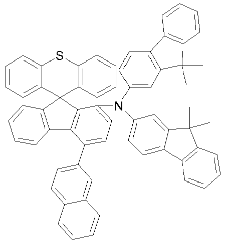 | 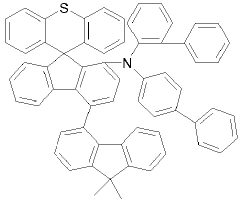 |
| Formel (S-37) | Formel (S-38) | Formel (S-39) |
| 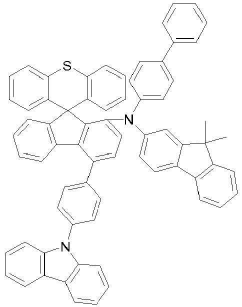 | 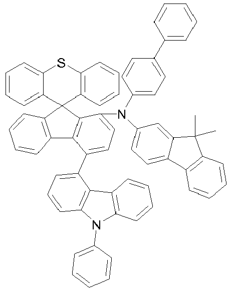 | 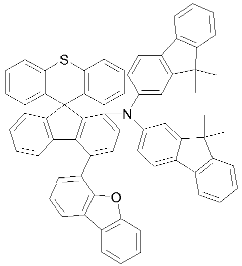 |
| Formel (S-40) | Formel (S-41) | Formel (S-42) |
| 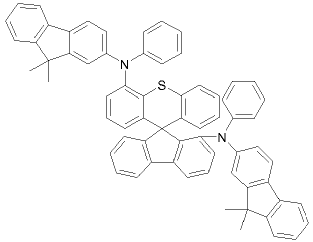 | 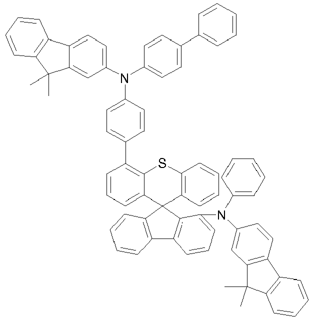 | 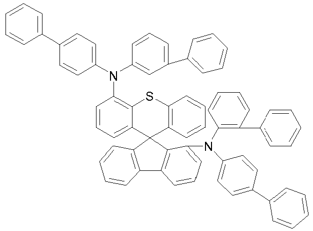 |
| Formel (S-43) | Formel (S-44) | Formel (S-45) |
| 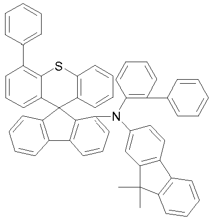 | 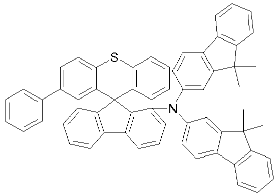 | 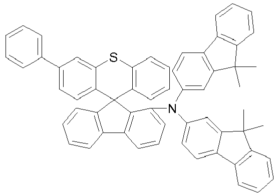 |
| Formel (S-46) | Formel (S-47) | Formel (S-48) |
| 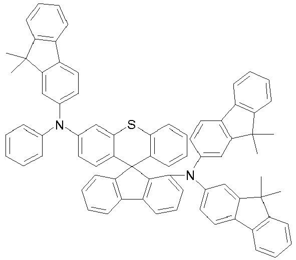 | 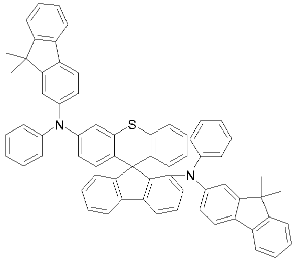 | 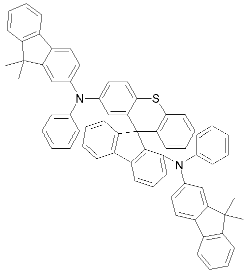 |
| Formel (S-49) | Formel (S-50) | Formel (S-51) |
| 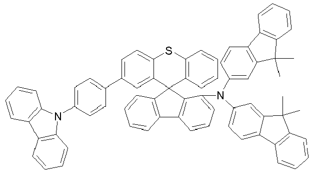 | 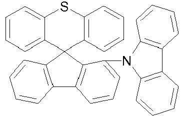 | 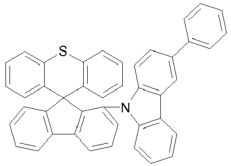 |
| Formel (S-52) | Formel (S-53) | Formel (S-54) |
| 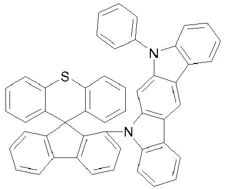 | 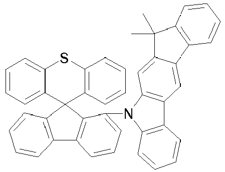 | 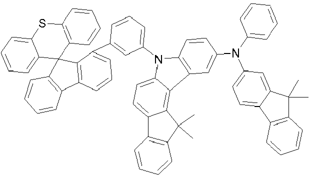 |
| Formel (S-55) | Formel (S-56) | Formel (S-57) |
| 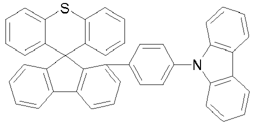 | 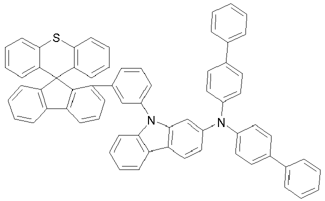 | 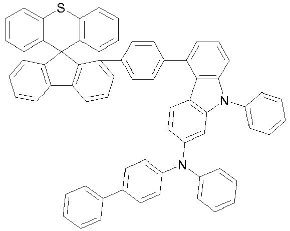 |
| Formel (S-58) | Formel (S-59) | Formel (S-60) |
| 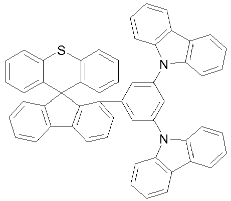 | 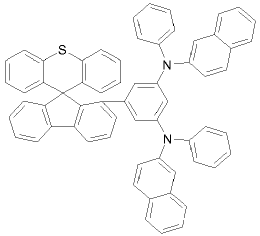 | 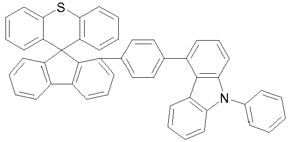 |
| Formel (S-61) | Formel (S-62) | Formel (S-63) |
| 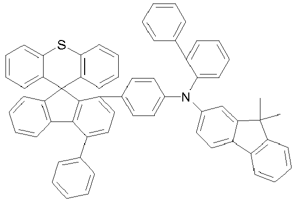 | 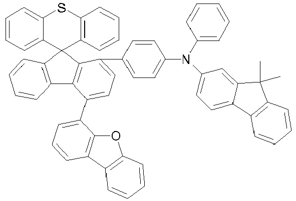 | 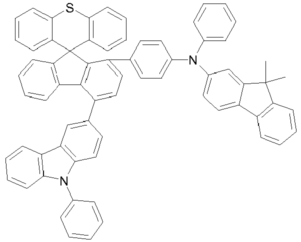 |
| Formel (S-64) | Formel (S-65) | Formel (S-66) |
| 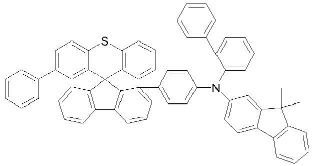 | 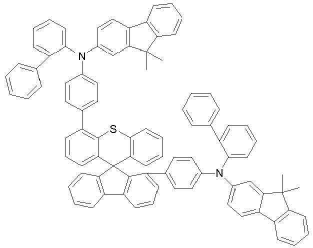 | 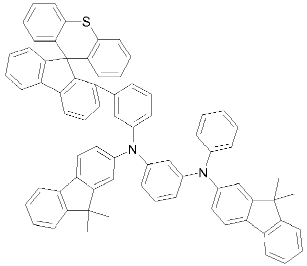 |
| Formel (S-67) | Formel (S-68) | Formel (S-69) |
| 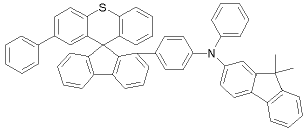 | 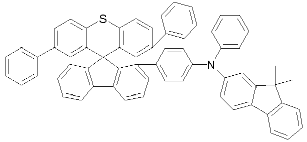 | |
| Formel (S-70) | Formel (S-71) | Formel (S-72) |
| 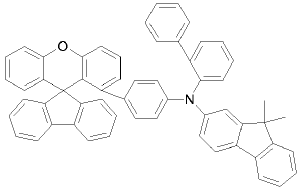 | 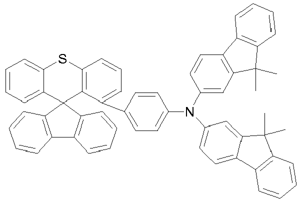 | 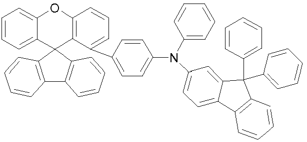 |
| Formel (S-73) | Formel (S-74) | Formel (S-75) |
| 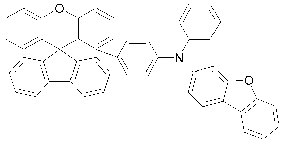 | 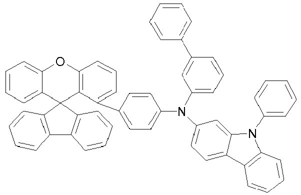 | 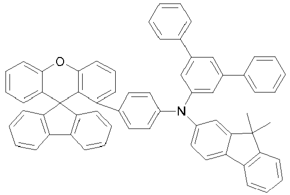 |
| Formel (S-76) | Formel (S-77) | Formel (S-78) |
| 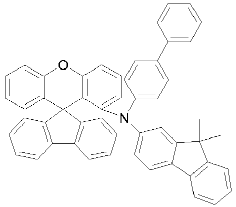 | 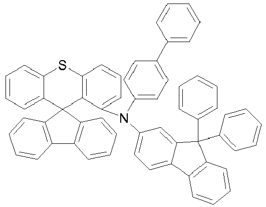 | 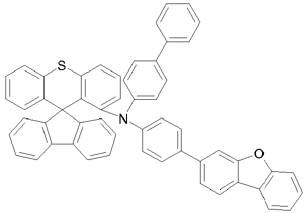 |
| Formel (S-79) | Formel (S-80) | Formel (S-81) |
| 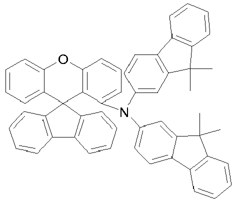 | 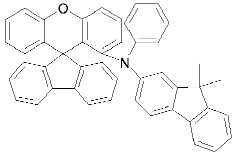 | 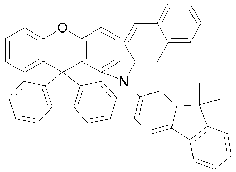 |
| Formel (S-82) | Formel (S-83) | Formel (S-84) |

Die Verbindungen der Formel (S) können mittels üblicher Synthesemethoden der organischen Chemie hergestellt werden. Insbesondere werden dabei Buchwald- und Suzuki-Reaktionen, nukleophile Additionsreaktionen an Carbonylgruppen sowie Ringschlussreaktionen durch elektrophile aromatische Substitution eingesetzt.

Ein bevorzugtes Verfahren zur Herstellung von Verbindungen der Formel (S) verläuft wie folgt: Zunächst wird eine metallierte Ether- oder Thioether-Verbindung (B in untenstehendem Schema 1) an ein Keton C addiert, gefolgt von einer Ringschlussreaktion. Anschließend wird eine Aminogruppe oder eine Arylgruppe, die eine Aminogruppe enthält, über eine Buchwald- bzw. eine Suzuki-Reaktion eingefügt. Die metallierte Ether- bzw. Thioether-Verbindung ist bevorzugt eine lithiierte Verbindung oder eine entsprechende Grignard-Verbindung.

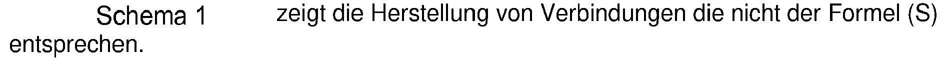

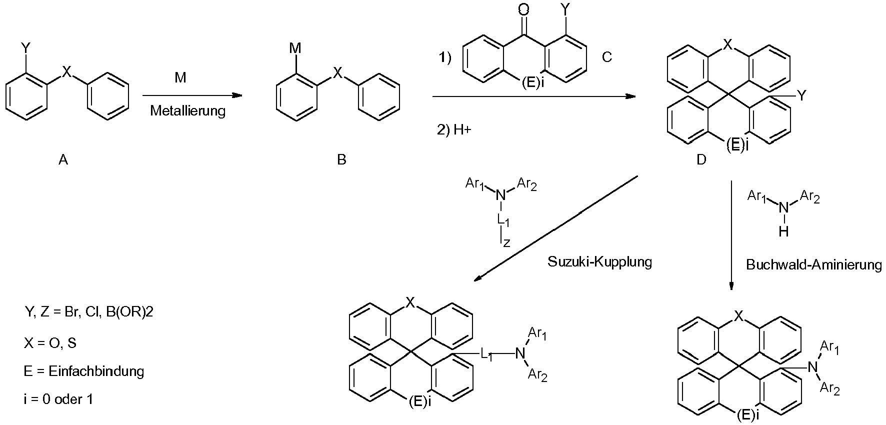

Alternativ können die Addition der metallierten Ether- bzw. Thioether-Gruppe an das Keton und die Ringschluss-Reaktion auch im Anschluss an eine Suzuki- oder Buchwald-Kupplung am Keton stattfinden, wie in Schema 2 gezeigt ist.

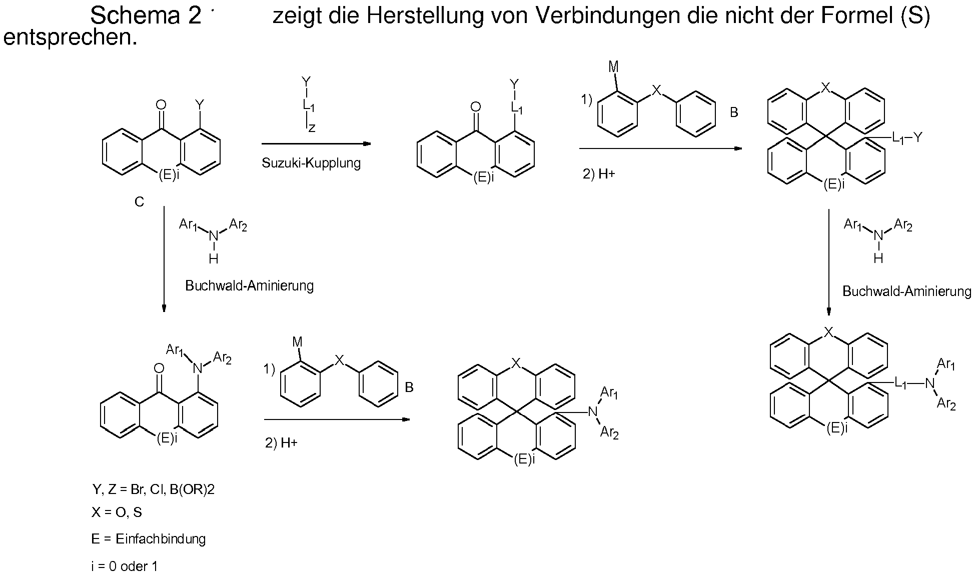

Gegenstand der Anmeldung ist damit ein Verfahren zur Herstellung einer Verbindung der Formel (S), dadurch gekennzeichnet, dass es eine Addition einer metallierten Ether- oder Thioether-Verbindung an ein Diarylketon und eine anschließende Ringschlussreaktion umfasst. Die metallierte Ether- oder Thioether-Verbindung ist bevorzugt eine metallierte Diarylether- oder Diarylthioether-Verbindung, ganz besonders bevorzugt eine lithiierte Diarylether- oder Diarylthioether-Verbindung oder ein entsprechendes Grignard-Derivat der Diarylether- oder Diarylthioether-Verbindung.

Die oben beschriebenen Verbindungen, insbesondere Verbindungen, welche mit reaktiven Abgangsgruppen, wie Brom, lod, Chlor, Boronsäure oder Boronsäureester, substituiert sind, können als Monomere zur Erzeugung entsprechender Oligomere, Dendrimere oder Polymere Verwendung finden. Geeignete reaktive Abgangsgruppen sind beispielsweise Brom, lod, Chlor, Boronsäuren, Boronsäureester, Amine, Alkenyl- oder Alkinylgruppen mit endständiger C-C-Doppelbindung bzw. C-C-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Die vorliegende Anmeldung offenbart auch Oligomere, Polymere oder Dendrimere, enthaltend eine oder mehrere Verbindungen gemäß Formel (S), wobei die Bindung(en) zum Polymer, Oligomer oder Dendrimer an beliebigen, in Formel (S) mit R¹ oder R² substituierten Positionen lokalisiert sein können. Je nach Verknüpfung der Verbindung gemäß Formel (S) ist die Verbindung Bestandteil einer Seitenkette des Oligomers oder Polymers oder Bestandteil der Hauptkette. Unter einem Oligomer im Sinne dieser Anmeldung wird eine Verbindung verstanden, welche aus mindestens drei Monomereinheiten aufgebaut ist. Unter einem Polymer im Sinne der Anmeldung wird eine Verbindung verstanden, die aus mindestens zehn Monomereinheiten aufgebaut ist. Die Polymere, Oligomere oder Dendrimere können konjugiert, teilkonjugiert oder nichtkonjugiert sein. Die Oligomere oder Polymere können linear, verzweigt oder dendritisch sein. In den linear verknüpften Strukturen können die Einheiten gemäß Formel (S) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß Formel (S) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein. Für die Wiederholeinheiten gemäß Formel (S) in Oligomeren, Dendrimeren und Polymeren gelten dieselben Bevorzugungen wie oben für Verbindungen gemäß Formel (S) beschrieben.

Zur Herstellung der Oligomere oder Polymere werden die Monomere homopolymerisiert oder mit weiteren Monomeren copolymerisiert. Geeignete und bevorzugte Comonomere sind gewählt aus Fluorenen (z. B. gemäß EP 842208 oder WO 2000/22026), Spirobifluorenen (z. B. gemäß EP 707020, EP 894107 oder WO 2006/061181), Paraphenylenen (z. B. gemäß WO 1992/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689 oder WO 2007/006383), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten, insbesondere solche basierend auf Triarylaminen.

Die Polymere und Oligomere werden in der Regel durch Polymerisation von einer oder mehreren Monomersorten hergestellt, von denen mindestens ein Monomer im Polymer zu Wiederholungseinheiten der Formel (S) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind die Suzuki-Polymerisation, die Yamamoto-Polymerisation; die Stille-Polymerisation; und die Hartwig-Buchwald-Polymerisation.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösemittel.

Die vorliegende Anmeldung offenbart auch eine Formulierung, insbesondere eine Lösung, Dispersion oder Emulsion, enthaltend mindestens eine Verbindung gemäß Formel (S) sowie mindestens ein Lösungsmittel, bevorzugt ein organisches Lösungsmittel. Wie solche Lösungen hergestellt werden können, ist dem Fachmann bekannt und beispielsweise in WO 2002/072714, WO 2003/019694 und der darin zitierten Literatur beschrieben.

Die erfindungsgemäßen Verbindungen eignen sich für den Einsatz in elektronischen Vorrichtungen, insbesondere in organischen Elektrolumineszenzvorrichtungen (OLEDs). Abhängig von der Substitution werden die Verbindungen in unterschiedlichen Funktionen und Schichten eingesetzt. Hierzu gelten dieselben bevorzugten Ausführungsformen wie oben für die Verbindungen der Formel (I) beschrieben. Zusätzlich sind die Verbindungen der Formel (S) auch besonders geeignet, um in einer Elektronenblockierschicht einer OLED eingesetzt zu werden.

### Ausführungsbeispiele

### A) Synthesebeispiele

### Beispiel 1-1:

### Synthese der erfindungsgemäßen Verbindung 1-1 und Varianten

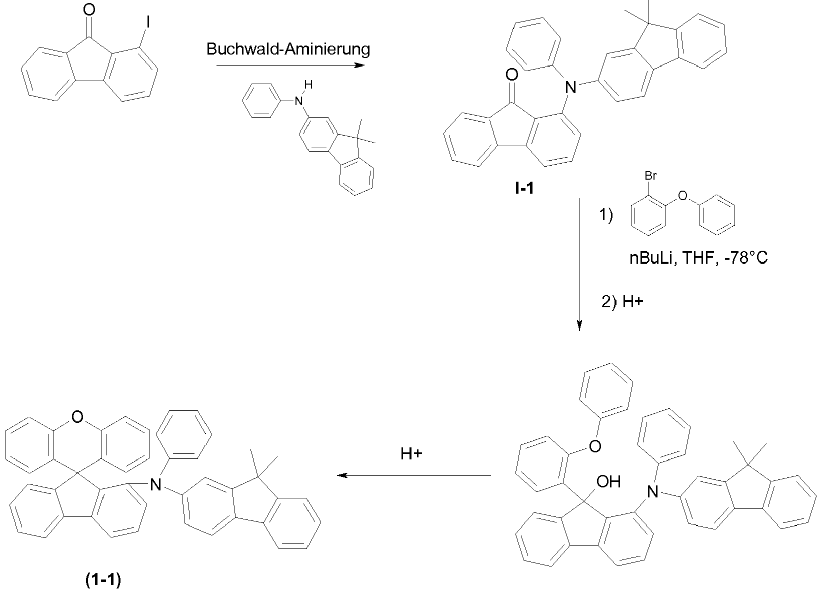

### Zwischenstufe I-1

26,8 Phenyl-(9,9-dimethyl-9H-fluoren-2-yl)-amin (87,6 mmol) und 25 g lod-Benzofluorenon (87,6 mmol) werden in 700 mL Toluol gelöst. Die Lösung wird entgast und mit N₂ gesättigt. Danach wird sie mit 3,5 mL (3,5 mmol) einer Tri-tert-Butylphosphin 1M-Lösung und 0,46 g (1,75 mmol) Palladium(II)acetat versetzt und anschließend werden 16,8 g Natrium-tert-butylat (175 mol) zugegeben. Die Reaktionsmischung wird 5 h unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Nach Filtration des Rohproduktes über Kieselgel mit Toluol wird der verbleibende Rückstand aus Heptan/Toluol umkristallisiert. Die Ausbeute beträgt 33 g (81% d. Th).

Analog dazu werden folgende Verbindungen hergestellt:

| | Edukt 1 | Edukt 2 | Produkt | Ausbe ute |
|---|---|---|---|---|
| **I-2** | 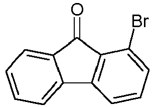 | 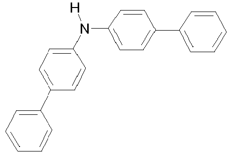 | 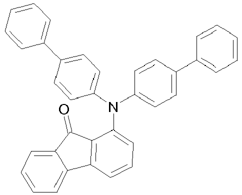 | 85% |
| **I-3** | 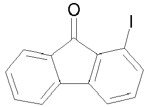 | 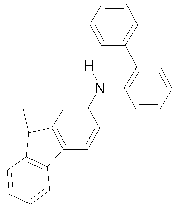 | 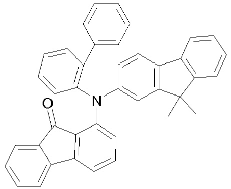 | 71% |
| **I-4** | 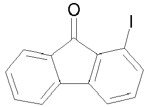 | 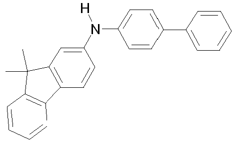 | 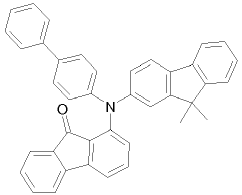 | 82% |
| **I-5** | 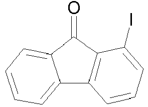 | 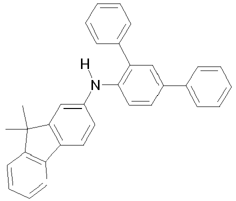 | 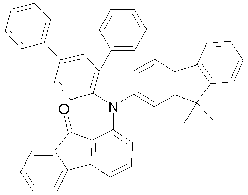 | 72% |
| **I-6** | 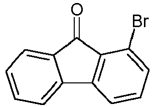 | 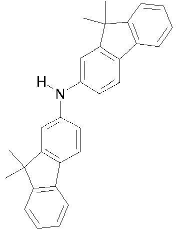 | 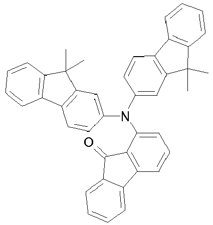 | 74% |
| **I-7** | 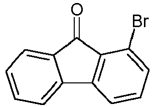 | 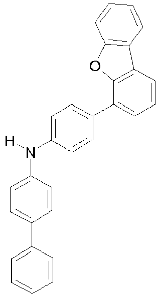 | 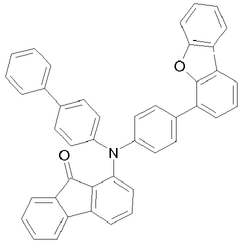 | 74% |
| **I-8** | 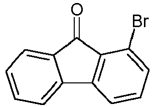 | 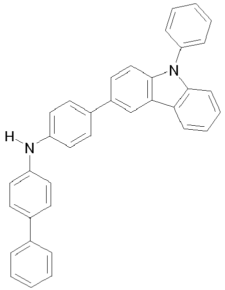 | 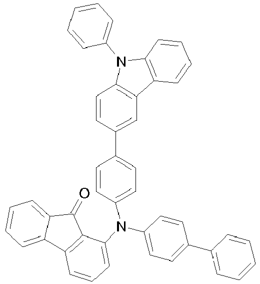 | 62% |
| **I-9** | 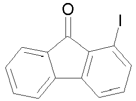 | 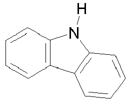 | 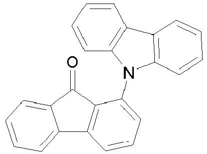 | 35% |
| **I-10** | 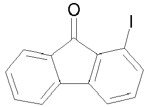 | 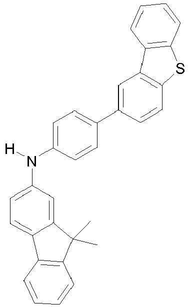 | 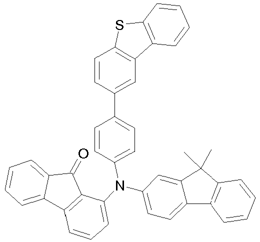 | 70% |
| **I-11** | 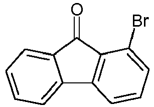 | 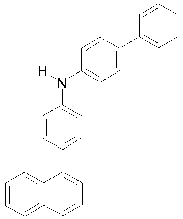 | 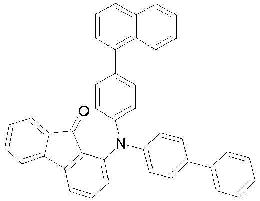 | 67% |

### Verbindung 1-1

17,37 g (69,6 mmol) 1-Bromo-2-diphenylether werden in einem ausgeheizten Kolben in 300 mL getrocknetem THF gelöst. Die Reaktionsmischung wird auf -78°C gekühlt. Bei dieser Temperatur werden 30 mL einer 2,5M-Lösung n-BuLi in Hexan (69,7 mmol) langsam zugetropft. Der Ansatz wird 1 Stunde bei -70°C nachgerührt. Anschließend werden 30 g des Bromfluorenon Derivates (63 mmol) in 200 ml THF gelöst und bei -70°C zugetropft. Nach beendeter Zugabe wird die Reaktionsmischung langsam auf Raumtemperatur erwärmt, mit NH₄Cl gequencht und anschließend am Rotationsverdampfer eingeengt.

Die einrotierte Lösung wird vorsichtig mit 300 ml Essigsäure versetzt und anschließend werden 20 ml rauchende HCl zugegeben. Der Ansatz wird auf 75°C erhitzt und 6 Stunden dort gehalten. Dabei fällt ein weißer Feststoff aus. Der Ansatz wird nun auf Raumtemperatur abgekühlt und der ausgefallene Feststoff wird abgesaugt und mit Wasser und Methanol nachgewaschen. Ausbeute: 35 g (88%)

Der Feststoff wird aus Heptan/Toluol umkristallisiert und abschließend im Hochvakuum sublimiert.

Analog dazu werden folgende Verbindungen hergestellt:

| | Edukt 1 | Edukt 2 | Produkt | Ausb eute |
|---|---|---|---|---|
| **1-2** | 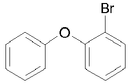 | 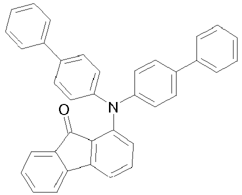 | 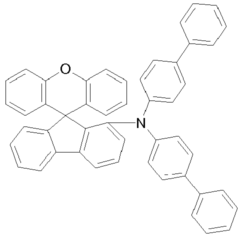 | 70% |
| **1-3** | 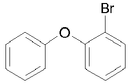 | 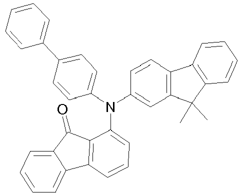 | 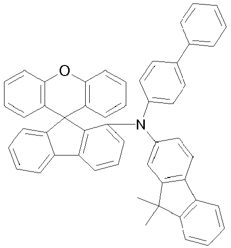 | 77% |
| **1-5** | 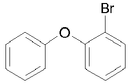 | 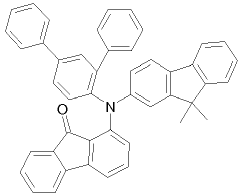 | 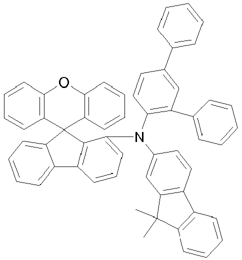 | 65% |
| **1-6** | 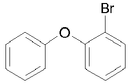 | 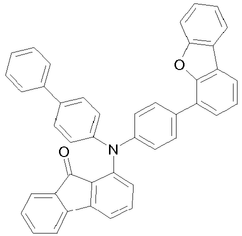 | 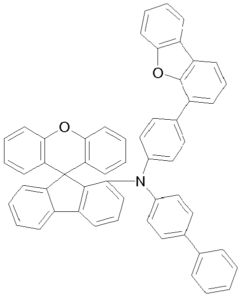 | 69% |
| **1-7** | 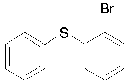 | 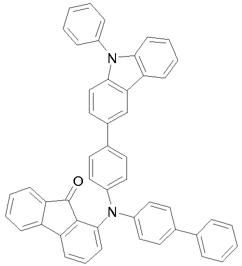 | 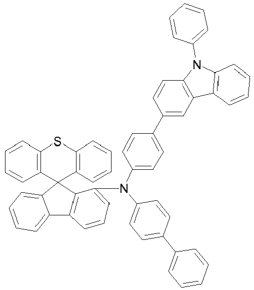 | 79% |
| **1-8** | 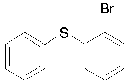 | 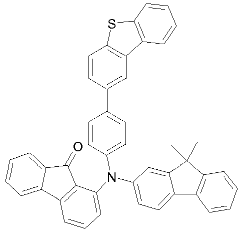 | 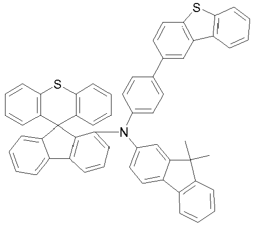 | 81% |
| **1-9** | 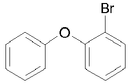 | 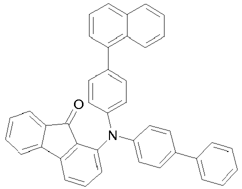 | 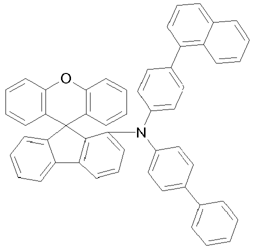 | 80% |
| **1-10** | 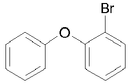 | 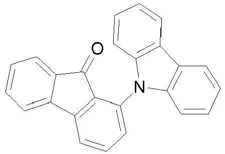 | 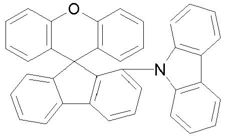 | 40% |
| **1-11** | 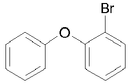 | 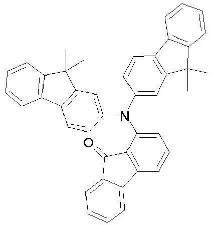 | 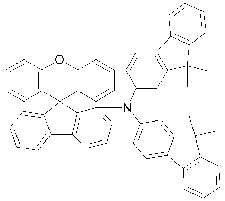 | 79% |

### Beispiel 2-1:

### Synthese der erfindungsgemäßen Verbindung 2-1 und Varianten

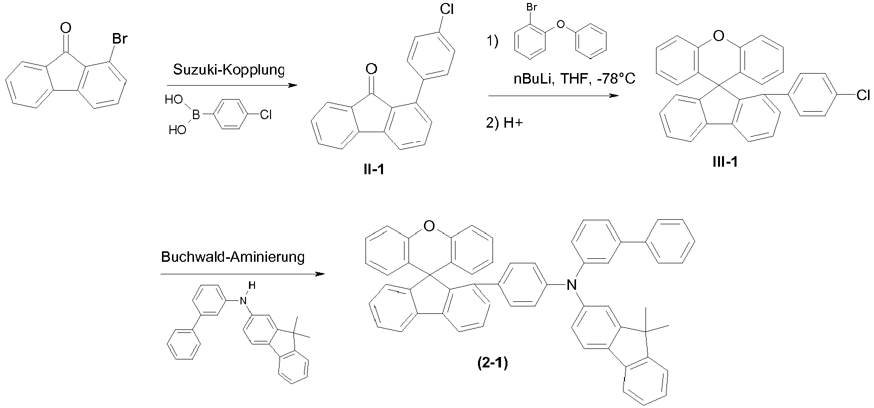

### Zwischenstufe II-1

38 g 4-Chlorophenylboronsäure (243 mmol) und 60 g 1-Brom-fluoren-9-on (232 mmol) werden in 800 mL THF suspendiert. 230 ml von 2 M Kaliumcarbonat Lösung werden langsam zugetropft. Die Lösung wird entgast und mit N₂ gesättigt. Danach wird sie mit 8 g (7 mmol) Pd(Ph3P)4 versetzt. Die Reaktionsmischung wird 16 h unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Nach Filtration des Rohproduktes über Kieselgel mit Toluol wird der verbleibende Rückstand aus MeOH umkristallisiert. Die Ausbeute beträgt 63 g (90% d. Th).

Analog dazu werden folgende Verbindungen hergestellt:

| | Edukt 1 | Edukt 2 | Produkt | Ausbeute |
|---|---|---|---|---|
| **II-2** | 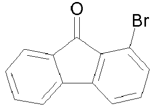 | 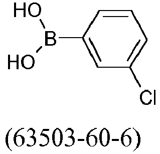 | 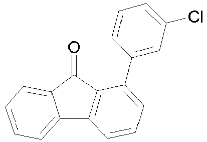 | 80% |
| **II-3** | 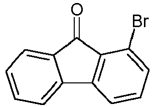 | 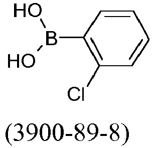 | 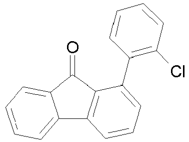 | 88% |
| **II-4** | 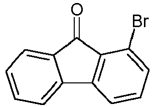 | 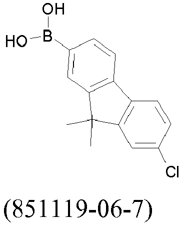 | 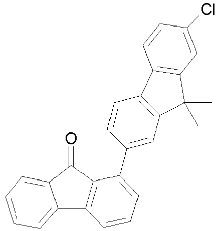 | 82% |
| **II-5** | 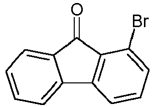 | 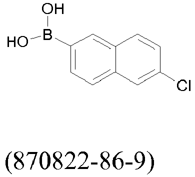 | 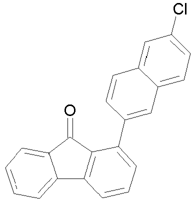 | 89% |
| **II-6** | 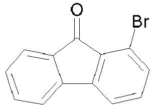 | 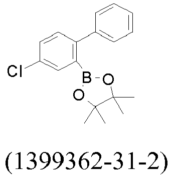 | 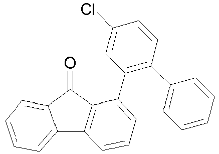 | 64% |
| **II-7** | 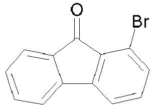 | 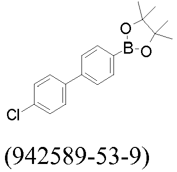 | 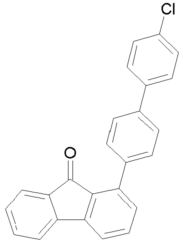 | 80% |
| **II-8** | 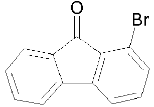 | 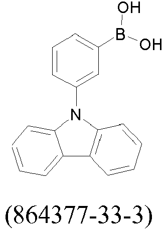 | 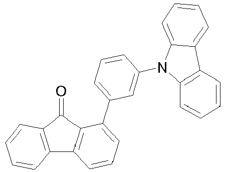 | 83% |

### Zwischenstufe III-1

30 g (120 mmol) 1-Bromo-2-diphenylether werden in einem ausgeheizten Kolben in 500 mL getrocknetem THF gelöst. Die Reaktionsmischung wird auf -78°C gekühlt. Bei dieser Temperatur werden 480 mL einer 2,5M-Lösung n-BuLi in Hexan (120 mmol) langsam zugetropft. Der Ansatz wird 1 Stunde bei -70°C nachgerührt. Anschließend werden 33 g 1-(4-Chlorphenyl)-fluorenon (114 mmol) in 100 ml THF gelöst und bei -70°C zugetropft. Nach beendeter Zugabe wird die Reaktionsmischung langsam auf Raumtemperatur erwärmt, mit NH₄Cl gequencht und anschließend am Rotationsverdampfer eingeengt.

Die einrotierte Lösung wird vorsichtig mit 300 ml Essigsäure versetzt und anschließend werden 20 ml rauchende HCl zugegeben. Der Ansatz wird auf 75°C erhitzt und 6 Stunden dort gehalten. Dabei fällt ein weißer Feststoff aus. Der Ansatz wird nun auf Raumtemperatur abgekühlt und der ausgefallene Feststoff wird abgesaugt und mit Wasser und Methanol nachgewaschen. Ausbeute: 38 g (70%).

Abschließend wird der Rückstand umkristallisiert.

Analog dazu werden folgende Verbindungen hergestellt:

| | Edukt 1 | Edukt 2 | Produkt | Ausb eute |
|---|---|---|---|---|
| **III-2** | 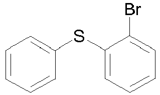 | 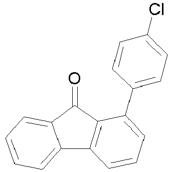 | 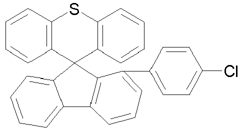 | 70% |
| **III-3** | 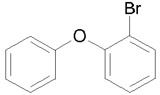 | 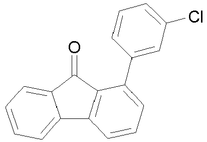 | 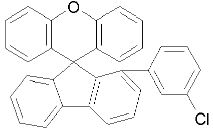 | 77% |
| **III-4** | 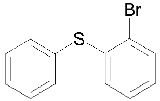 | 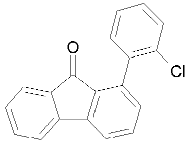 | 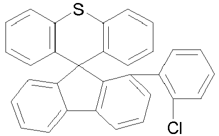 | 67% |
| **III-5** | 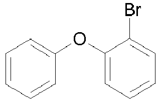 | 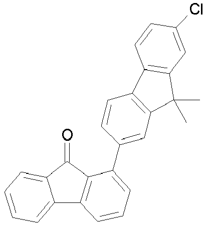 | 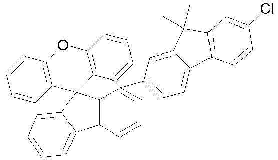 | 65% |
| **III-6** | 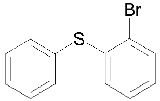 | 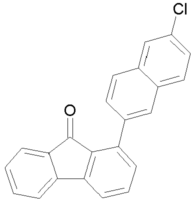 | 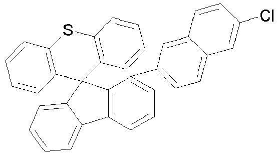 | 73% |
| **III-7** | 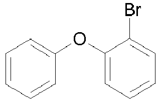 | 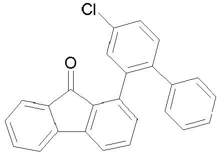 | 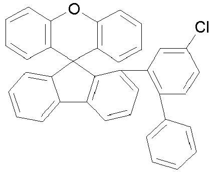 | 69% |
| **III-8** | 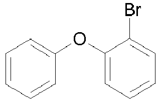 | 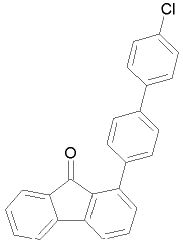 | 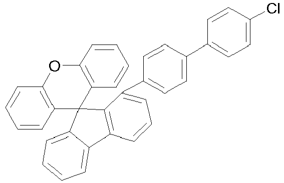 | 83% |
| **III-9** | 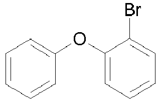 | 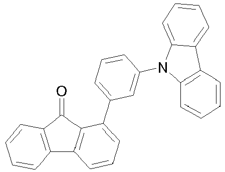 | 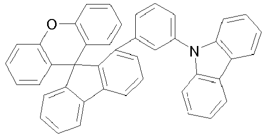 | 71% |

### Verbindung 2-1

16,3 g Biphenyl-3-yl-(9,9-dimethyl-9H-fluoren-2-yl)-amin (45,26 mmol) und 29 g des Chlor-Derivats III-1 (45,2 mmol) werden in 400 mL Toluol gelöst. Die Lösung wird entgast und mit N₂ gesättigt. Danach wird sie mit 740 mg (1,81 mmol) S-Phos und 830 mg (0,9 mmol) Pd2(dba)3 versetzt und anschließend werden 6,5 g Natrium-tert-butylat (67,7mmol) zugegeben. Die Reaktionsmischung wird 5 h unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Nach Filtration des Rohproduktes über Kieselgel mit Toluol wird der verbleibende Rückstand aus Heptan/Toluol umkristallisiert. Die Ausbeute beträgt 27 g (78% d. Th).

Der Feststoff wird aus Heptan/Toluol umkristallisiert und abschließend im Hochvakuum sublimiert.

Analog dazu werden folgende Verbindungen hergestellt:

| | Edukt 1 | Edukt 2 | Produkt | Ausbeute |
|---|---|---|---|---|
| **2-2** | 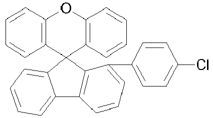 | 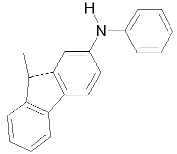 | 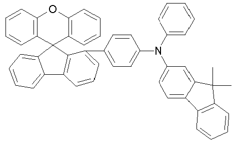 | 78% |
| **2-3** | 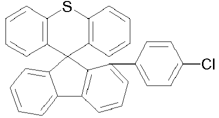 | 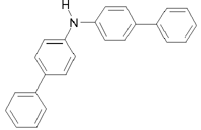 | 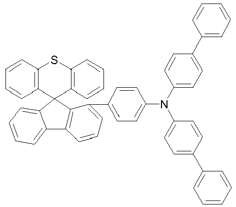 | 71% |
| **2-4** | 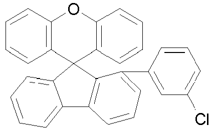 | 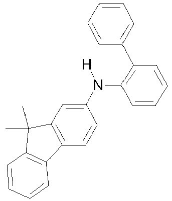 | 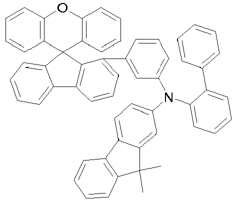 | 82% |
| **2-5** | 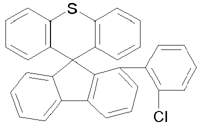 | 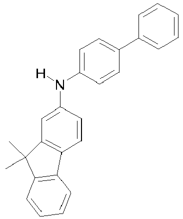 | 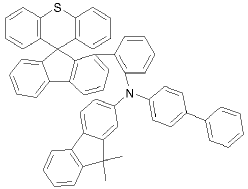 | 89% |
| **2-6** | 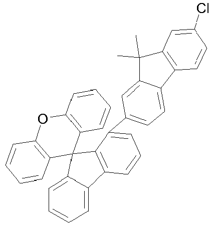 | 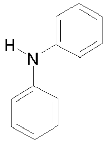 | 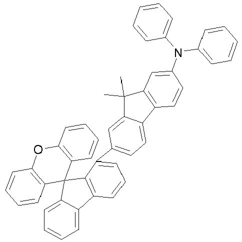 | 69% |
| **2-7** | 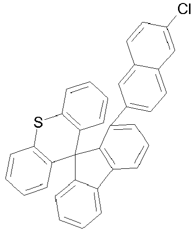 | 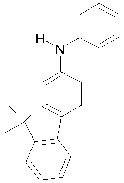 | 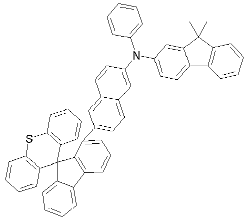 | 88% |
| **2-8** | 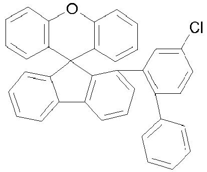 | 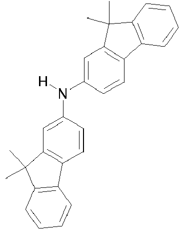 | 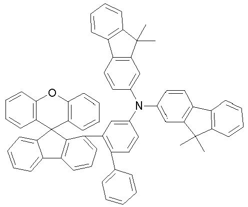 | 85% |
| **2-9** | 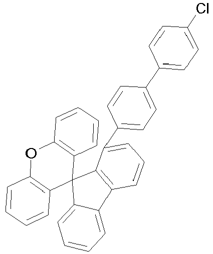 | 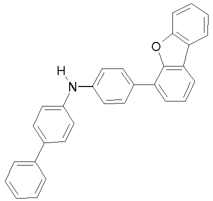 | 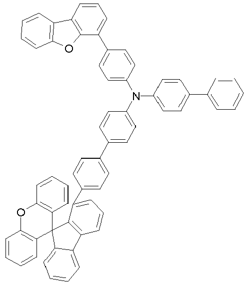 | 75% |
| **2-10** | 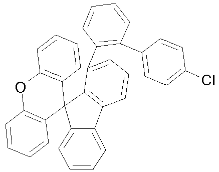 | 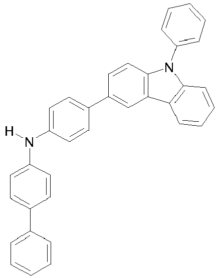 | 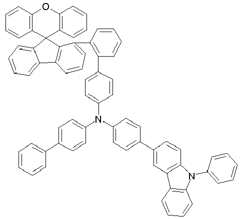 | 75% |

### B) Verwendungsbeispiele

Es werden OLED-Vorrichtungen gemäß der vorliegenden Anmeldung sowie Vergleichsvorrichtungen hergestellt, um die technischen Effekte der erfindungsgemäßen OLED-Devices zu zeigen. Die OLEDs werden gemäß dem allgemeinen Verfahren, das in den Ausführungsbeispielen der Offenlegungsschrift WO 2004/058911 beschrieben ist, hergestellt, sofern unten nichts anderes angegeben ist.

Die hergestellten OLEDs haben als Substrate Glassplatten, welche mit strukturiertem ITO (Indium-Zinn-Oxid) in einer Dicke von 50 nm beschichtet sind. Die Schichten, welche auf das Substrat folgen, ihre Dicke und die Substanzen, aus denen sie bestehen, werden für jede Beispielvorrichtung in einer der folgenden Tabellen separat aufgelistet. Als Gegenelektrode wird als letzte Schicht eine Aluminiumschicht in einer Dicke von 100 nm aufgetragen.

Alle Materialien werden durch thermische Gasphasenabscheidung in einer Vakuumkammer aufgetragen. In den Beispielen besteht die Emissionsschicht immer aus wenigstens einem Matrixmaterial und einer emittierenden Verbindung als Dotand. Letztere wird dem Matrixmaterial bzw. den Matrixmaterialien durch Co-Evaporation zugefügt. Ein Ausdruck SMB:SEB (5%) bedeutet dabei, dass das Material SMB in einem Anteil von 95 Vol.-% in der Schicht vorliegt, und das Material SEB in einem Anteil von 5 Vol.-% in der Schicht vorliegt. Nicht nur die Emissionsschicht, sondern auch andere Schichten können analog aus einer Mischung von zwei oder mehr Materialien bestehen.

Die OLEDs werden nach Standardmethoden charakterisiert. Hierzu werden die Elektrolumineszenzspektren, die externe Quanteneffizienz (EQE, gemessen in %) als Funktion der Leuchtdichte, berechnet aus Strom/Spannung/Leuchdichte-Kennlinien (IUL-Kennlinien) unter Annahme Lambert'scher Emissionscharakteristik, und die Lebensdauer bestimmt. Der Ausdruck EQE @ 40 mA/cm² bedeutet dann zum Beispiel die externe Quanteneffizienz bei einer Betriebsleuchtdichte von 40 mA/cm². Die Lebensdauer wird für grün emittierende Vorrichtungen bei 20 mA/cm², und für blau emittierende Vorrichtungen bei 60 mA/cm² gemessen. Unter Annahme eines exponentiellen Abfalls bei den OLEDs werden die LT80-Werte für die Lebensdauer dann mit einem Beschleunigungsfaktor von 1.8 auf die Lebensdauer bei 1000 cd/m² hin angenähert. LT80 @ 1000 cd/m² ist dann die angenäherte Lebensdauer, bis zu der die OLED von einer initialen Leuchtdichte von 1000 cd/m² auf eine Leuchtdichte von 800 cd/m² abgefallen ist.

Die chemischen Strukturen der Materialien, die in den Beispielen eingesetzt werden, ist in Tabelle A genannt. Die Synthese der Spiroxanthen-Amine erfolgt wie im vorangehenden Synthesebeispiel-Teil, oder sie kann wie im Stand der Technik bekannt, beispielsweise wie in WO 2014/072017 offenbart, erfolgen.

| **Tabelle A** | | |
|---|---|---|
| 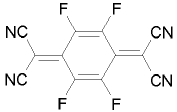 | 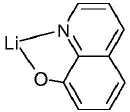 | 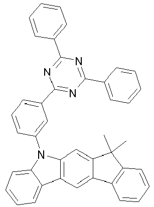 |
| F4TCNQ | LiQ | H1 |
| 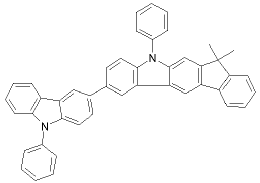 | 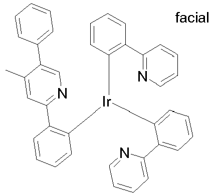 | 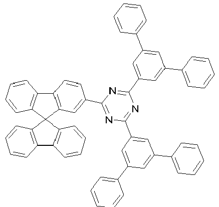 |
| H2 | TEG | ETM |
| 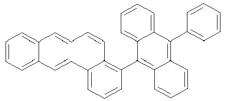 | 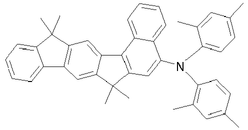 | 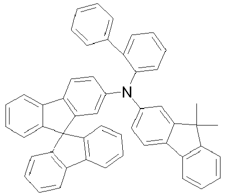 |
| SMB | SEB | HTMV1 = HIM |
| 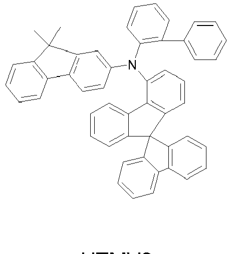 | 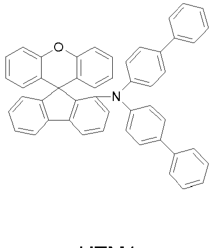 | 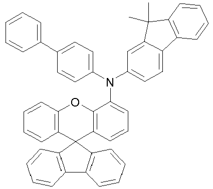 |
| HTMV2 | HTM1 | HTM2 |
| 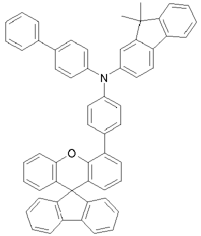 | 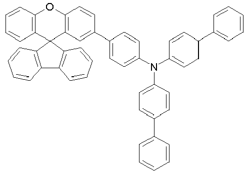 | 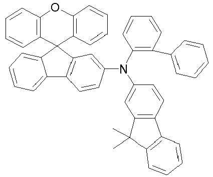 |
| HTM4 | HTM5 | HTM6 |
| 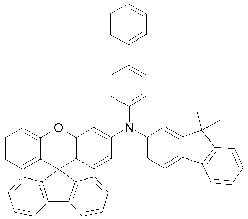 | 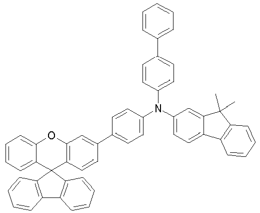 | 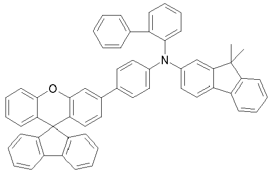 |
| HTM7 | HTM8 | HTM9 |
| 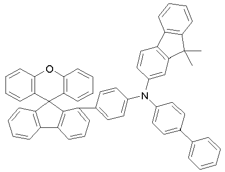 | 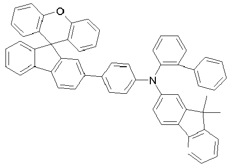 | 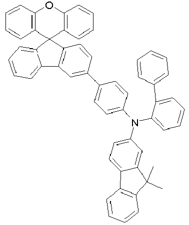 |
| HTM 13 | HTM 14 | HTM 15 |

### 1) Verwendung von Spiroxanthen-Aminen als HTL und HIL-Materialien

Es werden die folgenden OLEDs V3 (Vergleichsbeispiel) und E3, E5, E7, E9, E10, E14, E15, und E16 (erfindungsgemäße Beispiele) hergestellt.

V3 als Vergleichsbeispiel enthält die Verbindung HIM (ein Spirobifluorenderivat) als HTL- und HIL-Material. Die oben genannten Verwendungsbeispiele E3, E5, E7, E9, E10, E14, E15, und E16 enthalten die Materialien HTM2, HTM4, HTM5, HTM6, HTM7, HTM8, HTM9, HTM13, HTM14, und HTM15 als HTL- und HIL-Materialien. Ansonsten ist ihr Aufbau identisch zu dem von V3 (Tabelle 1).

Für alle erfindungsgemäßen Vorrichtungen wird ein starker Anstieg in der Lebensdauer verglichen mit dem Beispiel V3 beobachtet (Tabelle 2).

Dies zeigt die hervorragende Eignung der Spiroxanthen-Amine als HIL- und HTL-Materialien, verglichen mit dem HTL-/HIL-Material HIM gemäß dem Stand der Technik.

| **Tabelle 1: Aufbau der OLEDs** | | | | | | |
|---|---|---|---|---|---|---|
| **Bsp** | **HIL** | **HTL** | **EBL** | **EML** | **ETL** | **EIL** |
| | Dicke / nm | Dicke / nm | Dicke / nm | Dicke / nm | Dicke / nm | Dicke / nm |
| V3 | HIM:F4TCNQ(5%) | HIM | HTMV2 | SMB:SEB(5%) | ETM:LiQ(50%) | LiQ |
| | 20nm | 180nm | 10nm | 20 nm | 30 nm | 1nm |
| E3 | HTM2:F4TCNQ(5%) | HTM2 | HTMV2 | SMB:SEB(5%) | ETM:LiQ(50%) | LiQ |
| | 20nm | 180nm | 10nm | 20 nm | 30 nm | 1nm |
| E5 | HTM4:F4TCNQ(5%) | HTM4 | HTMV2 | SMB:SEB(5%) | ETM:LiQ(50%) | LiQ |
| | 20nm | 180nm | 10nm | 20 nm | 30 nm | 1nm |
| E7 | HTM6:F4TCNQ(5%) | HTM6 | HTMV2 | SMB:SEB(5%) | ETM:LiQ(50%) | LiQ |
| | 20nm | 180nm | 10nm | 20 nm | 30 nm | 1nm |
| E9 | HTM8: F4TCNQ(5%) | HTM8 | HTMV2 | SMB:SEB(5%) | ETM:LiQ(50%) | LiQ |
| | 20nm | 180nm | 10nm | 20 nm | 30 nm | 1nm |
| E10 | HTM9:F4TCNQ(5%) | HTM9 | HTMV2 | SMB:SEB(5%) | ETM:LiQ(50%) | LiQ |
| | 20nm | 180nm | 10nm | 20 nm | 30 nm | 1nm |
| E14 | HTM5:F4TCNQ(5%) | HTM13 | HTMV2 | SMB:SEB(5%) | ETM:LiQ(50%) | LiQ |
| | 20nm | 180nm | 10nm | 20 nm | 30 nm | 1nm |
| E15 | HTM6: F4TCNQ(5%) | HTM14 | HTMV2 | SMB:SEB(5%) | ETM:LiQ(50%) | LiQ |
| | 20nm | 180nm | 10nm | 20 nm | 30 nm | 1nm |
| E16 | HTM7:F4TCNQ(5%) | HTM15 | HTMV2 | SMB:SEB(5%) | ETM:LiQ(50%) | LiQ |
| | 20nm | 180nm | 10nm | 20 nm | 30 nm | 1nm |

| **Tabelle 2: Daten der OLEDs** | | |
|---|---|---|
| **Bsp.** | **U @ 10mA/cm²** | **LT80 @ 1000cd/m²** |
| | [V] | [h] |
| V3 | 3.8 | 4790 |
| E3 | 4.4 | 6800 |
| E5 | 4.3 | 4960 |
| E7 | 3.8 | 5610 |
| E9 | 4.3 | 5180 |
| E10 | 4.2 | 7390 |
| E14 | 3.9 | 5500 |
| E15 | 3.8 | 6600 |
| E16 | 4.0 | 7400 |

Ein Vergleich zwischen OLEDs, die sich lediglich durch die Tatsache, dass die Spiroxanthen-Amine in der EBL anstatt in der HTL / HIL enthalten sind, unterscheiden, ist in den folgenden Tabellen 3 und 4 gezeigt.

Tabelle 3 zeigt dabei den Aufbau der Vergleichs-OLEDs.

Tabelle 4 zeigt dabei die Ergebnisse der Direktvergleiche einander gegenübergestellt. In einer Zeile sind jeweils die miteinander zu vergleichenden Daten aufgelistet. In allen Fällen werden für den Fall, dass die Spiro-Xanthene in der HIL/HTL enthalten sind, signifikant höhere Lebensdauern erhalten (Beispiele auf der rechten Seite der Tabelle 4).

Dies zeigt die Vorteile, die durch die Verwendung der Spiroxanthen-Aminverbindungen in der HIL und der HTL von OLEDs erhalten werden.

| **Tabelle 3: Aufbau der OLEDs** | | | | | | |
|---|---|---|---|---|---|---|
| **Bsp** | **HIL** | **HTL** | **EBL** | **EML** | **ETL** | **EIL** |
| | Dicke / nm | Dicke / nm | Dicke / nm | Dicke / nm | Dicke / nm | Dicke / nm |
| E17 | HIM:F4TCNQ(5%) | HIM | HTM2 | SMB:SEB(5%) | ETM:LiQ(50%) | LiQ |
| | 20nm | 180nm | 10nm | 20 nm | 30 nm | 1nm |
| E19 | HIM:F4TCNQ(5%) | HIM | HTM4 | SMB:SEB(5%) | ETM:LiQ(50%) | LiQ |
| | 20nm | 180nm | 10nm | 20 nm | 30 nm | 1nm |
| E21 | HIM:F4TCNQ(5%) | HIM | HTM6 | SMB:SEB(5%) | ETM:LiQ(50%) | LiQ |
| | 20nm | 180nm | 20nm | 20 nm | 30 nm | 1nm |
| E23 | HIM:F4TCNQ(5%) | HIM | HTM8 | SMB:SEB(5%) | ETM:LiQ(50%) | LiQ |
| | 20nm | 180nm | 10nm | 20 nm | 30 nm | 1nm |
| E24 | HIM:F4TCNQ(5%) | HIM | HTM9 | SMB:SEB(5%) | ETM:LiQ(50%) | LiQ |
| | 20nm | 180nm | 10nm | 20 nm | 30 nm | 1nm |

| **Tabelle 4: Daten der OLEDs** | | | | | |
|---|---|---|---|---|---|
| **Bsp.** | **U @ 10mA/cm²** | **LT80 @ 1000cd/m²** | **LT80 @ 1000cd/m²** | **U @ 10mA/cm²** | **Bsp.** |
| | [V] | [h] | [h] | [V] | |
| E17 | 3.9 | 3082 | 6800 | 4.4 | E3 |
| E19 | 3.8 | 2278 | 4960 | 4.3 | E5 |
| E21 | 3.9 | 2464 | 5610 | 3.8 | E7 |
| E23 | 3.7 | 3881 | 5180 | 4.3 | E9 |
| E24 | 3.7 | 4126 | 7390 | 4.2 | E10 |

### 2) Verwendung von Spiroxanthenen, die in der 1-Position mit einer Aminogruppe substituiert sind, als EBL-Materialien

Die folgenden OLEDs V1, V2, E1 und E2 werden hergestellt (Aufbau s. Tabelle 5).

V1 und V2 sind Vergleichsbeispiele, die ein 4-Spirobifluoren-Amin (HTMV2) als EBL-Material verwenden. V1 unterscheidet sich von V2 dadurch, dass ein anderes Spirobifluoren-Amin als HIL- und HTL-Material verwendet wird (HTMV1 in V1, und HTMV2 in V2).

E1 ist ein Direktvergleich zu V1. In E1 wird das Spiroxanthen-Amin HTM1 als EBL-Material anstelle des Spirobifluoren-Amins HTMV2 verwendet. E2 ist ein Direktvergleich zu V2. In V2 wird das Spiroxanthen-Amin HTM1 als EBL-Material anstelle des Spirobifluoren-Amins HTMV2 verwendet.

Sowohl für E1 als auch für E2 wird ein starker relativer Anstieg der Lebensdauer (LT80) beobachtet, verglichen mit den Beispielen V1 und V2. Parallel dazu verbessert sich die Effizienz der OLEDs (Tabelle 6).

Dies zeigt den technischen Effekt, der mit 1-Spiroxanthen-Aminen erzielt wird, insbesondere bei der Verwendung als EBL-Materialien.

| **Tabelle 5: Aufbau der OLEDs** | | | | | | | |
|---|---|---|---|---|---|---|---|
| **Bsp.** | **HIL** | **HTL** | **EBL** | **EML** | **HBL** | **ETL** | **EIL** |
| | Dicke / nm | Dicke / nm | Dicke / nm | Dicke / nm | Dicke / nm | Dicke / nm | Dicke / nm |
| V1 | HTMV1: | HTMV1 | HTMV2 | H1:H2(29%): | ETM | ETM:LiQ(50%) | LiQ |
| | F4TCNQ(5%) 20nm | 220nm | 10nm | TEG(12%) 30 nm | 10 nm | 30 nm | 1nm |
| E1 | HTMV1: | HTMV1 | HTM1 | H1:H2(29%): | ETM | ETM:LiQ(50%) | LiQ |
| | F4TCNQ(5%) 20nm | 220nm | 10nm | TEG(12%) 30 nm | 10 nm | 30 nm | 1nm |
| V2 | HTMV2: | HTMV2 | HTMV2 | H1:H2(29%): | ETM | ETM:LiQ(50%) | LiQ |
| | F4TCNQ(5%) 20nm | 220nm | 10nm | TEG(12%) 30 nm | 10 nm | 30 nm | 1nm |
| E2 | HTMV2: | HTMV2 | HTM1 | H1:H2(29%): | ETM | ETM:LiQ(50%) | LiQ |
| | F4TCNQ(5%) 20nm | 220nm | 10nm | TEG(12%) 30 nm | 10 nm | 30 nm | 1nm |

| **Tabelle 6: Daten der OLEDs** | | | |
|---|---|---|---|
| **Ex.** | **u @ 2mA/cm²** | **EQE @ 2mA/cm²** | **LT80 @ 1000 cd/m²** |
| | [V] | % | [h] |
| V1 | 3.1 | 17.4 | 53400 |
| E1 | 3.3 | 18.0 | 69900 |
| V2 | 3.2 | 17.7 | 69000 |
| E2 | 3.5 | 17.9 | 76400 |

## Patentansprüche

1. Elektronische Vorrichtung, umfassend, in dieser Reihenfolge, eine Anode, eine lochtransportierende Schicht, eine emittierende Schicht, und eine Kathode, wobei die lochtransportierende Schicht eine Verbindung einer Formel (I) enthält 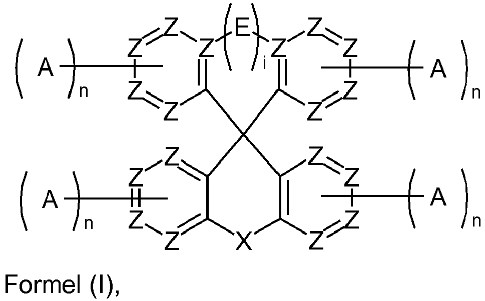 wobei gilt:
A ist eine Arylaminogruppe, wahlweise substituiert mit einem oder mehreren Resten R¹, oder eine Carbazol enthaltende Gruppe, wahlweise substituiert mit einem oder mehreren Resten R¹;
E ist eine Einfachbindung;
X ist O oder S;
Z ist bei jedem Auftreten gleich oder verschieden CR² oder N oder C, wobei eine Gruppe Z genau dann gleich C ist, wenn eine Gruppe A oder E an die betreffende Gruppe Z gebunden ist;
R¹ ist bei jedem Auftreten gleich oder verschieden gewählt aus H, D, F, C(=O)R³, CN, Si(R³)₃, N(R³)₂, P(=O)(R³)₂, OR³, S(=O)R³, S(=O)₂R³, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R¹ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R³ substituiert sein können; und wobei eine oder mehrere CH₂-Gruppen in den genannten Alkyl-, Alkoxy-, Alkenyl- und Alkinylgruppen durch -R³C=CR³-, -C=C-, Si(R³)₂, C=O, C=NR³, -C(=O)O-, -C(=O)NR³-, NR³, P(=O)(R³), -O-, -S-, SO oder SO₂ ersetzt sein können;
R² ist bei jedem Auftreten gleich oder verschieden gewählt aus H, D, F, C(=O)R³, CN, Si(R³)₃, N(R³)₂, P(=O)(R³)₂, OR³, S(=O)R³, S(=O)₂R³, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R² miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R³ substituiert sein können; und wobei eine oder mehrere CH₂-Gruppen in den genannten Alkyl-, Alkoxy-, Alkenyl- und Alkinylgruppen durch -R³C=CR³-, -C=C-, Si(R³)₂, C=O, C=NR³, -C(=O)O-, -C(=O)NR³-, NR³, P(=O)(R³), -O-, -S-, SO oder SO₂ ersetzt sein können;
R³ ist bei jedem Auftreten gleich oder verschieden gewählt aus H, D, F, C(=O)R⁴, CN, Si(R⁴)₃, N(R⁴)₂, P(=O)(R⁴)₂, OR⁴, S(=O)R⁴, S(=O)₂R⁴, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R³ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁴ substituiert sein können; und wobei eine oder mehrere CH₂-Gruppen in den genannten Alkyl-, Alkoxy-, Alkenyl- und Alkinylgruppen durch -R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können;
R⁴ ist bei jedem Auftreten gleich oder verschieden gewählt aus H, D, F, CN, Alkyl- oder Alkoxygruppen mit 1 bis 20 C-Atomen, Alkenyl- oder Alkinylgruppen mit 2 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R⁴ miteinander verknüpft sein können und einen Ring bilden können; und wobei die genannten Alkyl-, Alkoxy-, Alkenyl- und Alkinylgruppen, aromatischen Ringsysteme und heteroaromatischen Ringsysteme mit F oder CN substituiert sein können;
i ist gleich 1;
n ist bei jedem Auftreten gleich oder verschieden 0 oder 1, wobei die Summe aller Indices n gleich 1, 2, 3 oder 4 ist, und wobei zwischen der lochtransportierenden Schicht und der emittierenden Schicht mindestens zwei weitere Schichten angeordnet sind, und wobei keine weiteren emittierenden Schichten zwischen der emittierenden Schicht und der Anode angeordnet sind.

2. Elektronische Vorrichtung nach Anspruch 1, **dadurch gekennzeichnet, dass** X gleich O ist.

3. Elektronische Vorrichtung nach Anspruch 1 oder 2, **dadurch gekennzeichnet, dass** die Summe der Indices n gleich 1 ist.

4. Elektronische Vorrichtung nach einem oder mehreren der Ansprüche 1 bis 3, **dadurch gekennzeichnet, dass** R¹ bei jedem Auftreten gleich oder verschieden gewählt ist aus H, F, CN, geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkylgruppen, die genannten aromatischen Ringsysteme und die genannten heteroaromatischen Ringsysteme jeweils mit einem oder mehreren Resten R³ substituiert sein können.

5. Elektronische Vorrichtung nach einem oder mehreren der Ansprüche 1 bis 4, **dadurch gekennzeichnet, dass** R² bei jedem Auftreten gleich oder verschieden gewählt ist aus H, F, CN, geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkylgruppen, die genannten aromatischen Ringsysteme und die genannten heteroaromatischen Ringsysteme jeweils mit einem oder mehreren Resten R³ substituiert sein können.

6. Elektronische Vorrichtung nach einem oder mehreren der Ansprüche 1 bis 5, **dadurch gekennzeichnet, dass** R³ bei jedem Auftreten gleich oder verschieden gewählt ist aus H, F, CN, geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkylgruppen, die genannten aromatischen Ringsysteme und die genannten heteroaromatischen Ringsysteme jeweils mit einem oder mehreren Resten R⁴ substituiert sein können.

7. Elektronische Vorrichtung nach einem oder mehreren der Ansprüche 1 bis 6, **dadurch gekennzeichnet, dass** die Gruppe A eine Arylaminogruppe ist, die mit einem oder mehreren Resten R¹ substituiert sein kann.

8. Elektronische Vorrichtung nach einem oder mehreren der Ansprüche 1 bis 7, **dadurch gekennzeichnet, dass** die Arylaminogruppe als Gruppe A einer Formel (A) entspricht 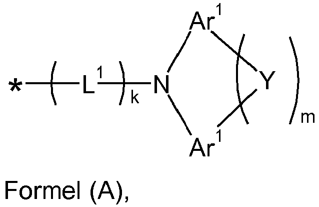 wobei gilt:
L¹ ist bei jedem Auftreten gleich oder verschieden C=O, Si(R¹)₂, PR¹, P(=O)(R¹), O, S, SO, SO₂, eine Alkylengruppe mit 1 bis 20 C-Atomen oder eine Alkenylen- oder Alkinylengruppe mit 2 bis 20 C-Atomen, wobei in den genannten Gruppen eine oder mehrere CH₂-Gruppen durch C=O, C=NR¹, C=O-O, C=O-NR¹, Si(R¹)₂, NR¹, P(=O)(R¹), O, S, SO oder SO₂ ersetzt sein können und wobei ein oder mehrere H-Atome in den oben genannten Gruppen durch D, F oder CN ersetzt sein können, oder ein aromatisches oder heteroaromatisches Ringsystem mit 6 bis 24 aromatischen Ringatomen, welches durch einen oder mehrere Reste R¹ substituiert sein kann;
Ar¹ ist bei jedem Auftreten gleich oder verschieden ein aromatisches oder heteroaromatisches Ringsystem mit 6 bis 30 aromatischen Ringatomen, das mit einem oder mehreren Resten R¹ substituiert sein kann;
Y ist gewählt aus einer Einfachbindung, BR¹, C(R¹)₂, C(R¹)₂-C(R¹)₂, Si(R¹)₂, Si(R¹)₂-Si(R¹)₂, C=O, C=NR¹, C=C(R¹)₂, C(=O)N(R¹), O, S, S=O, SO₂ und NR¹;
k ist gleich 0, 1, 2 oder 3;
m ist gleich 0 oder 1;
wobei die Gruppe A über die mit * markierte Bindung an den Rest der Verbindung der Formel (I) gebunden ist.

9. Elektronische Vorrichtung nach einem oder mehreren der Ansprüche 1 bis 8, **dadurch gekennzeichnet, dass** die Verbindung der Formel (I) einer der folgenden Formeln entspricht 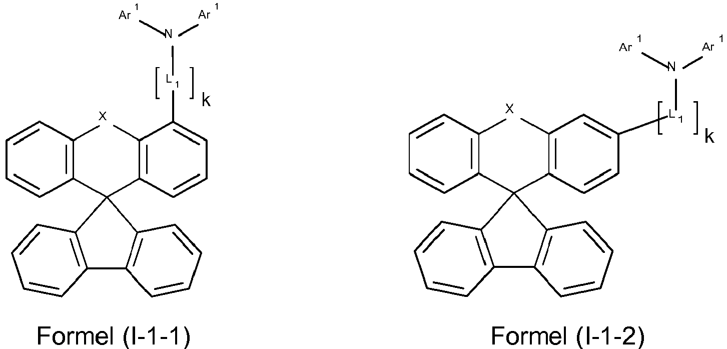 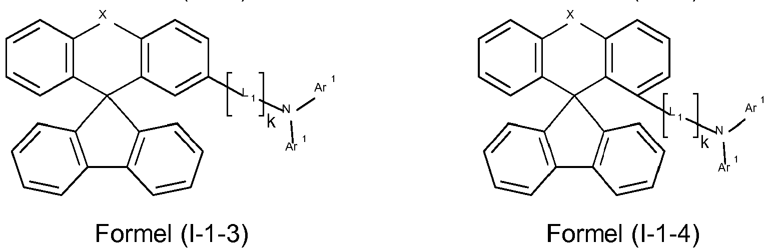 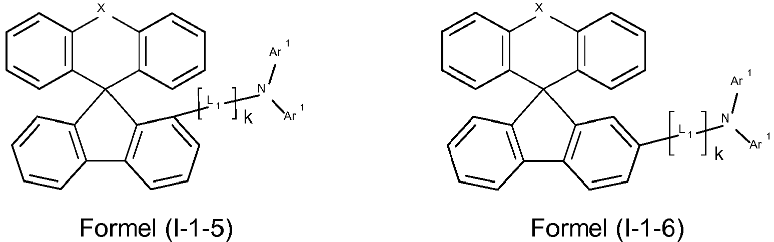 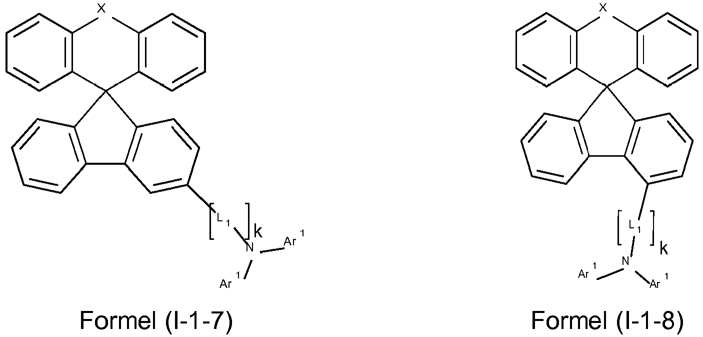 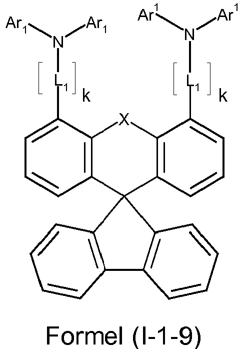 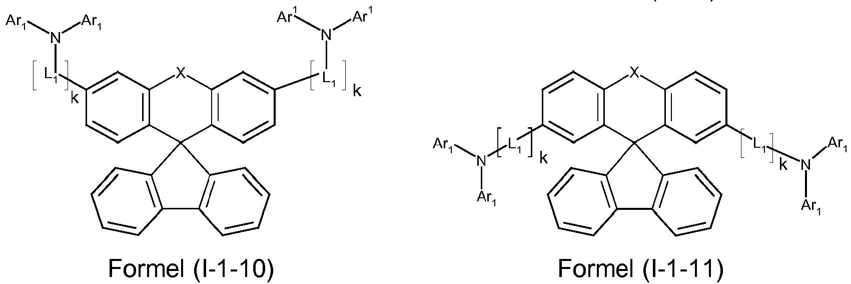 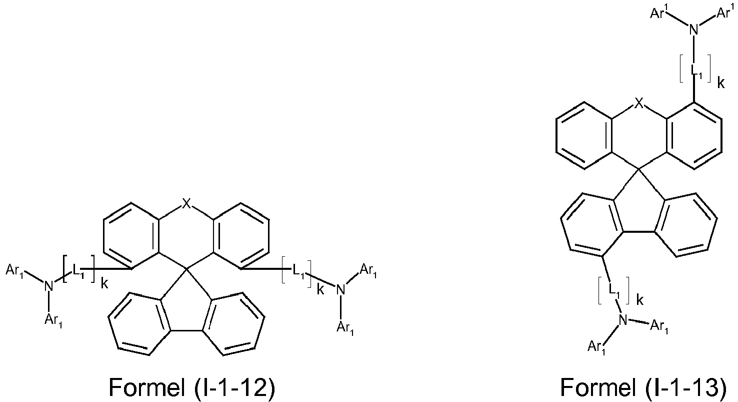 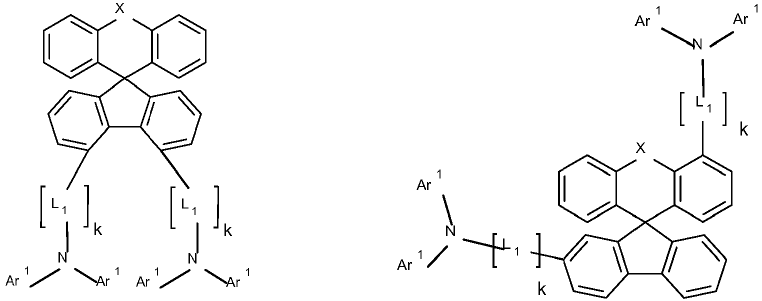 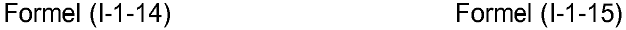 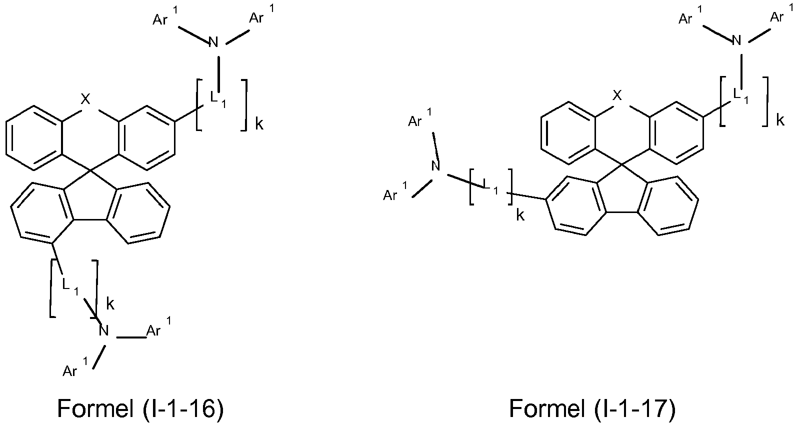 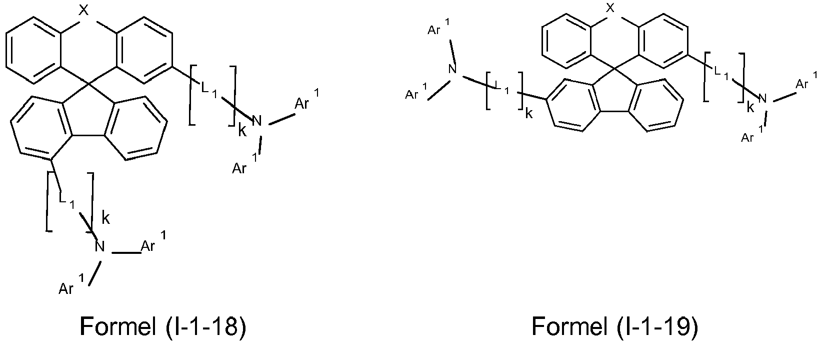 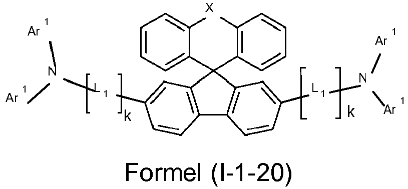 wobei X, L¹, Ar¹ und k definiert sind wie in einem oder mehreren der Ansprüche 1 bis 8, und wobei die Verbindungen an den unsubstituiert gezeichneten Positionen an den Benzolringen jeweils mit Resten R², definiert wie in Anspruch 1 oder 5, substituiert sein können.

10. Elektronische Vorrichtung nach einem oder mehreren der Ansprüche 1 bis 9, **dadurch gekennzeichnet, dass** zwischen der Schicht enthaltend die Verbindung der Formel (I) und der anodennächsten emittierenden Schicht mindestens eine weitere Schicht vorhanden ist, welche keine Verbindung der Formel (I) aufweist.

11. Elektronische Vorrichtung nach Anspruch 10, **dadurch gekennzeichnet, dass** die mindestens eine weitere Schicht eine lochtransportierende Schicht ist, die eine Monoamin-Verbindung enthält, die mindestens eine Gruppe gewählt aus Spirobifluorenylgruppen, Phenanthrenylgruppen, Fluorenylgruppen, Carbazolylgruppen, Dibenzofuranylgruppen und Dibenzothiophenylgruppen enthält.

12. Elektronische Vorrichtung nach einem oder mehreren der Ansprüche 1 bis 11, **dadurch gekennzeichnet, dass** die lochtransportierende Schicht direkt an die Anode angrenzt, dass zwischen der lochtransportierenden Schicht und der emittierenden Schicht mindestens zwei weitere Schichten angeordnet sind, und dass keine weiteren emittierenden Schichten zwischen der emittierenden Schicht und der Anode angeordnet sind.

13. Verbindung einer Formel (S) 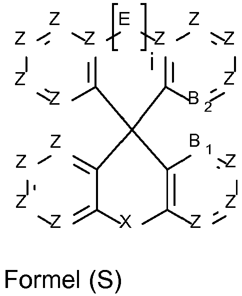 wobei an B₁ eine Gruppe A gebunden sein muss, und wobei für die auftretenden Variablen gilt:
B₂ ist N oder CR² oder C, und B₁ ist C;
Z ist bei jedem Auftreten gleich oder verschieden CR² oder N oder C, wobei eine Gruppe Z genau dann gleich C ist, wenn eine Gruppe E an die betreffende Gruppe Z gebunden ist;
A ist eine Arylaminogruppe, wahlweise substituiert mit einem oder mehreren Resten R¹, oder eine Carbazol enthaltende Gruppe, wahlweise substituiert mit einem oder mehreren Resten R¹;
E ist eine Einfachbindung;
X ist O oder S;
R¹ ist bei jedem Auftreten gleich oder verschieden gewählt aus H, D, F, C(=O)R³, CN, Si(R³)₃, N(R³)₂, P(=O)(R³)₂, OR³, S(=O)R³, S(=O)₂R³, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R¹ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R³ substituiert sein können; und wobei eine oder mehrere CH₂-Gruppen in den genannten Alkyl-, Alkoxy-, Alkenyl- und Alkinylgruppen durch -R³C=CR³-, -C=C-, Si(R³)₂, C=O, C=NR³, -C(=O)O-, -C(=O)NR³-, NR³, P(=O)(R³), -O-, -S-, SO oder SO₂ ersetzt sein können;
R² ist bei jedem Auftreten gleich oder verschieden gewählt aus H, D, F, C(=O)R³, CN, Si(R³)₃, P(=O)(R³)₂, OR³, S(=O)R³, S(=O)₂R³, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R² miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R³ substituiert sein können; und wobei eine oder mehrere CH₂-Gruppen in den genannten Alkyl-, Alkoxy-, Alkenyl- und Alkinylgruppen durch -R³C=CR³-, -C=C-, Si(R³)₂, C=O, -C(=O)O-, -C(=O)NR³-, , P(=O)(R³), -O-, -S-, SO oder SO₂ ersetzt sein können;
R³ ist bei jedem Auftreten gleich oder verschieden gewählt aus H, D, F, C(=O)R⁴, CN, Si(R⁴)₃, N(R⁴)₂, P(=O)(R⁴)₂, OR⁴, S(=O)R⁴, S(=O)₂R⁴, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R³ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁴ substituiert sein können; und wobei eine oder mehrere CH₂-Gruppen in den genannten Alkyl-, Alkoxy-, Alkenyl- und Alkinylgruppen durch -R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können;
R⁴ ist bei jedem Auftreten gleich oder verschieden gewählt aus H, D, F, CN, Alkyl- oder Alkoxygruppen mit 1 bis 20 C-Atomen, Alkenyl- oder Alkinylgruppen mit 2 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R⁴ miteinander verknüpft sein können und einen Ring bilden können; und wobei die genannten Alkyl-, Alkoxy-, Alkenyl- und Alkinylgruppen, aromatischen Ringsysteme und heteroaromatischen Ringsysteme mit F oder CN substituiert sein können;
i ist gleich 0 oder 1.

14. Verbindung nach Anspruch 13, **dadurch gekennzeichnet, dass** X gleich O ist.

15. Verbindung nach Anspruch 13 oder 14, **dadurch gekennzeichnet, dass** i gleich 1 ist.

16. Verfahren zur Herstellung einer Verbindung nach einem oder mehreren der Ansprüche 13 bis 15, **dadurch gekennzeichnet, dass** es eine Addition einer metallierten Ether- oder Thioether-Verbindung an ein Diarylketon und eine anschließende Ringschlussreaktion umfasst.

## Claims

1. Electronic device comprising, in this sequence, an anode, a hole-transporting layer, an emitting layer and a cathode, where the hole-transporting layer comprises a compound of a formula (I) 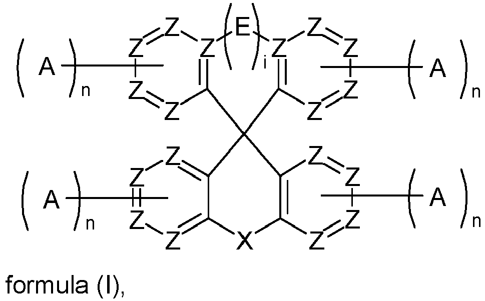 where the following applies:
A is an arylamino group, optionally substituted by one or more radicals R¹, or a carbazole-containing group, optionally substituted by one or more radicals R¹;
E is a single bond;
X is O or S;
Z is on each occurrence, identically or differently, CR² or N or C, where a group Z is equal to C precisely if a group A or E is bonded to the group Z in question;
R¹ is selected on each occurrence, identically or differently, from H, D, F, C(=O)R³, CN, Si(R³)₃, N(R³)₂, P(=O)(R³)₂, OR³, S(=O)R³, S(=O)₂R³, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R¹ may be linked to one another and may form a ring; where the said alkyl, alkoxy, alkenyl and alkynyl groups and the said aromatic ring systems and heteroaromatic ring systems may in each case be substituted by one or more radicals R³; and where one or more CH₂ groups in the said alkyl, alkoxy, alkenyl and alkynyl groups may be replaced by -R³C=CR³-, -C=C-, Si(R³)₂, C=O, C=NR³, -C(=O)O-, -C(=O)NR³-, NR³, P(=O)(R³), -O-, -S-, SO or SO₂;
R² is selected on each occurrence, identically or differently, from H, D, F, C(=O)R³, CN, Si(R³)₃, N(R³)₂, P(=O)(R³)₂, OR³, S(=O)R³, S(=O)₂R³, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R² may be linked to one another and may form a ring; where the said alkyl, alkoxy, alkenyl and alkynyl groups and the said aromatic ring systems and heteroaromatic ring systems may in each case be substituted by one or more radicals R³; and where one or more CH₂ groups in the said alkyl, alkoxy, alkenyl and alkynyl groups may be replaced by -R³C=CR³-, -C=C-, Si(R³)₂, C=O, C=NR³, -C(=O)O-, -C(=O)NR³-, NR³, P(=O)(R³), -O-, -S-, SO or SO₂;
R³ is selected on each occurrence, identically or differently, from H, D, F, C(=O)R⁴, CN, Si(R⁴)₃, N(R⁴)₂, P(=O)(R⁴)₂, OR⁴, S(=O)R⁴, S(=O)₂R⁴, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R³ may be linked to one another and may form a ring; where the said alkyl, alkoxy, alkenyl and alkynyl groups and the said aromatic ring systems and heteroaromatic ring systems may in each case be substituted by one or more radicals R⁴; and where one or more CH₂ groups in the said alkyl, alkoxy, alkenyl and alkynyl groups may be replaced by -R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO or SO₂;
R⁴ is selected on each occurrence, identically or differently, from H, D, F, CN, alkyl or alkoxy groups having 1 to 20 C atoms, alkenyl or alkynyl groups having 2 to 20 C atoms, aromatic ring systems having 6 to 40 aromatic ring atoms and heteroaromatic ring systems having 5 to 40 aromatic ring atoms; where two or more radicals R⁴ may be linked to one another and may form a ring; and where the said alkyl, alkoxy, alkenyl and alkynyl groups, aromatic ring systems and heteroaromatic ring systems may be substituted by F or CN;
i is equal to 1;
n is on each occurrence, identically or differently, 0 or 1, where the sum of all indices n is equal to 1, 2, 3 or 4, and
where at least two further layers are arranged between the hole-transporting layer and the emitting layer, and where no further emitting layers are arranged between the emitting layer and the anode.

2. Electronic device according to Claim 1, **characterised in that** X is equal to O.

3. Electronic device according to Claim 1 or 2, **characterised in that** the sum of the indices n is equal to 1.

4. Electronic device according to one or more of Claims 1 to 3, **characterised in that** R' is selected on each occurrence, identically or differently, from H, F, CN, straight-chain alkyl groups having 1 to 20 C atoms, branched or cyclic alkyl groups having 3 to 20 C atoms, aromatic ring systems having 6 to 40 aromatic ring atoms, and heteroaromatic ring systems having 5 to 40 aromatic ring atoms; where the said alkyl groups, the said aromatic ring systems and the said heteroaromatic ring systems may in each case be substituted by one or more radicals R³.

5. Electronic device according to one or more of Claims 1 to 4, **characterised in that** R² is selected on each occurrence, identically or differently, from H, F, CN, straight-chain alkyl groups having 1 to 20 C atoms, branched or cyclic alkyl groups having 3 to 20 C atoms, aromatic ring systems having 6 to 40 aromatic ring atoms, and heteroaromatic ring systems having 5 to 40 aromatic ring atoms; where the said alkyl groups, the said aromatic ring systems and the said heteroaromatic ring systems may in each case be substituted by one or more radicals R³.

6. Electronic device according to one or more of Claims 1 to 5, **characterised in that** R³ is selected on each occurrence, identically or differently, from H, F, CN, straight-chain alkyl groups having 1 to 20 C atoms, branched or cyclic alkyl groups having 3 to 20 C atoms, aromatic ring systems having 6 to 40 aromatic ring atoms, and heteroaromatic ring systems having 5 to 40 aromatic ring atoms; where the said alkyl groups, the said aromatic ring systems and the said heteroaromatic ring systems may in each case be substituted by one or more radicals R⁴.

7. Electronic device according to one or more of Claims 1 to 6, **characterised in that** the group A is an arylamino group, which may be substituted by one or more radicals R¹.

8. Electronic device according to one or more of Claims 1 to 7, **characterised in that** the arylamino group as group A corresponds to a formula A 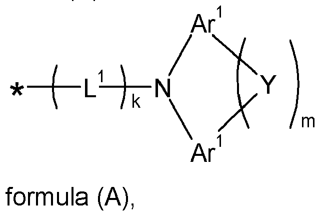 where the following applies:
L¹ is on each occurrence, identically or differently, C=O, Si(R¹)₂, PR¹, P(=O)(R¹), O, S, SO, SO₂, an alkylene group having 1 to 20 C atoms or an alkenylene or alkynylene group having 2 to 20 C atoms, where one or more CH₂ groups in the said groups may be replaced by C=O, C=NR¹, C=O-O, C=O-NR', Si(R¹)₂, NR¹, P(=O)(R¹), O, S, SO or SO₂ and where one or more H atoms in the above-mentioned groups may be replaced by D, F or CN, or an aromatic or heteroaromatic ring system having 6 to 24 aromatic ring atoms, which may be substituted by one or more radicals R¹;
Ar¹ is on each occurrence, identically or differently, an aromatic or heteroaromatic ring system having 6 to 30 aromatic ring atoms, which may be substituted by one or more radicals R¹;
Y is selected from a single bond, BR¹, C(R¹)₂, C(R¹)₂-C(R¹)₂, Si(R¹)₂, Si(R¹)₂-Si(R¹)₂, C=O, C=NR¹, C=C(R¹)₂, C(=O)N(R¹), O, S, S=O, SO₂ and NR¹;
k is equal to 0, 1, 2 or 3;
m is equal to 0 or 1;
where the group A is bonded to the remainder of the compound of the formula (I) via the bond marked with *.

9. Electronic device according to one or more of Claims 1 to 8, **characterised in that** the compound of the formula (I) corresponds to one of the following formulae 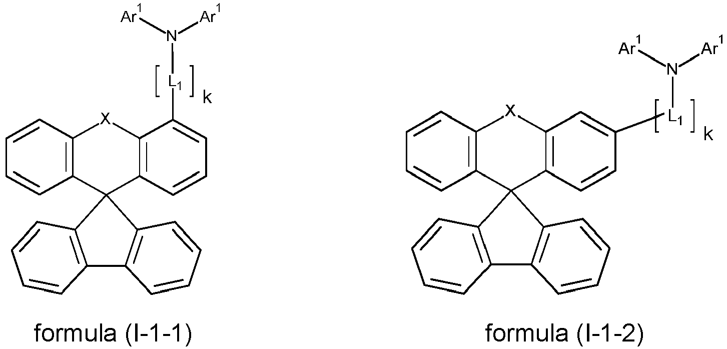 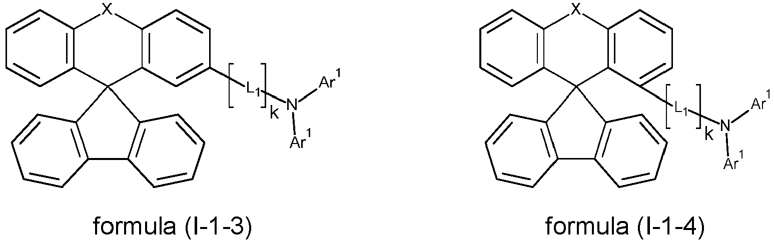 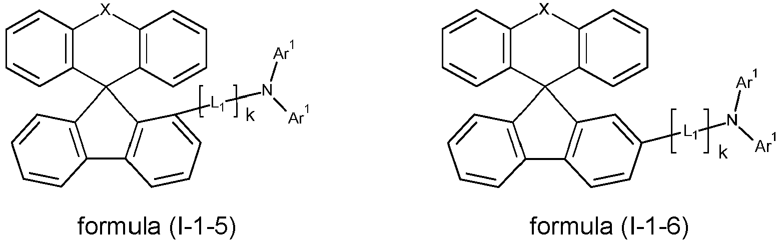 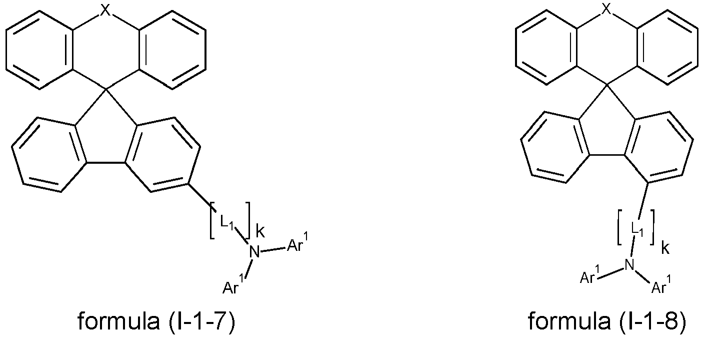 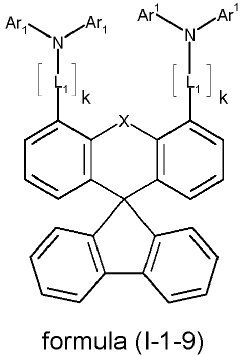 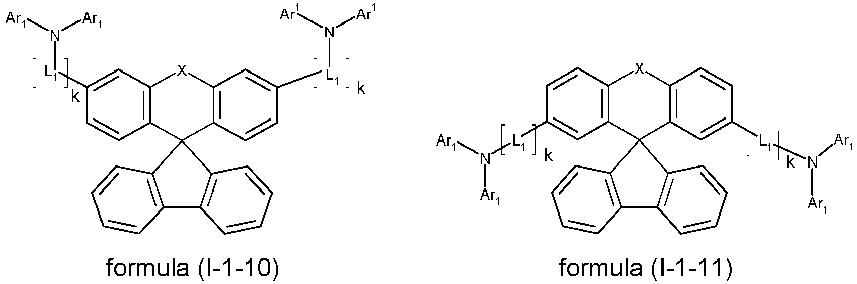 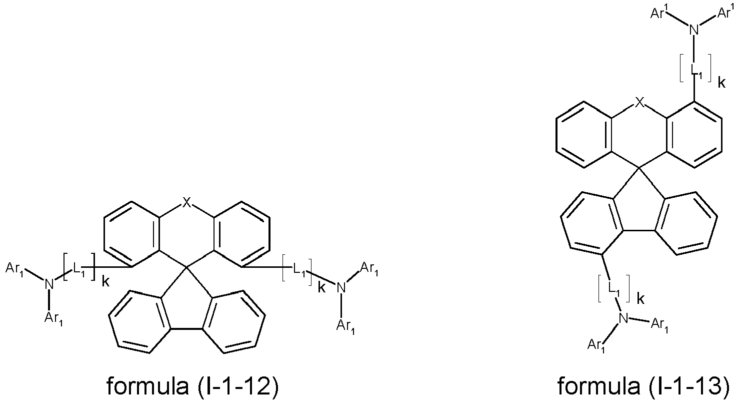 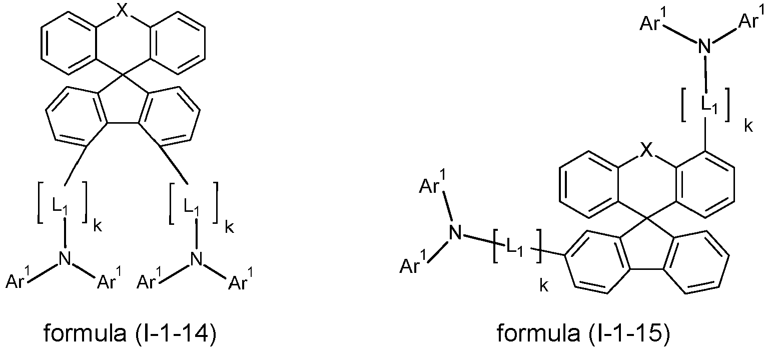 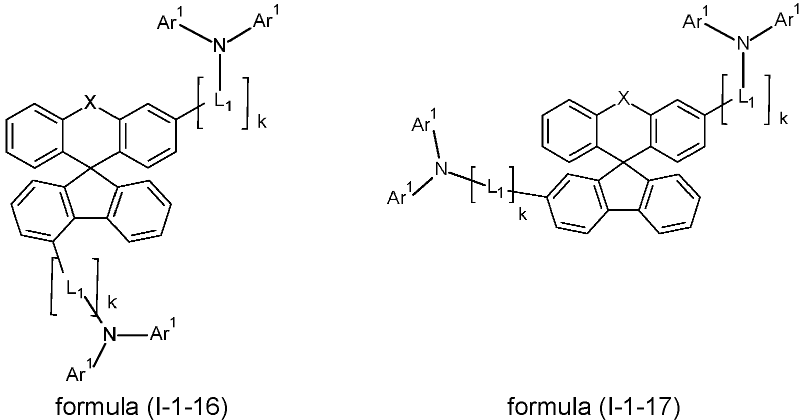 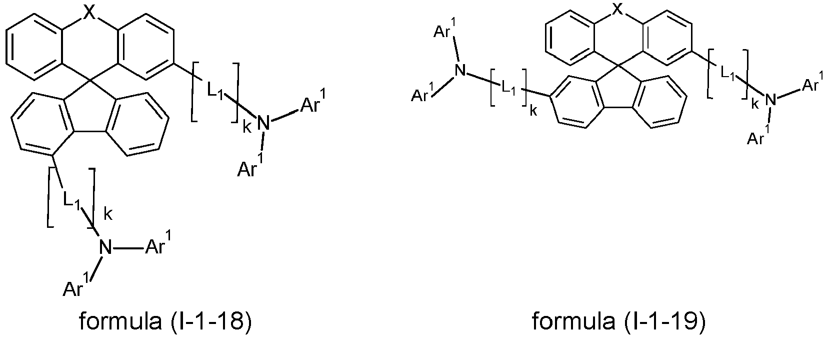 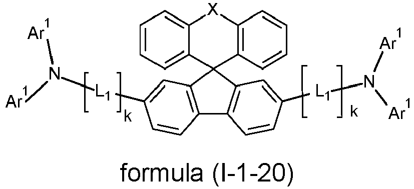 where X, L¹, Ar¹ and k are defined as in one or more of Claims 1 to 8, and where the compounds may be substituted by radicals R², defined as in Claim 1 or 5, at each of the positions on the benzene rings that are drawn as unsubstituted.

10. Electronic device according to one or more of Claims 1 to 9, **characterised in that** at least one further layer that does not comprise a compound of the formula (I) is present between the layer comprising the compound of the formula (I) and the emitting layer next to the anode.

11. Electronic device according to Claim 10, **characterised in that** the at least one further layer is a hole-transporting layer which comprises a monoamine compound containing at least one group selected from spirobifluorenyl groups, phenanthrenyl groups, fluorenyl groups, carbazolyl groups, dibenzofuranyl groups and dibenzothiophenyl groups.

12. Electronic device according to one or more of Claims 1 to 11, **characterised in that** the hole-transporting layer is directly adjacent to the anode, **in that** at least two further layers are arranged between the hole-transporting layer and the emitting layer, and **in that** no further emitting layers are arranged between the emitting layer and the anode.

13. Compound of a formula (S) 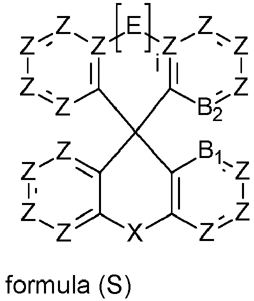 where a group A must be bonded to B₁, and where the following applies to the variables occurring:
B₂ is N or CR² or C, and B₁ is C;
Z is on each occurrence, identically or differently, CR² or N or C, where a group Z is equal to C precisely if a group E is bonded to the group Z in question;
A is an arylamino group, optionally substituted by one or more radicals R¹, or a carbazole-containing group, optionally substituted by one or more radicals R¹;
E is a single bond;
X is O or S;
R¹ is selected on each occurrence, identically or differently, from H, D, F, C(=O)R³, CN, Si(R³)₃, N(R³)₂, P(=O)(R³)₂, OR³, S(=O)R³, S(=O)₂R³, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R¹ may be linked to one another and may form a ring; where the said alkyl, alkoxy, alkenyl and alkynyl groups and the said aromatic ring systems and heteroaromatic ring systems may in each case be substituted by one or more radicals R³; and where one or more CH₂ groups in the said alkyl, alkoxy, alkenyl and alkynyl groups may be replaced by -R³C=CR³-, -C=C-, Si(R³)₂, C=O, C=NR³, -C(=O)O-, -C(=O)NR³-, NR³, P(=O)(R³), -O-, -S-, SO or SO₂;
R² is selected on each occurrence, identically or differently, from H, D, F, C(=O)R³, CN, Si(R³)₃, P(=O)(R³)₂, OR³, S(=O)R³, S(=O)₂R³, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R² may be linked to one another and may form a ring; where the said alkyl, alkoxy, alkenyl and alkynyl groups and the said aromatic ring systems and heteroaromatic ring systems may in each case be substituted by one or more radicals R³; and where one or more CH₂ groups in the said alkyl, alkoxy, alkenyl and alkynyl groups may be replaced by -R³C=CR³-, -C=C-, Si(R³)₂, C=O, -C(=O)O-, -C(=O)NR³-, P(=O)(R³), -O-, -S-, SO or SO₂;
R³ is selected on each occurrence, identically or differently, from H, D, F, C(=O)R⁴, CN, Si(R⁴)₃, N(R⁴)₂, P(=O)(R⁴)₂, OR⁴, S(=O)R⁴, S(=O)₂R⁴, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R³ may be linked to one another and may form a ring; where the said alkyl, alkoxy, alkenyl and alkynyl groups and the said aromatic ring systems and heteroaromatic ring systems may in each case be substituted by one or more radicals R⁴; and where one or more CH₂ groups in the said alkyl, alkoxy, alkenyl and alkynyl groups may be replaced by -R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO or SO₂;
R⁴ is selected on each occurrence, identically or differently, from H, D, F, CN, alkyl or alkoxy groups having 1 to 20 C atoms, alkenyl or alkynyl groups having 2 to 20 C atoms, aromatic ring systems having 6 to 40 aromatic ring atoms and heteroaromatic ring systems having 5 to 40 aromatic ring atoms; where two or more radicals R⁴ may be linked to one another and may form a ring; and where the said alkyl, alkoxy, alkenyl and alkynyl groups, aromatic ring systems and heteroaromatic ring systems may be substituted by F or CN;
i is equal to 0 or 1.

14. Compound according to Claim 13, **characterised in that** X is equal to O.

15. Compound according to Claim 13 or 14, **characterised in that** i is equal to 1.

16. Process for the preparation of a compound according to one or more of Claims 13 to 15, **characterised in that** it comprises an addition reaction of a metallated ether or thioether compound onto a diaryl ketone and a subsequent ring-closure reaction.

## Revendications

1. Dispositif électronique comprenant, dans cette séquence, une anode, une couche de transport de trous, une couche émettrice et une cathode, dans lequel la couche de transport de trous comprend un composé répondant à une formule (I) 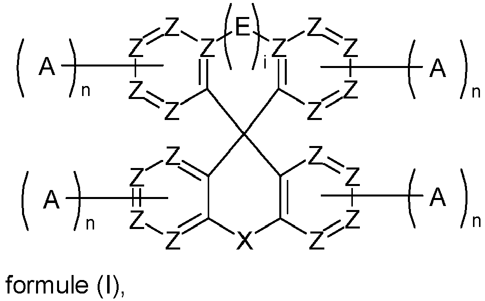 dans laquelle ce qui suit s'applique :
A est un groupement arylamino, éventuellement substitué par un ou plusieurs radicaux R¹, ou un groupement à base de carbazole, éventuellement substitué par un ou plusieurs radicaux R¹ ;
E est une liaison simple ;
X est O ou S ;
Z est à chaque occurrence, de manière identique ou différente, CR² ou N ou C, où un groupement Z est égal à C précisément si un groupement A ou E est lié au groupement Z en question ;
R¹ est choisi à chaque occurrence, de manière identique ou différente, parmi H, D, F, C(=O)R³, CN, Si(R³)₃, N(R³)₂, P(=O)(R³)₂, OR³, S(=O)R³, S(=O)₂R³, des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40 atomes de cycle aromatique, et des noyaux hétéroaromatiques ayant de 5 à 40 atomes de cycle aromatique ; où deux, ou plus, radicaux R¹ peuvent être liés les uns aux autres et peuvent former un cycle ; où lesdits groupements alkyle, alcoxy, alcényle et alcynyle et lesdits noyaux aromatiques et noyaux hétéroaromatiques peuvent dans chaque cas être substitués par un ou plusieurs radicaux R³ ; et où un ou plusieurs groupements CH₂ dans lesdits groupements alkyle, alcoxy, alcényle et alcynyle peuvent être remplacés par -R³C=CR³-, -C=C-, Si(R³)₂, C=O, C=NR³, -C(=O)O-, -C(=O)NR³-, NR³, P(=O)(R³), -O-, -S-, SO ou SO₂ ;
R² est choisi à chaque occurrence, de manière identique ou différente, parmi H, D, F, C(=O)R³, CN, Si(R³)₃, N(R³)₂, P(=O)(R³)₂, OR³, S(=O)R³, S(=O)₂R³, des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40 atomes de cycle aromatique, et des noyaux hétéroaromatiques ayant de 5 à 40 atomes de cycle aromatique ; où deux, ou plus, radicaux R² peuvent être liés les uns aux autres et peuvent former un cycle ; où lesdits groupements alkyle, alcoxy, alcényle et alcynyle et lesdits noyaux aromatiques et noyaux hétéroaromatiques peuvent dans chaque cas être substitués par un ou plusieurs radicaux R³ ; et où un ou plusieurs groupements CH₂ dans lesdits groupements alkyle, alcoxy, alcényle et alcynyle peuvent être remplacés par -R³C=CR³-, -C≡C-, Si(R³)₂, C=O, C=NR³, -C(=O)O-, -C(=O)NR³-, NR³, P(=O)(R³), -O-, -S-, SO ou SO₂ ;
R³ est choisi à chaque occurrence, de manière identique ou différente, parmi H, D, F, C(=O)R⁴, CN, Si(R⁴)₃, N(R⁴)₂, P(=O)(R⁴)₂, OR⁴, S(=O)R⁴, S(=O)₂R⁴, des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40 atomes de cycle aromatique, et des noyaux hétéroaromatiques ayant de 5 à 40 atomes de cycle aromatique ; où deux, ou plus, radicaux R³ peuvent être liés les uns aux autres et peuvent former un cycle ; où lesdits groupements alkyle, alcoxy, alcényle et alcynyle et lesdits noyaux aromatiques et noyaux hétéroaromatiques peuvent dans chaque cas être substitués par un ou plusieurs radicaux R⁴ ; et où un ou plusieurs groupements CH₂ dans lesdits groupements alkyle, alcoxy, alcényle et alcynyle peuvent être remplacés par-R⁴C=CR⁴-, -C≡C-, Si(R⁴)₂, C=O, C=NR⁴, -C(=O)O-,-C(=O)NR⁴-, NR⁴, P(=O)(R⁴), -O-, -S-, SO ou SO₂ ;
R⁴ est choisi à chaque occurrence, de manière identique ou différente, parmi H, D, F, CN, alkyle ou alcoxy groups ayant de 1 à 20 atomes de C, des groupements alcényle ou alcynyle ayant de 2 à 20 atomes de C, des noyaux aromatiques ayant de 6 à 40 atomes de cycle aromatique et des noyaux hétéroaromatiques ayant de 5 à 40 atomes de cycle aromatique ; où deux, ou plus, radicaux R⁴ peuvent être liés les uns aux autres et peuvent former un cycle ; et où lesdits groupements alkyle, alcoxy, alcényle et alcynyle, noyaux aromatiques et noyaux hétéroaromatiques peuvent être substitués par F ou CN ;
i est égal à 1 ;
n vaut à chaque occurrence, de manière identique ou différente, 0 ou 1, où la somme de tous les indices n est égale à 1, 2, 3 ou 4, et où au moins deux autres couches sont disposées entre la couche de transport de trous et la couche émettrice, et où aucune autre couche émettrice n'est disposée entre la couche émettrice et l'anode.

2. Dispositif électronique selon la revendication 1, **caractérisé en ce que** X est égal à O.

3. Dispositif électronique selon la revendication 1 ou 2, **caractérisé en ce que** la somme des indices n est égale à 1.

4. Dispositif électronique selon l'une ou plusieurs parmi les revendications 1 à 3, **caractérisé en ce que** R¹ est choisi à chaque occurrence, de manière identique ou différente, parmi H, F, CN, des groupements alkyle à chaîne linéaire ayant de 1 à 20 atomes de C, des groupements alkyle ramifiés ou cycliques ayant de 3 à 20 atomes de C, des noyaux aromatiques ayant de 6 à 40 atomes de cycle aromatique, et des noyaux hétéroaromatiques ayant de 5 à 40 atomes de cycle aromatique ; où lesdits groupements alkyle, lesdits noyaux aromatiques et lesdits noyaux hétéroaromatiques peuvent dans chaque cas être substitués par un ou plusieurs radicaux R³.

5. Dispositif électronique selon l'une ou plusieurs parmi les revendications 1 à 4, **caractérisé en ce que** R² est choisi à chaque occurrence, de manière identique ou différente, parmi H, F, CN, des groupements alkyle à chaîne linéaire ayant de 1 à 20 atomes de C, des groupements alkyle ramifiés ou cycliques ayant de 3 à 20 atomes de C, des noyaux aromatiques ayant de 6 à 40 atomes de cycle aromatique, et des noyaux hétéroaromatiques ayant de 5 à 40 atomes de cycle aromatique ; où lesdits groupements alkyle, lesdits noyaux aromatiques et lesdits noyaux hétéroaromatiques peuvent dans chaque cas être substitués par un ou plusieurs radicaux R³.

6. Dispositif électronique selon l'une ou plusieurs parmi les revendications 1 à 5, **caractérisé en ce que** R³ est choisi à chaque occurrence, de manière identique ou différente, parmi H, F, CN, des groupements alkyle à chaîne linéaire ayant de 1 à 20 atomes de C, des groupements alkyle ramifiés ou cycliques ayant de 3 à 20 atomes de C, des noyaux aromatiques ayant de 6 à 40 atomes de cycle aromatique, et des noyaux hétéroaromatiques ayant de 5 à 40 atomes de cycle aromatique ; où lesdits groupements alkyle, lesdits noyaux aromatiques et lesdits noyaux hétéroaromatiques peuvent dans chaque cas être substitués par un ou plusieurs radicaux R⁴.

7. Dispositif électronique selon l'une ou plusieurs parmi les revendications 1 à 6, **caractérisé en ce que** le groupement A est un groupement arylamino, qui peut être substitué par un ou plusieurs radicaux R¹.

8. Dispositif électronique selon l'une ou plusieurs parmi les revendications 1 à 7, **caractérisé en ce que** le groupement arylamino comme groupement A correspond à une formule (A) 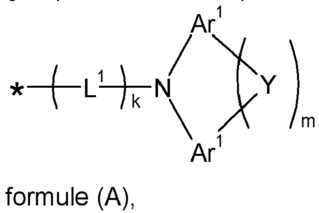 dans laquelle ce qui suit s'applique :
L¹ est à chaque occurrence, de manière identique ou différente, C=O, Si(R¹)₂, PR¹, P(=O)(R¹), O, S, SO, SO₂, un groupement alkylène ayant de 1 à 20 atomes de C ou un groupement alcénylène ou alcynylène ayant de 2 à 20 atomes de C, où un ou plusieurs groupements CH₂ dans lesdits groupements peuvent être remplacés par C=O, C=NR¹, C=O-O, C=O-NR¹, Si(R¹)₂, NR¹, P(=O)(R¹), O, S, SO ou SO₂ et où un ou plusieurs atomes de H dans les groupements susmentionnés peuvent être remplacés par D, F ou CN, ou un noyau aromatique ou hétéroaromatique ayant de 6 à 24 atomes de cycle aromatique, qui peut être substitué par un ou plusieurs radicaux R¹ ;
Ar¹ est à chaque occurrence, de manière identique ou différente, un noyau aromatique ou hétéroaromatique ayant de 6 à 30 atomes de cycle aromatique, qui peut être substitué par un ou plusieurs radicaux R¹ ;
Y est choisi parmi une liaison simple, BR¹, C(R¹)₂, C(R¹)₂-C(R¹)₂, Si(R¹)₂, Si(R¹)₂-Si(R¹)₂, C=O, C=NR¹, C=C(R¹)₂, C(=O)N(R¹), O, S, S=O, SO₂ et NR¹ ;
k est égal à 0, 1, 2 ou 3 ;
m est égal à 0 ou 1 ;
où le groupement A est lié au reste du composé de formule (I) via la liaison marquée par *.

9. Dispositif électronique selon l'une ou plusieurs parmi les revendications 1 à 8, **caractérisé en ce que** le composé de formule (I) correspond à l'une des formules suivantes 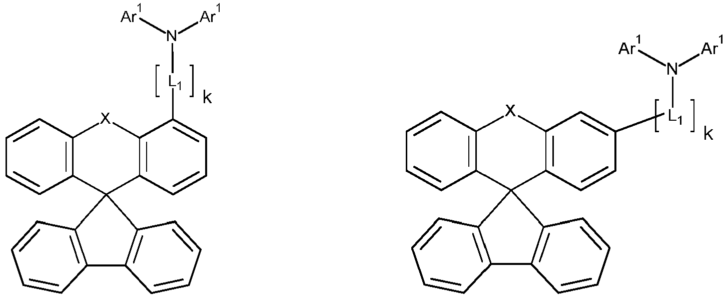 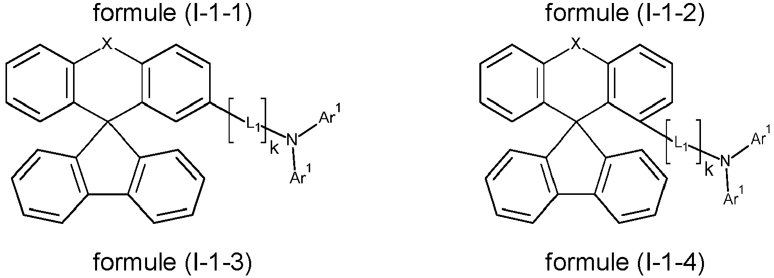 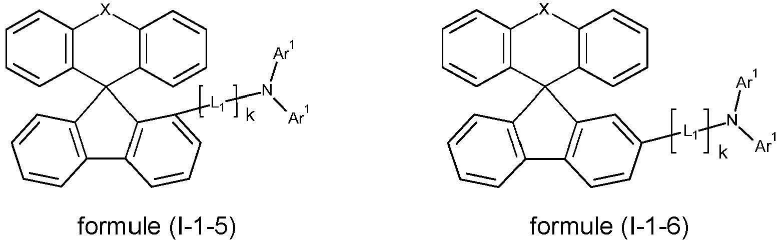 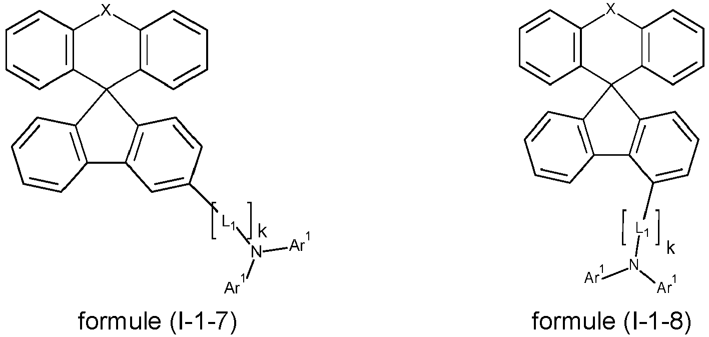 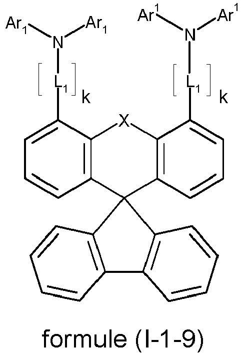 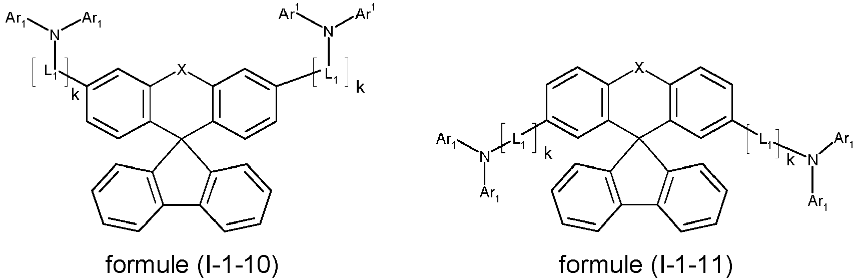 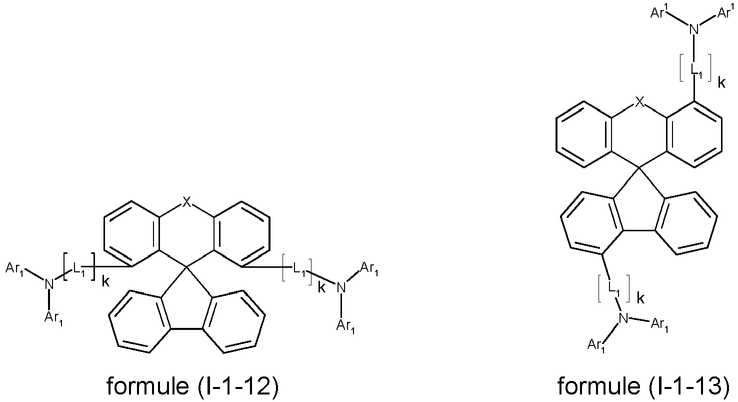 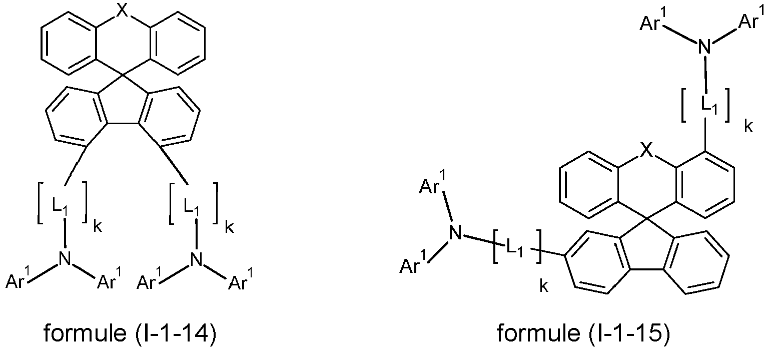 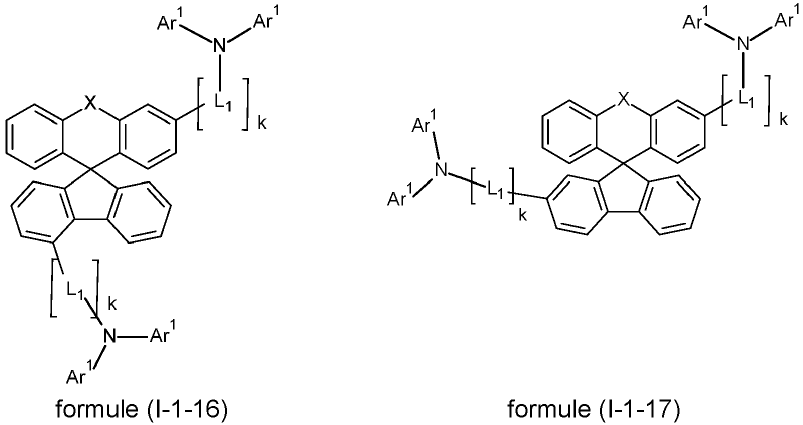 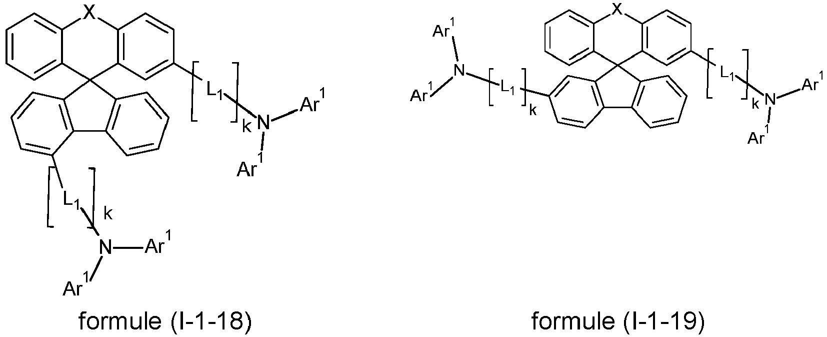 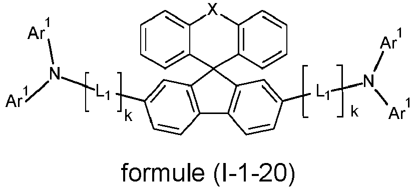 dans lesquelles X, L¹, Ar¹ et k sont tels que définis selon l'une ou plusieurs parmi les revendications 1 à 8, et où les composés peuvent être substitués par des radicaux R², tels que définis selon la revendication 1 ou 5, au niveau de chacune des positions sur les cycles benzéniques qui sont dessinées comme étant non substituées.

10. Dispositif électronique selon l'une ou plusieurs parmi les revendications 1 à 9, **caractérisé en ce qu'**au moins une autre couche qui ne comprend pas de composé de formule (I) est présente entre la couche comprenant le composé de formule (I) et la couche émettrice à proximité de l'anode.

11. Dispositif électronique selon la revendication 10, **caractérisé en ce que** la au moins une couche supplémentaire est une couche de transport de trous qui comprend un composé de monoamine contenant au moins un groupement choisi parmi les groupements spirobifluorényle, les groupements phénanthrényle, les groupements fluorényle, les groupements carbazolyle, les groupements dibenzofuranyle et les groupements dibenzothiophényle.

12. Dispositif électronique selon l'une ou plusieurs parmi les revendications 1 à 11, **caractérisé en ce que** la couche de transport de trous est directement adjacente à l'anode, **en ce qu'**au moins deux autres couches sont disposées entre la couche de transport de trous et la couche émettrice, et **en ce qu'**aucune autre couche émettrice n'est disposée entre la couche émettrice et l'anode.

13. Composé répondant à une formule (S) 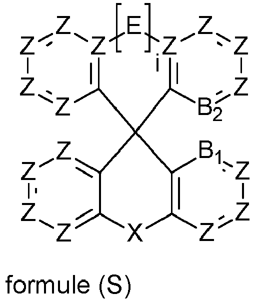 dans laquelle un groupement A doit être lié à B₁, et où ce qui suit s'applique aux variables présentes :
B₂ est N ou CR² ou C, et B₁ est C ;
Z est à chaque occurrence, de manière identique ou différente, CR² ou N ou C, où un groupement Z est égal à C précisément si un groupement E est lié au groupement Z en question ;
A est un groupement arylamino, éventuellement substitué par un ou plusieurs radicaux R¹, ou un groupement à base de carbazole, éventuellement substitué par un ou plusieurs radicaux R1 ;
E est une liaison simple ;
X est O ou S ;
R¹ est choisi à chaque occurrence, de manière identique ou différente, parmi H, D, F, C(=O)R³, CN, Si(R³)₃, N(R³)₂, P(=O)(R³)₂, OR³, S(=O)R³, S(=O)₂R³, des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40 atomes de cycle aromatique, et des noyaux hétéroaromatiques ayant de 5 à 40 atomes de cycle aromatique ; où deux, ou plus, radicaux R¹ peuvent être liés les uns aux autres et peuvent former un cycle ; où lesdits groupements alkyle, alcoxy, alcényle et alcynyle et lesdits noyaux aromatiques et noyaux hétéroaromatiques peuvent dans chaque cas être substitués par un ou plusieurs radicaux R³ ; et où un ou plusieurs groupements CH₂ dans lesdits groupements alkyle, alcoxy, alcényle et alcynyle peuvent être remplacés par -R³C=CR³-, -C≡C-, Si(R³)₂, C=O, C=NR³, -C(=O)O-, -C(=O)NR³-, NR³, P(=O)(R³), -O-, -S-, SO ou SO₂ ;
R² est choisi à chaque occurrence, de manière identique ou différente, parmi H, D, F, C(=O)R³, CN, Si(R³)₃, P(=O)(R³)₂, OR³, S(=O)R³, S(=O)₂R³, des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40 atomes de cycle aromatique, et des noyaux hétéroaromatiques ayant de 5 à 40 atomes de cycle aromatique ; où deux, ou plus, radicaux R² peuvent être liés les uns aux autres et peuvent former un cycle ; où lesdits groupements alkyle, alcoxy, alcényle et alcynyle et lesdits noyaux aromatiques et noyaux hétéroaromatiques peuvent dans chaque cas être substitués par un ou plusieurs radicaux R³ ; et où un ou plusieurs groupements CH₂ dans lesdits groupements alkyle, alcoxy, alcényle et alcynyle peuvent être remplacés par -R³C=CR³-, -C=C-, Si(R³)₂, C=O, -C(=O)O-, -C(=O)NR³-, P(=O)(R³), -O-, -S-, SO ou SO₂ ;
R³ est choisi à chaque occurrence, de manière identique ou différente, parmi H, D, F, C(=O)R⁴, CN, Si(R⁴)₃, N(R⁴)₂, P(=O)(R⁴)₂, OR⁴, S(=O)R⁴, S(=O)₂R⁴, des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40 atomes de cycle aromatique, et des noyaux hétéroaromatiques ayant de 5 à 40 atomes de cycle aromatique ; où deux, ou plus, radicaux R³ peuvent être liés les uns aux autres et peuvent former un cycle ; où lesdits groupements alkyle, alcoxy, alcényle et alcynyle et lesdits noyaux aromatiques et noyaux hétéroaromatiques peuvent dans chaque cas être substitués par un ou plusieurs radicaux R⁴ ; et où un ou plusieurs groupements CH₂ dans lesdits groupements alkyle, alcoxy, alcényle et alcynyle peuvent être remplacés par -R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO ou SO₂ ;
R⁴ est choisi à chaque occurrence, de manière identique ou différente, parmi H, D, F, CN, des groupements alkyle ou alcoxy ayant de 1 à 20 atomes de C, des groupements alcényle ou alcynyle ayant de 2 à 20 atomes de C, des noyaux aromatiques ayant de 6 à 40 atomes de cycle aromatique et des noyaux hétéroaromatiques ayant de 5 à 40 atomes de cycle aromatique ; où deux, ou plus, radicaux R⁴ peuvent être liés les uns aux autres et peuvent former un cycle ; et où lesdits groupements alkyle, alcoxy, alcényle et alcynyle, noyaux aromatiques et noyaux hétéroaromatiques peuvent être substitués par F ou CN ;
i est égal à 0 ou 1.

14. Composé selon la revendication 13, **caractérisé en ce que** X est égal à O.

15. Composé selon la revendication 13 ou 14, **caractérisé en ce que** i est égal à 1.

16. Procédé de préparation d'un composé selon l'une ou plusieurs parmi les revendications 13 à 15, **caractérisé en ce qu'**il comprend une réaction d'addition d'un composé d'éther ou de thioéther métallé sur une diaryl cétone et une réaction ultérieure de fermeture de cycle.
